# EUROPEAN PATENT APPLICATION

(11) **EP 1 666 067 A1**
(43) Date of publication of application: **07.06.2006**
(21) Application number: 04772291.3
(22) Date of filing: 20.08.2004
(51) Int. Cl.: A61K 45/00, A61K 45/06, A61K 31/4184, A61P 3/00, A61P 3/04, A61P 3/10, A61P 9/00, A61P 9/10, A61P 9/12, A61P 13/12, A61P 25/02, A61P 25/28, A61P 27/02, C07D 409/06

(54) **DRUG CONTAINING CHYMASE INHIBITOR AS THE ACTIVE INGREDIENT**

(30) Priority: 22.08.2003 JP 2003298639
(71) Applicant: Teijin Pharma Limited, Tokyo 100-0011 (JP)
(72) Inventor: URATA, Hidenori, Fukuoka-shi, Fukuoka 8140143 (JP); HASE, Naoki, c/o Teijin Pharma Limited, Hino-shi, Tokyo 1910065 (JP); TSUCHIYA, Naoki, c/o Teijin Pharma Limited, Hino-shi, Tokyo 1910065 (JP)
(74) Representative: Hallybone, Huw George
(86) International application number: PCT/JP2004/012335
(87) International publication number: WO 2005/018672

(57) **Abstract**

The present invention provides drugs containing chymase inhibitors as active ingredients for improving glucose intolerance or preventing and/or treating diseases caused by glucose intolerance. The diseases caused by glucose intolerance are diabetes and/or diabetes complications, wherein the diabetes complications include diabetic nephropathy, diabetic retinopathy, diabetic peripheral neuropathy, hyperinsulinism, insulin resistance syndrome, arteriosclerosis, acute coronary syndrome, arteriosclerosis obliterans, angitis, stroke, hypertension, renal insufficiency, nephropathy, nephritis, renal artery aneurysm, renal infarction, obesity and the like.

## Description

### TECHNICAL FIELD

The present invention relates to drugs that contain chymase inhibitors as active ingredients, wherein the drugs are agents for improving glucose intolerance or for preventing and/or treating diseases caused by glucose intolerance.

More specifically, the present invention relates to the drugs for diseases caused by glucose intolerance, wherein the diseases are diabetes and/or diabetes complications, wherein the diabetes complications are diabetic nephropathy, diabetic retinopathy, diabetic peripheral neuropathy, hyperinsulinism, insulin resistance syndrome, arteriosclerosis, acute coronary syndrome, arteriosclerosis obliterans, angitis, stroke, hypertension, nephropathy, nephritis, renal artery aneurysm, renal infarction, or obesity.

### BACKGROUND ART

Glucose intolerance refers to insufficiency of insulin secretion response due to glucose load and/or reduction of insulin action in skeletal muscles or adipose tissues. In many cases, glucose intolerance is caused by insulin resistance. Glucose intolerance is regarded as conditions that precede the onset of diabetes and is multiply associated with various metabolic diseases (obesity, hypertension, hypertriglyceridemia and the like). This multiple metabolic disorder is also referred to as the deadly quartet ("The deadly quartet. Upper-body obesity, glucose intolerance, hyper triglyceridemia, and hypertension." Archives of International Medicine, (USA) 1989, Vol. 149, No. 7, p. 1514), insulin resistance syndrome ("Insulin resistance. A multifaceted syndrome responsible for NIDDM, obesity, hypertension, dyslipidemia, and atherosclerotic cardiovascular disease." Diabetes Care, (USA) 1991, Vol. 14, No. 3, p. 173), or multiple risk factor syndrome. Insulin intolerance also enhances occurrence frequency of coronary artery diseases such as angina pectoris, myocardial infarction and stroke.

Continuous glucose intolerant conditions induce new onset of diabetes and also enhances its progress. Therefore, improvement of glucose intolerance is considered effective in preventing onset of diabetes, inhibiting its progress and preventing onset of diabetes complications.

Diabetes is a disease that causes increase in the blood glucose level before or after meals. Two types of diabetes are known: type I diabetes that significantly reduces insulin secretion from pancreas and type II diabetes that causes insulin resistance in liver, skeletal muscles, or adipose tissues and deficiency of insulin secretion by pancreas due to an excessive intake of food, insufficient exercise and the like. Most of diabetics belong to type II diabetics.

Diabetes, as it progresses, induces complications such as diabetic retinopathy, diabetic nephropathy and diabetic peripheral neuropathy, and furthermore causes various serious diseases such as renal insufficiency, arteriosclerosis and hypertension. Accordingly prevention and treatment of diabetes is important for prevention of diabetes complications.

Treatment of glucose intolerance and/or diabetes widely uses, in addition to dietetic treatment and kinesitherapy, blood glucose level controlling drugs such as sulfonylureas, biguanides, α-glycosidase inhibitors and agonists for peroxisome proliferation-related receptor γ, or other various therapeutic agents. Either of the treatments is, however, not yet satisfactory with respect to effectiveness, patients' compliance, side effects, etc. In fact, the number of diabetics and that of potential diabetics tend to increase in these years. There is still a demand for therapeutic agents with high effectiveness and insignificant side effects.

Chymase is one of neutral proteases occurring in mast cell granules and is deeply involved in various biological reactions related to mast cells. There has been reported various actions of chymase, for example, enhancement of degranulation in mast cells, activation of interleukin- 1β (IL-1β), activation of matrix protease, degradation of fibronectins and type IV collagen, enhancement of release of transforming growth factor-β (TGF-β), activation of substance P and vasoactive intestinal polypeptide (VIP), conversion of angiotensin (Ang)I into AngII, conversion of endothelin and the like.

With respect to the relationship between mast cells containing chymase and glucose metabolism, however, there have been few reports so far, and a number of questions remain unresolved. Some study reported that mast cells are scarcely present in pancreas or kidney ("Mast cell distribution in rats" Arzneimittelforschung, (Germany) 1994, Vol. 44, No. 3, p. 370). Further, nothing has been reported on the relationship between β cells in Langerhans island and mast cells or on involvement of chymase in insulin secretion in pancreas.

### DISCLOSURE OF THE INVENTION

An object of the present invention is to provide drugs that contain chymase inhibitors as active ingredients, wherein the drugs are agents for improving glucose intolerance or for preventing and/or treating diseases caused by glucose intolerance such as diabetes and/or diabetes complications.

The present investors found that chymase inhibitors improve glucose intolerance and achieved the present invention.

In other words, the present invention is drugs that contain chymase inhibitors as active ingredients, wherein the drugs are agents for improving glucose intolerance or for preventing and/or treating diseases caused by glucose intolerance.

Furthermore, the present invention is the preventive agents and/or therapeutic agents for diseases caused by glucose intolerance, wherein the diseases are diabetes and/or diabetes complications, wherein the diabetes complications are diabetic nephropathy, diabetic retinopathy, diabetic peripheral neuropathy, hyperinsulinism, insulin resistance syndrome, arteriosclerosis, acute coronary syndrome, arteriosclerosis obliterans, angitis, stroke, hypertension, renal insufficiency, nephropathy, nephritis, renal artery aneurysm, renal infarction or obesity.

### BRIEF DESCRIPTION OF THE DRAWINGS

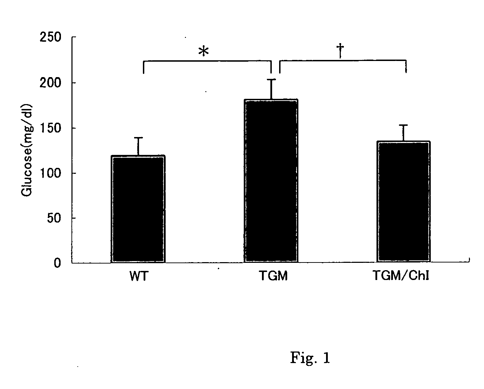

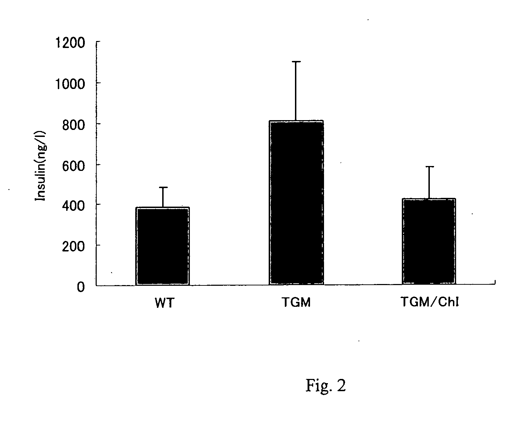

Fig. 1 is a graph showing the blood glucose levels for Wild, TGM and TGM/ChI after glucose loading. Fig. 2 is a graph showing the concentrations of blood insulin for Wild, TGM and TGM/ChI after glucose loading.

### BEST MODE FOR CARRYING OUT THE INVENTION

Drugs in the present invention use chymase inhibitors as active ingredients. The diseases caused by glucose intolerance related to the present invention include diabetes and/or diabetes complications. The diabetes complications include diabetic nephropathy, diabetic retinopathy, diabetic peripheral neuropathy, hyperinsulinism, insulin resistance syndrome, arteriosclerosis, acute coronary syndrome, arteriosclerosis obliterans, angitis, stroke, hypertension, renal insufficiency, nephropathy, nephritis, renal artery aneurysm, renal infarction, obesity and the like.

Chymase inhibitors used in the present invention are, although not particularly limited, preferably the benzimidazole derivatives or medically acceptable salts thereof described in WO 01/53291, WO 01/53272 and WO 00/03997. In particular, preferred is the following compound (I): 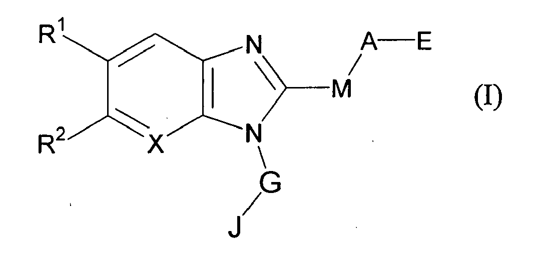 [wherein R¹ and R² simultaneously or each independently represent hydrogen, halogen, trihalomethyl, cyano, hydroxyl, C₁-C₄ alkyl or C₁-C₄ alkoxy, or R¹ and R² taken together represent -O-CH₂-O-, -O-CH₂CH₂-O- or -CH₂CH₂CH₂- (wherein the carbon atoms may be optionally substituted with one or more C₁-C₄ alkyl);
A represents substituted or unsubstituted straight, cyclic or branched C₁-C₇ alkylene or C₁-C₇ alkenylene, which may be interrupted by one or more of atoms or groups selected from -O-, -S-, -SO₂- and -NR³-, (wherein R³ represents hydrogen or straight or branched C₁-C₆ alkyl). The substituents on these groups are selected from halogen, hydroxyl, nitro, cyano, straight or branched C₁-C₆ alkyl, straight or branched C₁-C₆ alkoxy (including cases wherein the neighboring two form an acetal), straight or branched C₁-C₆ alkylthio, straight or branched C₁-C₆ alkylsulfonyl, straight or branched C₁-C₆ acyl, straight or branched C₁-C₆ acylamino, trihalomethyl, trihalomethoxy, phenyl, oxo or phenoxy optionally substituted with one or more halogen atoms. These substituents may be present each independently at one or more arbitrary positions in the alkylene or alkenylene, except for the case wherein M represents a single bond and the carbon atom of A directly bonded to M is substituted with a hydroxyl and a phenyl at the same time;
E represents -COOR³, -SO₃R³, -CONHR³, -SO₂NHR³, tetrazol-5-yl,
5-oxo-1,2,4-oxadiazol-3-yl or 5-oxo-1,2,4-thaidiazol-3-yl, (wherein R³ is as defined above);
G represents substituted or unsubstituted straight or branched C₁-C₆ alkylene, which may be interrupted by one or more of atoms or groups selected from -O-, -S-, -SO₂- and -NR³- , (wherein R³ is as defined above, provided that either of these atoms or groups is not directly bonded to the benzimidazole ring). The substituents on the alkylene are selected from halogen, hydroxyl, nitro, cyano, straight or branched C₁-C₆ alkyl, straight or branched C₁-C₆ alkoxy (including cases wherein neighboring two form an acetal), trihalomethyl, trihalomethoxy, phenyl or oxo;
M represents a single bond or -S(O)ₘ-, wherein m is an integer ranging from 0 to 2;
J represents substituted or unsubstituted C₄-C₁₀ heteroaryl (one or more heteroatoms selected from the group consisting of oxygen, nitrogen and sulfur in the ring), except for imidazole or unsubstituted pyridine ring. The substituents on the heteroaryl are selected from halogen, hydroxyl, nitro, cyano, straight or branched C₁-C₆ alkyl, straight or branched C₁-C₆ alkoxy (including cases wherein neighboring two form an acetal), straight or branched C₁-C₆ alkylthio, straight or branched C₁-C₆ alkylsulfonyl, straight or branched C₁-C₆ acyl, straight or branched C₁-C₆ acylamino, substituted or unsubstituted anilido, trihalomethyl, trihalomethoxy, phenyl, oxo, COOR³ and phenoxy optionally substituted with one or more halogen atoms. One or more of these substituents each may be present at any positions in the ring; and
X represents -CH= or nitrogen.]

The substituents in the compounds of formula (I) in the present invention are as follows:

R¹ and R² simultaneously or each independently represent hydrogen, halogen, trihalomethyl, cyano, hydroxyl, C₁-C₄ alkyl or C₁-C₄ alkoxy, or R¹ and R² taken together represent -O-CH₂-O-, -O-CH₂CH₂-O- or -CH₂CH₂CH₂-, wherein the carbon atoms may be optionally substituted with one or more C₁-C₄ alkyl.

The C₁-C₄ alkyl of R¹ and R² includes specifically methyl, ethyl, (n-, i-)propyl and (n-, i-, s-, t-)butyl, and is preferably methyl. The C₁-C₄ alkoxy includes specifically methoxy, ethoxy, (n-, i-)propyloxy and (n-, i-, s-, t-)butyloxy.

Groups suitable to R¹ and R² include hydrogen, halogen, trihalomethyl, cyano, hydroxyl, C₁-C₄ alkyl and C₁-C₄ alkoxy. R¹ and R² are preferably hydrogen, halogen, trihalomethyl, cyano, C₁-C₄ alkyl or C₁-C₄ alkoxy, more preferably hydrogen, C₁-C₄ alkyl, C₁-C₄ alkoxy, halogen or cyano, further preferably hydrogen, Cl, F, trifluoromethyl, methyl, methoxy or ethoxy, further more preferably hydrogen, C₁-C₄ alkyl, or C₁-C₄ alkoxy, and especially preferably hydrogen, methyl or methoxy.

A represents substituted or unsubstituted straight, cyclic or branched C₁-C₇ alkylene or alkenylene. The unsubstituted straight, cyclic or branched C₁-C₇ alkylene includes methylene, ethylene, (n-, i-)propylene, 2,2-dimethylpropylene, (n-, i-, t-)butylene, 1,1-dimethylbutylene, n-pentylene, cyclohexylene and the like. It is preferably ethylene, n-propylene, 2,2-dimethylpropylene, or (n-, t-)butylene, more preferably n-propylene or 2,2-dimethylpropylene, and especially preferably, n-propylene. The unsubstituted straight or branched C₁-C₇ alkenylene includes vinylene, propenylene, butenylene, pentenylene and the like.

These alkylene or alkenylene may be interrupted by one or more of atoms or groups selected from -O-, -S-, -SO₂- and -NR³-, (wherein R³ represents hydrogen or straight or branched C₁-C₆ alkyl), provided that either of these atoms or groups is not directly bonded to M. An example is ethylene, n-propylene, or (n-, t-)butylene interrupted by these atoms or groups. Further specifically it is -CH₂OCH₂- -CH₂OCH₂CH₂- -CH₂SCH₂-, -CH₂SCH₂CH₂-, -CH₂SO₂CH₂-, -CH₂SO₂CH₂CH₂-, -CH₂NR⁴CH₂-, -CH₂NR⁴CH₂CH₂- or the like, and preferably -CH₂OCH₂-, -CH₂SCH₂- or -CH₂SO₂CH₂-.

The substituents on these alkylene are selected from halogen, hydroxyl, nitro, cyano, straight or branched C₁-C₆ alkyl, straight or branched C₁-C₆ alkoxy (including cases wherein neighboring two form an acetal), straight or branched C₁-C₆ alkylthio, straight or branched C₁-C₆ alkylsulfonyl, straight or branched C₁-C₆ acyl, straight or branched C₁-C₆ acylamino, trihalomethyl, trihalomethoxy, phenyl, oxo and phenoxy optionally substituted with one or more halogen atoms. One or more of these substituents each may independently be present at any positions in the alkylene or alkenylene, except for the case wherein M is a single bond and the carbon atom bonded to M in A is substituted with a hydroxyl and a phenyl at the same time.

The halogen is F, Cl, Br or I, preferably F or C1.

The straight or branched C₁-C₆ alkyl is specifically methyl, ethyl, (n-, i-)propyl, (n-, i-, s-, t-)butyl, or the like, preferably methyl or ethyl, more preferably methyl.

The straight or branched C₁-C₆ alkoxy is specifically methoxy, ethoxy, (n-, i-)propyloxy, (n-, i-, s-, t-)butyloxy, or the like, preferably methoxy or ethoxy, more preferably methoxy.

The straight or branched C₁-C₆ alkylthio is specifically methylthio, ethylthio, (n-, i-)propylthio, (n-, i-, s-, t-)butylthio, or the like, preferably methylthio or ethylthio, more preferably methylthio.

The straight or branched C₁-C₆ alkylsulfonyl is specifically methylsulfonyl, ethylsulfonyl, (n-, i-)propylsulfonyl, (n-, i-, s-, t-)butylsulfonyl, or the like, preferably methylsulfonyl or ethylsulfonyl, more preferably methylsulfonyl.

The straight or branched C₁-C₆ acyl is specifically acetyl, ethylcarbonyl, (n-, i-)propylcarbonyl, (n-, i-, s-, t-)carbonyl, or the like, preferably acetyl or ethylcarbonyl, more preferably acetyl.

The straight or branched C₁-C₆ acylamino is specifically acetylamino, ethylcarbonylamino, (n-, i-)propylcarbonylamino, (n-, i-, s-, t-)carbonylamino, or the like, preferably acetylamino or ethylcarbonylamino, more preferably acetylamino.

The trihalomethyl group is specifically trifluoromethyl, tribromomethyl or trichloromethyl, preferably trifluoromethyl.

Above all, suitable examples of A are substituted or unsubstituted straight, cyclic or branched C₁-C₇ alkylene, {which may be interrupted by one or more of atoms or groups selected from -O-, -S-, -SO₂- and NR³-, (wherein NR³ is as defined above), provided that either of these atoms or groups is not directly bonded to M}. Preferably, A is -CH₂CH₂-, -CH₂CH₂CH₂-, -CH₂C(=O)CH₂-, -CH₂OCH₂-, -CH₂SCH₂-, -CH₂S(=O)CH₂-, -CH₂CF₂CH₂-, -CH₂SO₂CH₂-, -CH₂CH₂CH₂CH₂-, -CH₂C(CH₃)₂CH₂-, -CH₂SO₂CH₂CH₂-, -CH₂C(=O)CH₂CH₂-, -CH₂C(=O)(CH₃)₂CH₂-, -CH₂C(=O)C(=O)CH₂- or the like, more preferably -CH₂CH₂-, -CH₂CH₂CH₂-, -CH₂C(=O)CH₂-, -CH₂OCH₂-, -CH₂SCH₂-, -CH₂S(=O)CH₂-, -CH₂CF₂CH₂-, -CH₂SO₂CH₂- or -CH₂C(CH₃)₂CH₂-, further preferably -CH₂CH₂-, -CH₂CH₂CH₂- or -CH₂C(CH₃)₂CH₂-, especially preferably -CH₂CH₂CH₂-.

E represents -COOR³, -SO₃R³, -CONHR³, -SO₂NHR³, tetrazol-5-yl, 5-oxo-1,2,4-oxadiazol-3-yl or 5-oxo-1,2,4-thiadiazol-3-yl, (wherein R³ represents hydrogen or straight or branched C₁-C₆ alkyl).

R³ is specifically hydrogen, methyl, ethyl, (n-, i-)propyl, (n-, i-, s-, t-)butyl or the like, preferably hydrogen, methyl or ethyl, especially preferably hydrogen.

Above all, E is preferably -COOR³, -SO₃R³ or tetrazol-5-yl, more preferably -COOR³, especially preferably -COOH.

G represents substituted or unsubstituted straight or branched C₁-C₆ alkylene, which may be interrupted by one or more of atoms or groups selected from -O-, -S-, -SO₂- and NR³-, wherein R³ is as defined above. Also, either of these heteroatoms or groups is, if present, not directly bonded to the benzimidazole ring. The substituents on the alkylene are selected from halogen, hydroxyl, nitro, cyano, straight or branched C₁-C₆ alkyl, straight or branched C₁-C₆ alkoxy (including cases wherein neighboring two form an acetal), trihalomethyl, trihalomethoxy, phenyl and oxo. Specifically G is -CH₂-, -CH₂CH₂-, -CH₂CO-, -CH₂CH₂O-, -CH₂CONH-, -CO-, -SO₂-, -CH₂SO₂-, -CH₂S-, -CH₂CH₂S- or the like, preferably -CH₂-, -CH₂CH₂-, -CH₂CO- or -CH₂CH₂O-, more preferably -CH₂- or -CH₂CH₂-, especially preferably -CH₂-. Each of the groups listed here is bonded to the 1-position (N atom) of the benzimidazole at the left side whereas it is attached to J at the right side.

M represents a single bond or -S(O)ₘ- wherein m is an integer ranging from 0 to 2. M is preferably -S- or -SO₂- , especially preferably -S-.

J represents substituted or unsubstituted C₄-C₁₀ heteroaryl (one or more heteroatoms selected from oxygen, nitrogen and sulfur in the ring), except for imidazole or unsubstituted pyridine ring. Furthermore, J is limited to chemically synthesizable groups.

The unsubstituted C₄-C₁₀ heteroaryl (one or more heteroatoms selected from oxygen, nitrogen and sulfur in the ring) is, specifically, furyl, thienyl, thiazolyl, pyrimidinyl, oxazolyl, isoxazolyl, benzofuryl, benzimidazolyl, quinolyl, isoquinolyl, quinoxalinyl, benzoxadiazolyl, benzothiadiazolyl, indolyl, benzothiazolyl, benzothienyl, benzoisoxazolyl or the like, preferably bicyclic heteroaryl, more preferably benzofuryl, benzimidazolyl, quinolyl, isoquinolyl, quinoxalinyl, benzoxadiazolyl, benzothiadiazolyl, indolyl, benzothiazolyl, benzothienyl or benzoisoxazolyl, especially preferably benzothienyl or indolyl, further preferably benzothienyl.

The substituents on the heteroaryl described above are halogen, hydroxyl, nitro, cyano, straight or branched C₁-C₆ alkyl, straight or branched C₁-C₆ alkoxy (including cases wherein neighboring two form an acetal), straight or branched C₁-C₆ alkylthio, straight or branched C₁-C₆ alkylsulfonyl, straight or branched C₁-C₆ acyl, straight or branched C₁-C₆ acylamino, substituted or unsubstituted anilido, trihalomethyl, trihalomethoxy, phenyl or phenoxy optionally substituted with one or more halogen atoms. One or more of these substituents each may independently be present at any positions in the ring.

The halogen is F, Cl, Br or I, preferably F or Cl.

The straight or branched C₁-C₆ alkyl is specifically methyl, ethyl, (n-, i-)propyl, (n-, i-, s-, t-)butyl, or the like, preferably methyl or ethyl, further preferably methyl.

The straight or branched C₁-C₆ alkoxy is specifically methoxy, ethoxy, (n-, i-)propyloxy, (n-, i-, s-, t-)butyloxy, methylenedioxy or the like, preferably methoxy or ethoxy, further preferably methoxy.

The straight or branched C₁-C₆ alkylthio is specifically methylthio, ethylthio, (n-, i-)propylthio, (n-, i-, s-, t-)butylthio, or the like, preferably methylthio or ethylthio, further preferably methylthio.

The straight or branched C₁-C₆ alkylsulfonyl is specifically methylsulfonyl, ethylsulfonyl, (n-, i-)propylsulfonyl, (n-, i-, s-, t-)butylsulfonyl, or the like, preferably methylsulfonyl or ethylsulfonyl, further preferably methylsulfonyl.

The straight or branched C₁-C₆ acyl is specifically acetyl, ethylcarbonyl, (n-, i-)propylcarbonyl, (n-, i-, s-, t-)carbonyl, or the like, preferably acetyl or ethylcarbonyl, further preferably acetyl.

The straight or branched C₁-C₆ acylamino is specifically acetylamino, ethylcarbonylamino, (n-, i-)propylcarbonylamino, (n-, i-, s-, t-)carbonylamino, or the like, preferably acetylamino or ethylcarbonylamino, further preferably acetylamino.

The trihalomethyl is specifically trifluoromethyl, tribromomethyl or trichloromethyl.

The substituent in J is preferably halogen, cyano, straight or branched C₁-C₄ alkyl, straight or branched C₁-C₄ alkoxy (including cases wherein neighboring two form an acetal) or trihalomethyl, more preferably F, Cl, cyano, methyl, methoxy or trifluoromethyl, further preferably methyl.

X represents -CH= or nitrogen, preferably -CH=.

Preferable compounds represented by formula (I) are a set of various compounds composed by each of the groups referred to as preferred. Although not to be limited, compounds listed in the following table are preferred. Above all, those particularly preferred are the compounds ofNo. 34, 38, 39, 41, 42, 52, 54, 56, 58, 59, 63, 135, 137, 148, 152, 154,244, 340, 436, 514, 519, 521, 532, 534, 536, 538, 615, 628, 1112 and 1114.

In the following table A1 to A3 and J1 to J32 are groups represented by the following formulae, wherein E, G, M, m and X are as defined above. As representative examples, here are shown those wherein E is COOH, G is CH₂, M is S (m is 0) or a single bond (represented by "^{_}" in the table) and X is -CH=, although not to be limited thereto.

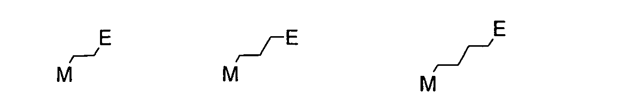

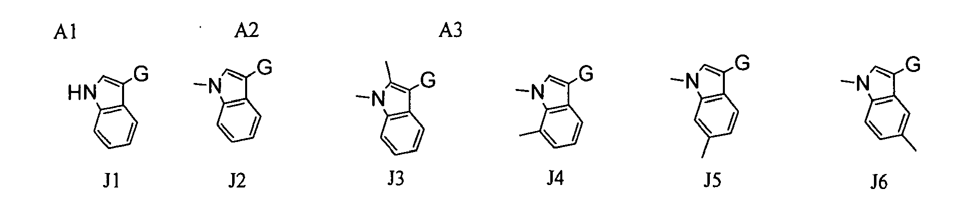

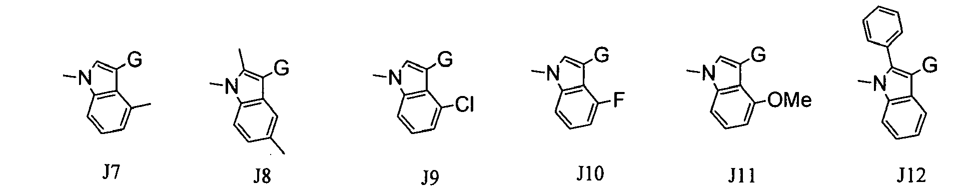

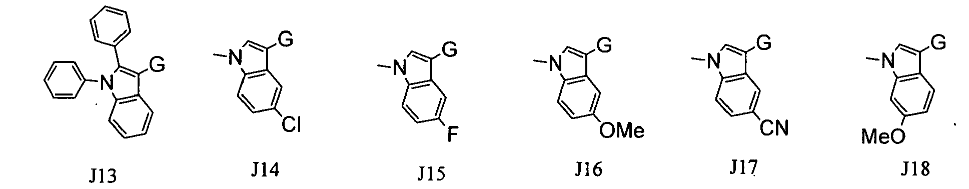

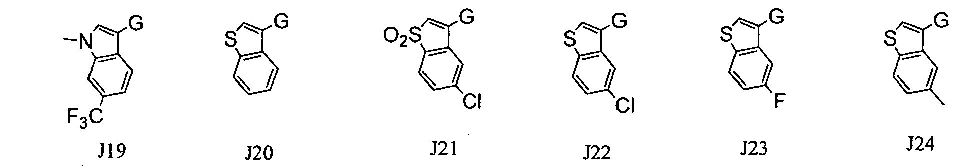

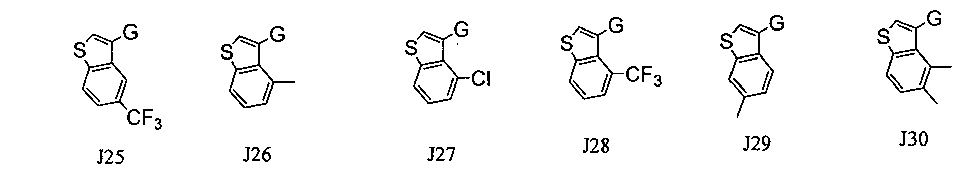

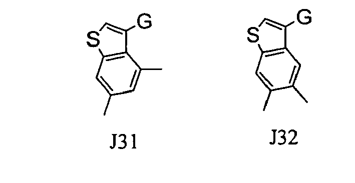

| COMPOUND NO. | R1 | R2 | A | J | M |
|---|---|---|---|---|---|
| 1 | H | H | A1 | J1 | S |
| 2 | H | H | A1 | J2 | S |
| 3 | H | H | A1 | J3 | S |
| 4 | H | H | A1 | J4 | S |
| 5 | H | H | A1 | J5 | S |
| 6 | H | H | A1 | J6 | S |
| 7 | H | H | A1 | J7 | S |
| 8 | H | H | A1 | J8 | S |
| 9 | H | H | A1 | J9 | S |
| 10 | H | H | A1 | J10 | S |
| 11 | H | H | A1 | J11 | S |
| 12 | H | H | A1 | J12 | S |
| 13 | H | H | A1 | J13 | S |
| 14 | H | H | A1 | J14 | S |
| 15 | H | H | A1 | J15 | S |
| 16 | H | H | A1 | J16 | S |
| 17 | H | H | A1 | J17 | S |
| 18 | H | H | A1 | J18 | S |
| 19 | H | H | A1 | J19 | S |
| 20 | H | H | A1 | J20 | S |
| 21 | H | H | A1 | J21 | S |
| 22 | H | H | A1 | J22 | S |
| 23 | H | H | A1 | J23 | S |
| 24 | H | H | A1 | J24 | S |
| 25 | H | H | A1 | J25 | S |
| 26 | H | H | A1 | J26 | S |
| 27 | H | H | A1 | J27 | S |
| 28 | H | H | A1 | J28 | S |
| 29 | H | H | A1 | J29 | S |
| 30 | H | H | A1 | J30 | S |
| 31 | H | H | A1 | J31 | S |
| 32 | H | H | A1 | J32 | S |
| 33 | H | H | A2 | J1 | S |
| 34 | H | H | A2 | J2 | S |
| 35 | H | H | A2 | J3 | S |
| 36 | H | H | A2 | J4 | S |
| 37 | H | H | A2 | J5 | S |
| 38 | H | H | A2 | J6 | S |
| 39 | H | H | A2 | J7 | S |
| 40 | H | H | A2 | J8 | S |
| 41 | H | H | A2 | J9 | S |
| 42 | H | H | A2 | J10 | S |
| 43 | H | H | A2 | J11 | S |
| 44 | H | H | A2 | J12 | S |
| 45 | H | H | A2 | J13 | S |
| 46 | H | H | A2 | J14 | S |
| 47 | H | H | A2 | J15 | S |
| 48 | H | H | A2 | J16 | S |
| 49 | H | H | A2 | J17 | S |
| 50 | H | H | A2 | J18 | S |
| 51 | H | H | A2 | J19 | S |
| 52 | H | H | A2 | J20 | S |
| 53 | H | H | A2 | J21 | S |
| 54 | H | H | A2 | J22 | S |
| 55 | H | H | A2 | J23 | S |
| 56 | H | H | A2 | J24 | S |
| 57 | H | H | A2 | J25 | S |
| 58 | H | H | A2 | J26 | S |
| 59 | H | H | A2 | J27 | S |
| 60 | H | H | A2 | J28 | S |
| 61 | H | H | A2 | J29 | S |
| 62 | H | H | A2 | J30 | S |
| 63 | H | H | A2 | J31 | S |
| 64 | H | . H | A2 | J32 | S |
| 65 | H | H | A3 | J1 | S |
| 66 | H | H | A3 | J2 | S |
| 67 | H | H | A3 | J3 | S |
| 68 | H | H | A3 | J4 | S |
| 69 | H | H | A3 | J5 | S |
| 70 | H | H | A3 | J6 | S |
| 71 | H | H | A3 | J7 | S |
| 72 | H | H | A3 | J8 | S |
| 73 | H | H | A3 | J9 | S |
| 74 | H | H | A3 | J10 | S |
| 75 | H | H | A3 | J11 | S |
| 76 | H | H | A3 | J12 | S |
| 77 | H | H | A3 | J13 | S |
| 78 | H | H | A3 | J14 | S |
| 79 | H | H | A3 | J15 | S |
| 80 | H | H | A3 | J16 | S |
| 81 | H | H | A3 | J17 | S |
| 82 | H | H | A3 | J18 | S |
| 83 | H | H | A3 | J19 | S |
| 84 | H | H | A3 | J20 | S |
| 85 | H | H | A3 | J21 | S |
| 86 | H | H | A3 | J22 | S |
| 87 | H | H | A3 | J23 | S |
| 88 | H | H | A3 | J24 | S |
| 89 | H | H | A3 | J25 | S |
| 90 | H | H | A3 | J26 | S |
| 91 | H | H | A3 | J27 | S |
| 92 | H | H | A3 | J28 | S |
| 93 | H | H | A3 | J29 | S |
| 94 | H | H | A3 | J30 | S |
| 95 | H | H | A3 | J31 | S |
| 96 | H | H | A3 | J32 | S |
| 97 | MeO | H | A1 | J1 | S |
| 98 | MeO | H | A1 | J2 | S |
| 99 | MeO | H | A1 | J3 | S |
| 100 | MeO | H | A1 | J4 | S |
| 101 | MeO | H | A1 | J5 | S |
| 102 | MeO | H | A1 | J6 | S |
| 103 | MeO | H | A1 | J7 | S |
| 104 | MeO | H | A1 | J8 | S |
| 105 | MeO | H | A1 | J9 | S |
| 106 | MeO | H | A1 | J10 | S |
| 107 | MeO | H | A1 | J11 | S |
| 108 | MeO | H | A1 | J12 | S |
| 109 | MeO | H | A1 | J13 | S |
| 110 | MeO | H | A1 | J14 | S |
| 111 | MeO | H | A1 | J15 | S |
| 112 | MeO | H | A1 | J16 | S |
| 113 | MeO | H | A1 | J17 | S |
| 114 | MeO | H | A1 | J18 | S |
| 115 | MeO | H | A1 | J19 | S |
| 116 | MeO | H | A1 | J20 | S |
| 117 | MeO | H | A1 | J21 | S |
| 118 | MeO | H | A1 | J22 | S |
| 119 | MeO | H | A1 | J23 | S |
| 120 | MeO | H | A1 | J24 | S |
| 121 | MeO | H | A1 | J25 | S |
| 122 | MeO | H | A1 | J26 | S |
| 123 | MeO | H | A1 | J27 | S |
| 124 | MeO | H | A1 | J28 | S |
| 125 | MeO | H | A1 | J29 | S |
| 126 | MeO | H | A1 | J30 | S |
| 127 | MeO | H | A1 | J31 | S |
| 128 | MeO | H | A1 | J32 | S |
| 129 | MeO | H | A2 | J1 | S |
| 130 | MeO | H | A2 | J2 | S |
| 131 | MeO | H | A2 | J3 | S |
| 132 | MeO | H | A2 | J4 | S |
| 133 | MeO | H | A2 | J5 | S |
| 134 | MeO | H | A2 | J6 | S |
| 135 | MeO | H | A2 | J7 | S |
| 136 | MeO | H | A2 | J8 | S |
| 137 | MeO | H | A2 | J9 | S |
| 138 | MeO | H | A2 | J10 | S |
| 139 | MeO | H | A2 | J11 | S |
| 140 | MeO | H | A2 | J12 | S |
| 141 | MeO | H | A2 | J13 | S |
| 142 | MeO | H | A2 | J14 | S |
| 143 | MeO | H | A2 | J15 | S |
| 144 | MeO | H | A2 | J16 | S |
| 145 | MeO | H | A2 | J17 | S |
| 146 | MeO | H | A2 | J18 | S |
| 147 | MeO | H | A2 | J19 | S |
| 148 | MeO | H | A2 | J20 | S |
| 149 | MeO | H | A2 | J21 | S |
| 150 | MeO | H | A2 | J22 | S |
| 151 | MeO | H | A2 | J23 | S |
| 152 | MeO | H | A2 | J24 | S |
| 153 | MeO | H | A2 | J25 | S |
| 154 | MeO | H | A2 | J26 | S |
| 155 | MeO | H | A2 | J27 | S |
| 156 | MeO | H | A2 | J28 | S |
| 157 | MeO | H | A2 | J29 | S |
| 158 | MeO | H | A2 | J30 | S |
| 159 | MeO | H | A2 | J31 | S |
| 160 | MeO | H | A2 | J32 | S |
| 161 | MeO | H | A3 | J1 | S |
| 162 | MeO | H | A3 | J2 | S |
| 163 | MeO | H | A3 | J3 | S |
| 164 | MeO | H | A3 | J4 | S |
| 165 | MeO | H | A3 | J5 | S |
| 166 | MeO | H | A3 | J6 | S |
| 167 | MeO | H | A3 | J7 | S |
| 168 | MeO | H | A3 | J8 | S |
| 169 | MeO | H | A3 | J9 | S |
| 170 | MeO | H | A3 | J10 | S |
| 171 | MeO | H | A3 | J11 | S |
| 172' | MeO | H | A3 | J12 | S |
| 173 | MeO | H | A3 | J13 | S |
| 174 | MeO | H | A3 | J14 | S |
| 175 | MeO | H | A3 | J15 | S |
| 176 | MeO | H | A3 | J16 | S |
| 177 | MeO | H | A3 | J17 | S |
| 178 | MeO | H | A3 | J18 | S |
| 179 | MeO | H | A3 | J19 | S |
| 180 | MeO | H | A3 | J20 | S |
| 181 | MeO | H | A3 | J21 | S |
| 182 | MeO | H | A3 | J22 | S |
| 183 | MeO | H | A3 | J23 | S |
| 184 | MeO | H | A3 | J24 | S |
| 185 | MeO | H | A3 | J25 | S |
| 186 | MeO | H | A3 | J26 | S |
| 187 | MeO | H | A3 | J27 | S |
| 188 | MeO | H | A3 | J28 | S |
| 189 | MeO | H | A3 | J29 | S |
| 190 | MeO | H | A3 | J30 | S |
| 191 | MeO | H | A3 | J31 | S |
| 192 | MeO | H | A3 | J32 | S |
| 193 | CN | H | A1 | J1 | S |
| 194 | CN | H | A1 | J2 | S |
| 195 | CN | H | A1 | J3 | S |
| 196 | CN | H | A1 | J4 | S |
| 197 | CN | H | A1 | J5 | S |
| 198 | CN | H | A1 | J6 | S |
| 199 | CN | H | A1 | J7 | S |
| 200 | CN | H | A1 | J8 | S |
| 201 | CN | H | A1 | J9 | S |
| 202 | CN | H | A1 | J10 | S |
| 203 | CN | H | A1 | J11 | S |
| 204 | CN | H | A1 | J12 | S |
| 205 | CN | H | A1 | J13 | S |
| 206 | CN | H | A1 | J14 | S |
| 207 | CN | H | A1 | J15 | S |
| 208 | CN | H | A1 | J16 | S |
| 209 | CN | H | A1 | J17 | S |
| 210 | CN | H | A1 | J18 | S |
| 211 | CN | H | A1 | J19 | S |
| 212 | CN | H | A1 | J20 | S |
| 213 | CN | H | A1 | J21 | S |
| 214 | CN | H | A1 | J22 | S |
| 215 | CN | H | A1 | J23 | S |
| 216 | CN | H | A1 | J24 | S |
| 217 | CN | H | A1 | J25 | S |
| 218 | CN | H | A1 | J26 | S |
| 219 | CN | H | A1 | J27 | S |
| 220 | CN | H | A1 | J28 | S |
| 221 | CN | H | A1 | J29 | S |
| 222 | CN | H | A1 | J30 | S |
| 223 | CN | H | A1 | J31 | S |
| 224 | CN | H | A1 | J32 | S |
| 225 | CN | H | A2 | J1 | S |
| 226 | CN | H | A2 | J2 | S |
| 227 | CN | H | A2 | J3 | S |
| 228 | CN | H | A2 | J4 | S |
| 229 | CN | H | A2 | J5 | S |
| 230 | CN | H | A2 | J6 | S |
| 231 | CN | H | A2 | J7 | S |
| 232 | CN | H | A2 | J8 | S |
| 233 | CN | H | A2 | J9 | S |
| 234 | CN | H | A2 | J10 | S |
| 235 | CN | H | A2 | J11 | S |
| 236 | CN | H | A2 | J12 | S |
| 237 | CN | H | A2 | J13 | S |
| 238 | CN | H | A2 | J14 | S |
| 239 | CN | H | A2 | J15 | S |
| 240 | CN | H | A2 | J16 | S |
| 241 | CN | H | A2 | J17 | S |
| 242 | CN | H | A2 | J18 | S |
| 243 | CN | H | A2 | J19 | S |
| 244 | CN | H | A2 | J20 | S |
| 245 | CN | H | A2 | J21 | S |
| 246 | CN | H | A2 | J22 | S |
| 247 | CN | H | A2 | J23 | S |
| 248 | CN | H | A2 | J24 | S |
| 249 | CN | H | A2 | J25 | S |
| 250 | CN | H | A2 | J26 | S |
| 251 | CN | H | A2 | J27 | S |
| 252 | CN | H | A2 | J28 | S |
| 253 | CN | H | A2 | J29 | S |
| 254 | CN | H | A2 | J30 | S |
| 255 | CN | H | A2 | J31 | S |
| 256 | CN | H | A2 | J32 | S |
| 257 | CN | H | A3 | J1 | S |
| 258 | CN | H | A3 | J2 | S |
| 259 | CN | H | A3 | J3 | S |
| 260 | CN | H | A3 | J4 | S |
| 261 | CN | H | A3 | J5 | S |
| 262 | CN | H | A3 | J6 | S |
| 263 | CN | H | A3 | J7 | S |
| 264 | CN | H | A3 | J8 | S |
| 265 | CN | H | A3 | J9 | S |
| 266 | CN | H | A3 | J10 | S |
| 267 | CN | H | A3 | J11 | S |
| 268 | CN | H | A3 | J12 | S |
| 269 | CN | H | A3 | J13 | S |
| 270 | CN | H | A3 | J14 | S |
| 271 | CN | H | A3 | J15 | S |
| 272 | CN | H | A3 | J16 | S |
| 273 | CN | H | A3 | J17 | S |
| 274 | CN | H | A3 | J18 | S |
| 275 | CN | H | A3 | J19 | S |
| 276 | CN | H | A3 | J20 | S |
| 277 | CN | H | A3 | J21 | S |
| 278 | CN | H | A3 | J22 | S |
| 279 | CN | H | A3 | J23 | S |
| 280 | CN | H | A3 | J24 | S |
| 281 | CN | H | A3 | J25 | S |
| 282 | CN | H | A3 | J26 | S |
| 283 | CN | H | A3 | J27 | S |
| 284 | CN | H | A3 | J28 | S |
| 285 | CN | H | A3 | J29 | S |
| 286 | CN | H | A3 | J30 | S |
| 287 | CN | H | A3 | J31 | S |
| 288 | CN | H | A3 | J32 | S |
| 289 | Me | H | A1 | J1 | S |
| 290 | Me | H | A1 | J2 | S |
| 291 | Me | H | A1 | J3 | S |
| 292 | Me | H | A1 | J4 | S |
| 293 | Me | H | A1 | J5 | S |
| 294 | Me | H | A1 | J6 | S |
| 295 | Me | H | A1 | J7 | S |
| 296 | Me | H | A1 | J8 | S |
| 297 | Me | H | A1 | J9 | S |
| 298 | Me | H | A1 | J10 | S |
| 299 | Me | H | A1 | J11 | S |
| 300 | Me | H | A1 | J12 | S |
| 301 | Me | H | A1 | J13 | S |
| 302 | Me | H | A1 | J14 | S |
| 303 | Me | H | A1 | J15 | S |
| 304 | Me | H | A1 | J16 | S |
| 305 | Me | H | A1 | J17 | S |
| 306 | Me | H | A1 | J18 | S |
| 307 | Me | H | A1 | J19 | S |
| 308 | Me | H | A1 | J20 | S |
| 309 | Me | H | A1 | J21 | S |
| 310 | Me | H | A1 | J22 | S |
| 311 | Me | H | A1 | J23 | S |
| 312 | Me | H | A1 | J24 | S |
| 313 | Me | H | A1 | J25 | S |
| 314 | Me | H | A1 | J26 | S |
| 315 | Me | H | A1 | J27 | S |
| 316 | Me | H | A1 | J28 | S |
| 317 | Me | H | A1 | J29 | S |
| 318 | Me | H | A1 | J30 | S |
| 319 | Me | H | A1 | J31 | S |
| 320 | Me | H | A1 | J32 | S |
| 321 | Me | H | A2 | J1 | S |
| 322 | Me | H | A2 | J2 | S |
| 323 | Me | H | A2 | J3 | S |
| 324 | Me | H | A2 | J4 | S |
| 325 | Me | H | A2 | J5 | S |
| 326 | Me | H | A2 | J6 | S |
| 327 | Me | H | A2 | J7 | S |
| 328 | Me | H | A2 | J8 | S |
| 329 | Me | H | A2 | J9 | S |
| 330 | Me | H | A2 | J10 | S |
| 331 | Me | H | A2 | J11 | S |
| 332 | Me | H | A2 | J12 | S |
| 333 | Me | H | A2 | J13 | S |
| 334 | Me | H | A2 | J14 | S |
| 335 | Me | H | A2 | J15 | S |
| 336 | Me | H | A2 | J16 | S |
| 337 | Me | H | A2 | J17 | S |
| 338 | Me | H | A2 | J18 | S |
| 339 | Me | H | A2 | J19 | S |
| 340 | Me | H | A2 | J20 | S |
| 341 | Me | H | A2 | J21 | S |
| 342 | Me | H | A2 | J22 | S |
| 343 | Me | H | A2 | J23 | S |
| 344 | Me | H | A2 | J24 | S |
| 345 | Me | H | A2 | J25 | S |
| 346 | Me | H | A2 | J26 | S |
| 347 | Me | H | A2 | J27 | S |
| 348 | Me | H | A2 | J28 | S |
| 349 | Me | H | A2 | J29 | S |
| 350 | Me | H | A2 | J30 | S |
| 351 | Me | H | A2 | J31 | S |
| 352 | Me | H | A2 | J32 | S |
| 353 | Me | H | A3 | J1 | S |
| 354 | Me | H | A3 | J2 | S |
| 355 | Me | H | A3 | J3 | S |
| 356 | Me | H | A3 | J4 | S |
| 357 | Me | H | A3 | J5 | S |
| 358 | Me | H | A3 | J6 | S |
| 359 | Me | H | A3 | J7 | S |
| 360 | Me | H | A3 | J8 | S |
| 361 | Me | H | A3 | J9 | S |
| 362 | Me | H | A3 | J10 | S |
| 363 | Me | H | A3 | J11 | S |
| 364 | Me | H | A3 | J12 | S |
| 365 | Me | H | A3 | J13 | S |
| 366 | Me | H | A3 | J14 | S |
| 367 | Me | H | A3 | J15 | S |
| 368 | Me | H | A3 | J16 | S |
| 369 | Me | H | A3 | J17 | S |
| 370 | Me | H | A3 | J18 | S |
| 371 | Me | H | A3 | J19 | S |
| 372 | Me | H | A3 | J20 | S |
| 373 | Me | H | A3 | J21 | S |
| 374 | Me | H | A3 | J22 | S |
| 375 | Me | H | A3 | J23 | S |
| 376 | Me | H | A3 | J24 | S |
| 377 | Me | H | A3 | J25 | S |
| 378 | Me | H | A3 | J26 | S |
| 379 | Me | H | A3 | J27 | S |
| 380 | Me | H | A3 | J28 | S |
| 381 | Me | H | A3 | J29 | S |
| 382 | Me | H | A3 | J30 | S |
| 383 | Me | H | A3 | J31 | S |
| 384 | Me | H | A3 | J32 | S |
| 385 | H | Me | A1 | J1 | S |
| 386 | H | Me | A1 | J2 | S |
| 387 | H | Me | A1 | J3 | S |
| 388 | H | Me | A1 | J4 | S |
| 389 | H | Me | A1 | J5 | S |
| 390 | H | Me | A1 | J6 | S |
| 391 | H | Me | A1 | J7 | S |
| 392 | H | Me | A1 | J8 | S |
| 393 | H | Me | A1 | J9 | S |
| 394 | H | Me | A1 | J10 | S |
| 395 | H | Me | A1 | J11 | S |
| 396 | H | Me | A1 | J12 | S |
| 397 | H | Me | A1 | J13 | S |
| 398 | H | Me | A1 | J14 | S |
| 399 | H | Me | A1 | J15 | S |
| 400 | H | Me | A1 | J16 | S |
| 401 | H | Me | A1 | J17 | S |
| 402 | H | Me | A1 | J18 | S |
| 403 | H | Me | A1 | J19 | S |
| 404 | H | Me | A1 | J20 | S |
| 405 | H | Me | A1 | J21 | S |
| 406 | H | Me | A1 | J22 | S |
| 407 | H | Me | A1 | J23 | S |
| 408 | H | Me | A1 | J24 | S |
| 409 | H | Me | A1 | J25 | S |
| 410 | H | Me | A1 | J26 | S |
| 411 | H | Me | A1 | J27 | S |
| 412 | H | Me | A1 | J28 | S |
| 413 | H | Me | A1 | J29 | S |
| 414 | H | Me | A1 | J30 | S |
| 415 | H | Me | A1 | J31 | S |
| 416 | H | Me | A1 | J32 | S |
| 417 | H | Me | A2 | J1 | S |
| 418 | H | Me | A2 | J2 | S |
| 419 | H | Me | A2 | J3 | S |
| 420 | H | Me | A2 | J4 | S |
| 421 | H | Me | A2 | J5 | S |
| 422 | H | Me | A2 | J6 | S |
| 423 | H | Me | A2 | J7 | S |
| 424 | H | Me | A2 | J8 | S |
| 425 | H | Me | A2 | J9 | S |
| 426 | H | Me | A2 | J10 | S |
| 427 | H | Me | A2 | J11 | S |
| 428 | H | Me | A2 | J12 | S |
| 429 | H | Me | A2 | J13 | S |
| 430 | H | Me | A2 | J14 | S |
| 431 | H | Me | A2 | J15 | S |
| 432 | H | Me | A2 | J16 | S |
| 433 | H | Me | A2 | J17 | S |
| 434 | H | Me | A2 | J18 | S |
| 435 | H | Me | A2 | J19 | S |
| 436 | H | Me | A2 | J20 | S |
| 437 | H | Me | A2 | J21 | S |
| 438 | H | Me | A2 | J22 | S |
| 439 | H | Me | A2 | J23 | S |
| 440 | H | Me | A2 | J24 | S |
| 441 | H | Me | A2 | J25 | S |
| 442 | H | Me | A2 | J26 | S |
| 443 | H | Me | A2 | J27 | S |
| 444 | H | Me | A2 | J28 | S |
| 445 | H | Me | A2 | J29 | S |
| 446 | H | Me | A2 | J30 | S |
| 447 | H | Me | A2 | J31 | S |
| 448 | H | Me | A2 | J32 | S |
| 449 | H | Me | A3 | J1 | S |
| 450 | H | Me | A3 | J2 | S |
| 451 | H | Me | A3 | J3 | S |
| 452 | H | Me | A3 | J4 | S |
| 453 | H | Me | A3 | J5 | S |
| 454 | H | Me | A3 | J6 | S |
| 455 | H | Me | A3 | J7 | S |
| 456 | H | Me | A3 | J8 | S |
| 457 | H | Me | A3 | J9 | S |
| 458 | H | Me | A3 | J10 | S |
| 459 | H | Me | A3 | J11 | S |
| 460 | H | Me | A3 | J12 | S |
| 461 | H | Me | A3 | J13 | S |
| 462 | H | Me | A3 | J14 | S |
| 463 | H | Me | A3 | J15 | S |
| 464 | H | Me | A3 | J16 | S |
| 465 | H | Me | A3 | J17 | S |
| 466 | H | Me | A3 | J18 | S |
| 467 | H | Me | A3 | J19 | S |
| 468 | H | Me | A3 | J20 | S |
| 469 | H | Me | A3 | J21 | S |
| 470 | H | Me | A3 | J22 | S |
| 471 | H | Me | A3 | J23 | S |
| 472 | H | Me | A3 | J24 | S |
| 473 | H | Me | A3 | J25 | S |
| 474 | H | Me | A3 | J26 | S |
| 475 | H | Me | A3 | J27 | S |
| 476 | H | Me | A3 | J28 | S |
| 477 | H | Me | A3 | J29 | S |
| 478 | H | Me | A3 | J30 | S |
| 479 | H | Me | A3 | J31 | S |
| 480 | H | Me | A3 | J32 | S |
| 481 | Me | Me | A1 | J1 | S |
| 482 | Me | Me | A1 | J2 | S |
| 483 | Me | Me | A1 | J3 | S |
| 484 | Me | Me | A1 | J4 | S |
| 485 | Me | Me | A1 | J5 | S |
| 486 | Me | Me | A1 | J6 | S |
| 487 | Me | Me | A1 | J7 | S |
| 488 | Me | Me | A1 | J8 | S |
| 489 | Me | Me | A1 | J9 | S |
| 490 | Me | Me | A1 | J10 | S |
| 491 | Me | Me | A1 | J11 | S |
| 492 | Me | Me | A1 | J12 | S |
| 493 | Me | Me | A1 | J13 | S |
| 494 | Me | Me | A1 | J14 | S |
| 495 | Me | Me | A1 | J15 | S |
| 496 | Me | Me | A1 | J16 | S |
| 497 | Me | Me | A1 | J17 | S |
| 498 | Me | Me | A1 | J18 | S |
| 499 | Me | Me | A1 | J19 | S |
| 500 | Me | Me | A1 | J20 | S |
| 501 | Me | Me | A1 | J21 | S |
| 502 | Me | Me | A1 | J22 | S |
| 503 | Me | Me | A1 | J23 | S |
| 504 | Me | Me | A1 | J24 | S |
| 505 | Me | Me | A1 | J25 | S |
| 506 | Me | Me | A1 | J26 | S |
| 507 | Me | Me | A1 | J27 | S |
| 508 | Me | Me | A1 | J28 | S |
| 509 | Me | Me | A1 | J29 | S |
| 510 | Me | Me | A1 | J30 | S |
| 511 | Me | Me | A1 | J31 | S |
| 512 | Me | Me | A1 | J32 | S |
| 513 | Me | Me | A2 | J1 | S |
| 514 | Me | Me | A2 | J2 | S |
| 515 | Me | Me | A2 | J3 | S |
| 516 | Me | Me | A2 | J4 | S |
| 517 | Me | Me | A2 | J5 | S |
| 518 | Me | Me | A2 | J6 | S |
| 519 | Me | Me | A2 | J7 | S |
| 520 | Me | Me | A2 | J8 | S |
| 521 | Me | Me | A2 | J9 | S |
| 522 | Me | Me | A2 | J10 | S |
| 523 | Me | Me | A2 | J11 | S |
| 524 | Me | Me | A2 | J12 | S |
| 525 | Me | Me | A2 | J13 | S |
| 526 | Me | Me | A2 | J14 | S |
| 527 | Me | Me | A2 | J15 | S |
| 528 | Me | Me | A2 | J16 | S |
| 529 | Me | Me | A2 | J17 | S |
| 530 | Me | Me | A2 | J18 | S |
| 531 | Me | Me | A2 | J19 | S |
| 532 | Me | Me | A2 | J20 | S |
| 533 | Me | Me | A2 | J21 | S |
| 534 | Me | Me | A2 | J22 | S |
| 535 | Me | Me | A2 | J23 | S |
| 536 | Me | Me | A2 | J24 | S |
| 537 | Me | Me | A2 | J25 | S |
| 538 | Me | Me | A2 | J26 | S |
| 539 | Me | Me | A2 | J27 | S |
| 540 | Me | Me | A2 | J28 | S |
| 541 | Me | Me | A2 | J29 | S |
| 542 | Me | Me | A2 | J30 | S |
| 543 | Me | Me | A2 | J31 | S |
| 544 | Me | Me | A2 | J32 | S |
| 545 | Me | Me | A3 | J1 | S |
| 546 | Me | Me | A3 | J2 | S |
| 547 | Me | Me | A3 | J3 | S |
| 548 | Me | Me | A3 | J4 | S |
| 549 | Me | Me | A3 | J5 | S |
| 550 | Me | Me | A3 | J6 | S |
| 551 | Me | Me | A3 | J7 | S |
| 552 | Me | Me | A3 | J8 | S |
| 553 | Me | Me | A3 | J9 | S |
| 554 | Me | Me | A3 | J10 | S |
| 555 | Me | Me | A3 | J11 | S |
| 556 | Me | Me | A3 | J12 | S |
| 557 | Me | Me | A3 | J13 | S |
| 558 | Me | Me | A3 | J14 | S |
| 559 | Me | Me | A3 | J15 | S |
| 560 | Me | Me | A3 | J16 | S |
| 561 | Me | Me | A3 | J17 | S |
| 562 | Me | Me | A3 | J18 | S |
| 563 | Me | Me | A3 | J19 | S |
| 564 | Me | Me | A3 | J20 | S |
| 565 | Me | Me | A3 | J21 | S |
| 566 | Me | Me | A3 | J22 | S |
| 567 | Me | Me | A3 | J23 | S |
| 568 | Me | Me | A3 | J24 | S |
| 569 | Me | Me | A3 | J25 | S |
| 570 | Me | Me | A3 | J26 | S |
| 571 | Me | Me | A3 | J27 | S |
| 572 | Me | Me | A3 | J28 | S |
| 573 | Me | Me | A3 | J29 | S |
| 574 | Me | Me | A3 | J30 | S |
| 575 | Me | Me | A3 | J31 | S |
| 576 | Me | Me | A3 | J32 | S |
| 577 | Cl | Cl | A1 | J1 | S |
| 578 | Cl | Cl | A1 | J2 | S |
| 579 | Cl | Cl | A1 | J3 | S |
| 580 | Cl | Cl | A1 | J4 | S |
| 581 | Cl | Cl | A1 | J5 | S |
| 582 | Cl | Cl | A1 | J6 | S |
| 583 | Cl | Cl | A1 | J7 | S |
| 584 | Cl | Cl | A1 | J8 | S |
| 585 | Cl | Cl | A1 | J9 | S |
| 586 | Cl | Cl | A1 | J10 | S |
| 587 | Cl | Cl | A1 | J11 | S |
| 588 | Cl | Cl | A1 | J12 | S |
| 589 | Cl | Cl | A1 | J13 | S |
| 590 | Cl | Cl | A1 | J14 | S |
| 591 | Cl | Cl | A1 | J15 | S |
| 592 | Cl | Cl | A1 | J16 | S |
| 593 | Cl | Cl | A1 | J17 | S |
| 594 | Cl | Cl | A1 | J18 | S |
| 595 | Cl | Cl | A1 | J19 | S |
| 596 | Cl | Cl | A1 | J20 | S |
| 597 | Cl | Cl | A1 | J21 | S |
| 598 | Cl | Cl | A1 | J22 | S |
| 599 | Cl | Cl | A1 | J23 | S |
| 600 | Cl | Cl | A1 | J24 | S |
| 601 | Cl | Cl | A1 | J25 | S |
| 602 | Cl | Cl | A1 | J26 | S |
| 603 | Cl | Cl | A1 | J27 | S |
| 604 | Cl | Cl | A1 | J28 | S |
| 605 | Cl | Cl | A1 | J29 | S |
| 606 | Cl | Cl | A1 | J30 | S |
| 607 | Cl | Cl | A1 | J31 | S |
| 608 | Cl | Cl | A1 | J32 | S |
| 609 | Cl | Cl | A2 | J1 | S |
| 610 | Cl | Cl | A2 | J2 | S |
| 611 | Cl | Cl | A2 | J3 | S |
| 612 | Cl | Cl | A2 | J4 | S |
| 613 | Cl | Cl | A2 | J5 | S |
| 614 | Cl | Cl | A2 | J6 | S |
| 615 | Cl | Cl | A2 | J7 | S |
| 616 | Cl | Cl | A2 | J8 | S |
| 617 | Cl | Cl | A2 | J9 | S |
| 618 | Cl | Cl | A2 | J10 | S |
| 619 | Cl | Cl | A2 | J11 | S |
| 620 | Cl | Cl | A2 | J12 | S |
| 621 | Cl | Cl | A2 | J13 | S |
| 622 | Cl | Cl | A2 | J14 | S |
| 623 | Cl | Cl | A2 | J15 | S |
| 624 | Cl | Cl | A2 | J16 | S |
| 625 | Cl | Cl | A2 | J17 | S |
| 626 | Cl | Cl | A2 | J18 | S |
| 627 | Cl | Cl | A2 | J19 | S |
| 628 | Cl | Cl | A2 | J20 | S |
| 629 | Cl | Cl | A2 | J21 | S |
| 630 | Cl | Cl | A2 | J22 | S |
| 631 | Cl | Cl | A2 | J23 | S |
| 632 | Cl | Cl | A2 | J24 | S |
| 633 | Cl | Cl | A2 | J25 | S |
| 634 | Cl | Cl | A2 | J26 | S |
| 635 | Cl | Cl | A2 | J27 | S |
| 636 | Cl | Cl | A2 | J28 | S |
| 637 | Cl | Cl | A2 | J29 | S |
| 638 | Cl | Cl | A2 | J30 | S |
| 639 | Cl | Cl | A2 | J31 | S |
| 640 | Cl | Cl | A2 | J32 | S |
| 641 | Cl | Cl | A3 | J1 | S |
| 642 | Cl | Cl | A3 | J2 | S |
| 643 | Cl | Cl | A3 | J3 | S |
| 644 | Cl | Cl | A3 | J4 | S |
| 645 | Cl | Cl | A3 | J5 | S |
| 646 | Cl | Cl | A3 | J6 | S |
| 647 | Cl | Cl | A3 | J7 | S |
| 648 | Cl | Cl | A3 | J8 | S |
| 649 | Cl | Cl | A3 | J9 | S |
| 650 | Cl | Cl | A3 | J10 | S |
| 651 | Cl | Cl | A3 | J11 | S |
| 652 | Cl | Cl | A3 | J12 | S |
| 653 | Cl | Cl | A3 | J13 | S |
| 654 | Cl | Cl | A3 | J14 | S |
| 655 | Cl | Cl | A3 | J15 | S |
| 656 | Cl | Cl | A3 | J16 | S |
| 657 | Cl | Cl | A3 | J17 | S |
| 658 | Cl | Cl | A3 | J18 | S |
| 659 | Cl | Cl | A3 | J19 | S |
| 660 | Cl | Cl | A3 | J20 | S |
| 661 | Cl | Cl | A3 | J21 | S |
| 662 | Cl | Cl | A3 | J22 | S |
| 663 | Cl | Cl | A3 | J23 | S |
| 664 | Cl | Cl | A3 | J24 | S |
| 665 | Cl | Cl | A3 | J25 | S |
| 666 | Cl | Cl | A3 | J26 | S |
| 667 | Cl | Cl | A3 | J27 | S |
| 668 | Cl | Cl | A3 | J28 | S |
| 669 | Cl | Cl | A3 | J29 | S |
| 670 | Cl | Cl | A3 | J30 | S |
| 671 | Cl | Cl | A3 | J31 | S |
| 672 | Cl | Cl | A3 | J32 | S |
| 673 | H | H | A1 | J1 | - |
| 674 | H | H | A1 | J2 | - |
| 675 | H | H | A1 | J3 | - |
| 676 | H | H | A1 | J4 | - |
| 677 | H | H | A1 | J5 | - |
| 678 | H | H | A1 | J6 | - |
| 679 | H | H | A1 | J7 | - |
| 680 | H | H | A1 | J8 | - |
| 681 | H | H | A1 | J9 | - |
| 682 | H | H | A1 | J10 | - |
| 683 | H | H | A1 | J11 | - |
| 684 | H | H | A1 | J12 | - |
| 685 | H | H | A1 | J13 | - |
| 686 | H | H | A1 | J14 | - |
| 687 | H | H | A1 | J15 | - |
| 688 | H | H | A1 | J16 | - |
| 689 | H | H | A1 | J17 | - |
| 690 | H | H | A1 | J18 | - |
| 691 | H | H | A1 | J19 | - |
| 692 | H | H | A1 | J20 | - |
| 693 | H | H | A1 | J21 | - |
| 694 | H | H | A1 | J22 | - |
| 695 | H | H | A1 | J23 | - |
| 696 | H | H | A1 | J24 | - |
| 697 | H | H | A1 | J25 | - |
| 698 | H | H | A1 | J26 | - |
| 699 | H | H | A1 | J27 | - |
| 700 | H | H | A1 | J28 | - |
| 701 | H | H | A1 | J29 | - |
| 702 | H | H | A1 | J30 | - |
| 703 | H | H | A1 | J31 | - |
| 704 | H | H | A1 | J32 | - |
| 705 | H | H | A2 | J1 | - |
| 706 | H | H | A2 | J2 | - |
| 707 | H | H | A2 | J3 | - |
| 708 | H | H | A2 | J4 | - |
| 709 | H | H | A2 | J5 | - |
| 710 | H | H | A2 | J6 | - |
| 711 | H | H | A2 | J7 | - |
| 712 | H | H | A2 | J8 | - |
| 713 | H | H | A2 | J9 | - |
| 714 | H | H | A2 | J10 | - |
| 715 | H | H | A2 | J11 | - |
| 716 | H | H | A2 | J12 | - |
| 717 | H | H | A2 | J13 | - |
| 718 | H | H | A2 | J14 | - |
| 719 | H | H | A2 | J15 | - |
| 720 | H | H | A2 | J16 | - |
| 721 | H | H | A2 | J17 | - |
| 722 | H | H | A2 | J18 | - |
| 723 | H | H | A2 | J19 | - |
| 724 | H | H | A2 | J20 | - |
| 725 | H | H | A2 | J21 | - |
| 726 | H | H | A2 | J22 | - |
| 727 | H | H | A2 | J23 | - |
| 728 | H | H | A2 | J24 | - |
| 729 | H | H | A2 | J25 | - |
| 730 | H | H | A2 | J26 | - |
| 731 | H | H | A2 | J27 | - |
| 732 | H | H | A2 | J28 | - |
| 733 | H | H | A2 | J29 | - |
| 734 | H | H | A2 | J30 | - |
| 735 | H | H | A2 | J31 | - |
| 736 | H | H | A2 | J32 | - |
| 737 | H | H | A3 | J1 | - |
| 738 | H | H | A3 | J2 | - |
| 739 | H | H | A3 | J3 | - |
| 740 | H | H | A3 | J4 | - |
| 741 | H | H | A3 | J5 | - |
| 742 | H | H | A3 | J6 | - |
| 743 | H | H | A3 | J7 | - |
| 744 | H | H | A3 | J8 | - |
| 745 | H | H | A3 | J9 | - |
| 746 | H | H | A3 | J10 | - |
| 747 | H | H | A3 | J11 | - |
| 748 | H | H | A3 | J12 | - |
| 749 | H | H | A3 | J13 | - |
| 750 | H | H | A3 | J14 | - |
| 751 | H | H | A3 | J15 | - |
| 752 | H | H | A3 | J16 | - |
| 753 | H | H | A3 | J17 | - |
| 754 | H | H | A3 | J18 | - |
| 755 | H | H | A3 | J19 | - |
| 756 | H | H | A3 | J20 | - |
| 757 | H | H | A3 | J21 | - |
| 758 | H | H | A3 | J22 | - |
| 759 | H | H | A3 | J23 | - |
| 760 | H | H | A3 | J24 | - |
| 761 | H | H | A3 | J25 | - |
| 762 | H | H | A3 | J26 | - |
| 763 | H | H | A3 | J27 | - |
| 764 | H | H | A3 | J28 | - |
| 765 | H | H | A3 | J29 | - |
| 766 | H | H | A3 | J30 | - |
| 767 | H | H | A3 | J31 | - |
| 768 | H | H | A3 | J32 | - |
| 769 | MeO | H | A1 | J1 | - |
| 770 | MeO | H | A1 | J2 | - |
| 771 | MeO | H | A1 | J3 | - |
| 772 | MeO | H | A1 | J4 | - |
| 773 | MeO | H | A1 | J5 | - |
| 774 | MeO | H | A1 | J6 | - |
| 775 | MeO | H | A1 | J7 | - |
| 776 | MeO | H | A1 | J8 | - |
| 777 | MeO | H | A1 | J9 | - |
| 778 | MeO | H | A1 | J10 | - |
| 779 | MeO | H | A1 | J11 | - |
| 780 | MeO | H | A1 | J12 | - |
| 781 | MeO | H | A1 | J13 | - |
| 782 | MeO | H | A1 | J14 | - |
| 783 | MeO | H | A1 | J15 | - |
| 784 | MeO | H | A1 | J16 | - |
| 785 | MeO | H | A1 | J17 | - |
| 786 | MeO | H | A1 | J18 | - |
| 787 | MeO | H | A1 | J19 | - |
| 788 | MeO | H | A1 | J20 | - |
| 789 | MeO | H | A1 | J21 | - |
| 790 | MeO | H | A1 | J22 | - |
| 791 | MeO | H | A1 | J23 | - |
| 792 | MeO | H | A1 | J24 | - |
| 793 | MeO | H | A1 | J25 | - |
| 794 | MeO | H | A1 | J26 | - |
| 795 | MeO | H | A1 | J27 | - |
| 796 | MeO | H | A1 | J28 | - |
| 797 | MeO | H | A1 | J29 | - |
| 798 | MeO | H | A1 | J30 | - |
| 799 | MeO | H | A1 | J31 | - |
| 800 | MeO | H | A1 | J32 | - |
| 801 | Me0 | H | A2 | J1 | - |
| 802 | MeO | H | A2 | J2 | - |
| 803 | MeO | H | A2 | J3 | - |
| 804 | MeO | H | A2 | J4 | - |
| 805 | MeO | H | A2 | J5 | - |
| 806 | MeO | H | A2 | J6 | - |
| 807 | MeO | H | A2 | J7 | - |
| 808 | MeO | H | A2 | J8 | - |
| 809 | MeO | H | A2 | J9 | - |
| 810 | MeO | H | A2 | J10 | - |
| 811 | MeO | H | A2 | J11 | - |
| 812 | MeO | H | A2 | J12 | - |
| 813 | MeO | H | A2 | J13 | - |
| 814 | MeO | H | A2 | J14 | - |
| 815 | MeO | H | A2 | J15 | - |
| 816 | MeO | H | A2 | J16 | - |
| 817 | MeO | H | A2 | J17 | - |
| 818 | MeO | H | A2 | J18 | - |
| 819 | MeO | H | A2 | J19 | - |
| 820 | MeO | H | A2 | J20 | - |
| 821 | MeO | H | A2 | J21 | - |
| 822 | MeO | H | A2 | J22 | - |
| 823 | MeO | H | A2 | J23 | - |
| 824 | MeO | H | A2 | J24 | - |
| 825 | MeO | H | A2 | J25 | - |
| 826 | MeO | H | A2 | J26 | - |
| 827 | MeO | H | A2 | J27 | - |
| 828 | MeO | H | A2 | J28 | - |
| 829 | MeO | H | A2 | J29 | - |
| 830 | MeO | H | A2 | J30 | - |
| 831 | MeO | H | A2 | J31 | - |
| 832 | MeO | H | A2 | J32 | - |
| 833 | MeO | H | A3 | J1 | - |
| 834 | MeO | H | A3 | J2 | - |
| 835 | MeO | H | A3 | J3 | - |
| 836 | MeO | H | A3 | J4 | - |
| 837 | MeO | H | A3 | J5 | - |
| 838 | MeO | H | A3 | J6 | - |
| 839 | MeO | H | A3 | J7 | - |
| 840 | MeO | H | A3 | J8 | - |
| 841 | MeO | H | A3 | J9 | - |
| 842 | MeO | H | A3 | J10 | - |
| 843 | MeO | H | A3 | J11 | - |
| 844 | MeO | H | A3 | J12 | - |
| 845 | MeO | H | A3 | J13 | - |
| 846 | MeO | H | A3 | J14 | - |
| 847 | MeO | H | A3 | J15 | - |
| 848 | MeO | H | A3 | J16 | - |
| 849 | MeO | H | A3 | J17 | - |
| 850 | MeO | H | A3 | J18 | - |
| 851 | MeO | H | A3 | J19 | - |
| 852 | MeO | H | A3 | J20 | - |
| 853 | MeO | H | A3 | J21 | - |
| 854 | MeO | H | A3 | J22 | , - |
| 855 | MeO | H | A3 | J23 | - |
| 856 | MeO | H | A3 | J24 | - |
| 857 | MeO | H | A3 | J25 | - |
| 858 | MeO | H | A3 | J26 | - |
| 859 | MeO | H | A3 | J27 | - |
| 860 | MeO | H | A3 | J28 | - |
| 861 | MeO | H | A3 | J29 | - |
| 862 | MeO | H | A3 | J30 | - |
| 863 | MeO | H | A3 | J31 | - |
| 864 | MeO | H | A3 | J32 | - |
| 865 | CN | H | A1 | J1 | - |
| 866 | CN | H | A1 | J2 | - |
| 867 | CN | H | A1 | J3 | - |
| 868 | CN | H | A1 | J4 | - |
| 869 | CN | H | A1 | J5 | - |
| 870 | CN | H | A1 | J6 | - |
| 871 | CN | H | A1 | J7 | - |
| 872 | CN | H | A1 | J8 | - |
| 873 | CN | H | A1 | J9 | - |
| 874 | CN | H | A1 | J10 | - |
| 875 | CN | H | A1 | J11 | - |
| 876 | CN | H | A1 | J12 | - |
| 877 | CN | H | A1 | J13 | - |
| 878 | CN | H | A1 | J14 | - |
| 879 | CN | H | A1 | J15 | - |
| 880 | CN | H | A1 | J16 | - |
| 881 | CN | H | A1 | J17 | - |
| 882 | CN | H | A1 | J18 | - |
| 883 | CN | H | A1 | J19 | - |
| 884 | CN | H | A1 | J20 | - |
| 885 | CN | H | A1 | J21 | - |
| 886 | CN | H | A1 | J22 | - |
| 887 | CN | H | A1 | J23 | - |
| 888 | CN | H | A1 | J24 | - |
| 889 | CN | H | A1 | J25 | - |
| 890 | CN | H | A1 | J26 | - |
| 891 | CN | H | A1 | J27 | - |
| 892 | CN | H | A1 | J28 | - |
| 893 | CN | H | A1 | J29 | - |
| 894 | CN | H | A1 | J30 | - |
| 895 | CN | H | A1 | J31 | - |
| 896 | CN | H | A1 | J32 | - |
| 897 | CN | H | A2 | J1 | - |
| 898 | CN | H | A2 | J2 | - |
| 899 | CN | H | A2 | J3 | - |
| 900 | CN | H | A2 | J4 | - |
| 901 | CN | H | A2 | J5 | - |
| 902 | CN | H | A2 | J6 | - |
| 903 | CN | H | A2 | J7 | - |
| 904 | CN | H | A2 | J8 | - |
| 905 | CN | H | A2 | J9 | - |
| 906 | CN | H | A2 | J10 | - |
| 907 | CN | H | A2 | J11 | - |
| 908 | CN | H | A2 | J12 | - |
| 909 | CN | H | A2 | J13 | - |
| 910 | CN | H | A2 | J14 | - |
| 911 | CN | H | A2 | J15 | - |
| 912 | CN | H | A2 | J16 | - |
| 913 | CN | H | A2 | J17 | - |
| 914 | CN | H | A2 | J18 | - |
| 915 | CN | H | A2 | J19 | - |
| 916 | CN | H | A2 | J20 | - |
| 917 | CN | H | A2 | J21 | - |
| 918 | CN | H | A2 | J22 | - |
| 919 | CN | H | A2 | J23 | - |
| 920 | CN | H | A2 | J24 | - |
| 921 | CN | H | A2 | J25 | - |
| 922 | CN | H | A2 | J26 | - |
| 923 | CN | H | A2 | J27 | - |
| 924 | CN | H | A2 | J28 | - |
| 925 | CN | H | A2 | J29 | - |
| 926 | CN | H | A2 | J30 | - |
| 927 | CN | H | A2 | J31 | - |
| 928 | CN | H | A2 | J32 | - |
| 929 | CN | H | A3 | J1 | - |
| 930 | CN | H | A3 | J2 | - |
| 931 | CN | H | A3 | J3 | - |
| 932 | CN | H | A3 | J4 | - |
| 933 | CN | H | A3 | J5 | - |
| 934 | CN | H | A3 | J6 | - |
| 935 | CN | H | A3 | J7 | - |
| 936 | CN | H | A3 | J8 | - |
| 937 | CN | H | A3 | J9 | - |
| 938 | CN | H | A3 | J10 | - |
| 939 | CN | H | A3 | J11 | - |
| 940 | CN | H | A3 | J12 | - |
| 941 | CN | H | A3 | J13 | - |
| 942 | CN | H | A3 | J14 | - |
| 943 | CN | H | A3 | J15 | - |
| 944 | CN | H | A3 | J16 | - |
| 945 | CN | H | A3 | J17 | - |
| 946 | CN | H | A3 | J18 | - |
| 947 | CN | H | A3 | J19 | - |
| 948 | CN | H | A3 | J20 | - |
| 949 | CN | H | A3 | J21 | - |
| 950 | CN | H | A3 | J22 | - |
| 951 | CN | H | A3 | J23 | - |
| 952 | CN | H | A3 | J24 | - |
| 953 | CN | H | A3 | J25 | - |
| 954 | CN | H | A3 | J26 | - |
| 955 | CN | H | A3 | J27 | - |
| 956 | CN | H | A3 | J28 | - |
| 957 | CN | H | A3 | J29 | - |
| 958 | CN | H | A3 | J30 | - |
| 959 | CN | H | A3 | J31 | - |
| 960 | CN | H | A3 | J32 | - |
| 961 | Me | Me | A1 | J1 | - |
| 962 | Me | Me | A1 | J2 | - |
| 963 | Me | Me | A1 | J3 | - |
| 964 | Me | Me | A1 | J4 | - |
| 965 | Me | Me | A1 | J5 | - |
| 966 | Me | Me | A1 | J6 | - |
| 967 | Me | Me | A1 | J7 | - |
| 968 | Me | Me | A1 | J8 | - |
| 969 | Me | Me | A1 | J9 | - |
| 970 | Me | Me | A1 | J10 | - |
| 971 | Me | Me | A1 | J11 | - |
| 972 | Me | Me | A1 | J12 | - |
| 973 | Me | Me | A1 | J13 | - |
| 974 | Me | Me | A1 | J14 | - |
| 975 | Me | Me | A1 | J15 | - |
| 976 | Me | Me | A1 | J16 | - |
| 977 | Me | Me | A1 | J17 | - |
| 978 | Me | Me | A1 | J18 | - |
| 979 | Me | Me | A1 | J19 | - |
| 980 | Me | Me | A1 | J20 | - |
| 981 | Me | Me | A1 | J21 | - |
| 982 | Me | Me | A1 | J22 | - |
| 983 | Me | Me | A1 | J23 | - |
| 984 | Me | Me | A1 | J24 | - |
| 985 | Me | Me | A1 | J25 | - |
| 986 | Me | Me | A1 | J26 | - |
| 987 | Me | Me | A1 | J27 | - |
| 988 | Me | Me | A1 | J28 | - |
| 989 | Me | Me | A1 | J29 | - |
| 990 | Me | Me | A1 | J30 | - |
| 991 | Me | Me | A1 | J31 | - |
| 992 | Me | Me | A1 | J32 | - |
| 993 | Me | Me | A2 | J1 | - |
| 994 | Me | Me | A2 | J2 | - |
| 995 | Me | Me | A2 | J3 | - |
| 996 | Me | Me | A2 | J4 | - |
| 997 | Me | Me | A2 | J5 | - |
| 998 | Me | Me | A2 | J6 | - |
| 999 | Me | Me | A2 | J7 | - |
| 1000 | Me | Me | A2 | J8 | - |
| 1001 | Me | Me | A2 | J9 | - |
| 1002 | Me | Me | A2 | J10 | - |
| 1003 | Me | Me | A2 | J11 | - |
| 1004 | Me | Me | A2 | J12 | - |
| 1005 | Me | Me | A2 | J13 | - |
| 1006 | Me | Me | A2 | J14 | - |
| 1007 | Me | Me | A2 | J15 | - |
| 1008 | Me | Me | A2 | J16 | - |
| 1009 | Me | Me | A2 | J17 | - |
| 1010 | Me | Me | A2 | J18 | - |
| 1011 | Me | Me | A2 | J19 | - |
| 1012 | Me | Me | A2 | J20 | - |
| 1013 | Me | Me | A2 | J21 | - |
| 1014 | Me | Me | A2 | J22 | - |
| 1015 | Me | Me | A2 | J23 | - |
| 1016 | Me | Me | A2 | J24 | - |
| 1017 | Me | Me | A2 | J25 | - |
| 1018 | Me | Me | A2 | J26 | - |
| 1019 | Me | Me | A2 | J27 | - |
| 1020 | Me | Me | A2 | J28 | - |
| 1021 | Me | Me | A2 | J29 | - |
| 1022 | Me | Me | A2 | J30 | - |
| 1023 | Me | Me | A2 | J31 | - |
| 1024 | Me | Me | A2 | J32 | - |
| 1025 | Me | Me | A3 | J1 | - |
| 1026 | Me | Me | A3 | J2 | - |
| 1027 | Me | Me | A3 | J3 | - |
| 1028 | Me | Me | A3 | J4 | - |
| 1029 | Me | Me | A3 | J5 | - |
| 1030 | Me | Me | A3 | J6 | - |
| 1031 | Me | Me | A3 | J7 | - |
| 1032 | Me | Me | A3 | J8 | - |
| 1033 | Me | Me | A3 | J9 | - |
| 1034 | Me | Me | A3 | J10 | - |
| 1035 | Me | Me | A3 | J11 | - |
| 1036 | Me | Me | A3 | J12 | - |
| 1037 | Me | Me | A3 | J13 | - |
| 1038 | Me | Me | A3 | J14 | - |
| 1039 | Me | Me | A3 | J15 | - |
| 1040 | Me | Me | A3 | J16 | - |
| 1041 | Me | Me | A3 | J17 | - |
| 1042 | Me | Me | A3 | J18 | - |
| 1043 | Me | Me | A3 | J19 | - |
| 1044 | Me | Me | A3 | J20 | - |
| 1045 | Me | Me | A3 | J21 | - |
| 1046 | Me | Me | A3 | J22 | - |
| 1047 | Me | Me | A3 | J23 | - |
| 1048 | Me | Me | A3 | J24 | - |
| 1049 | Me | Me | A3 | J25 | - |
| 1050 | Me | Me | A3 | J26 | - |
| 1051 | Me | Me | A3 | J27 | - |
| 1052 | Me | Me | A3 | J28 | - |
| 1053 | Me | Me | A3 | J29 | - |
| 1054 | Me | Me | A3 | J30 | - |
| 1055 | Me | Me | A3 | J31 | - |
| 1056 | Me | Me | A3 | J32 | - |
| 1057 | H | MeO | A1 | J1 | S |
| 1058 | H | MeO | A1 | J2 | S |
| 1059 | H | MeO | A1 | J3 | S |
| 1060 | H | MeO | A1 | J4 | S |
| 1061 | H | MeO | A1 | J5 | S |
| 1062 | H | MeO | A1 | J6 | S |
| 1063 | H | MeO | A1 | J7 | S |
| 1064 | H | MeO | A1 | J8 | S |
| 1065 | H | MeO | A1 | J9 | S |
| 1066 | H | MeO | A1 | J10 | S |
| 1067 | H | MeO | A1 | J11 | S |
| 1068 | H | MeO | A1 | J12 | S |
| 1069 | H | MeO | A1 | J13 | S |
| 1070 | H | MeO | A1 | J14 | S |
| 1071 | H | MeO | A1 | J15 | S |
| 1072 | H | MeO | A1 | J16 | S |
| 1073 | H | MeO | A1 | J17 | S |
| 1074 | H | MeO | A1 | J18 | S |
| 1075 | H | MeO | A1 | J19 | S |
| 1076 | H | MeO | A1 | J20 | S |
| 1077 | H | MeO | A1 | J21 | S |
| 1078 | H | MeO | A1 | J22 | S |
| 1079 | H | MeO | A1 | J23 | S |
| 1080 | H | MeO | A1 | J24 | S |
| 1081 | H | MeO | A1 | J25 | S |
| 1082 | H | MeO | A1 | J26 | S |
| 1083 | H | MeO | A1 | J27 | S |
| 1084 | H | MeO | A1 | J28 | S |
| 1085 | H | MeO | A1 | J29 | S |
| 1086 | H | MeO | A1 | J30 | S |
| 1087 | H | MeO | A1 | J31 | S |
| 1088 | H | MeO | A1 | J32 | S |
| 1089 | H | MeO | A2 | J1 | S |
| 1090 | H | MeO | A2 | J2 | S |
| 1091 | H | MeO | A2 | J3 | S |
| 1092 | H | MeO | A2 | J4 | S |
| 1093 | H | MeO | A2 | J5 | S |
| 1094 | H | MeO | A2 | J6 | S |
| 1095 | H | MeO | A2 | J7 | S |
| 1096 | H | MeO | A2 | J8 | S |
| 1097 | H | MeO | A2 | J9 | S |
| 1098 | H | MeO | A2 | J10 | S |
| 1099 | H | MeO | A2 | J11 | S |
| 1100 | H | MeO | A2 | J12 | S |
| 1101 | H | MeO | A2 | J13 | S |
| 1102 | H | MeO | A2 | J14 | S |
| 1103 | H | MeO | A2 | J15 | S |
| 1104 | H | MeO | A2 | J16 | S |
| 1105 | H | MeO | A2 | J17 | S |
| 1106 | H | MeO | A2 | J18 | S |
| 1107 | H | MeO | A2 | J19 | S |
| 1108 | H | MeO | A2 | J20 | S |
| 1109 | H | MeO | A2 | J21 | S |
| 1110 | H | MeO | A2 | J22 | S |
| 1111 | H | MeO | A2 | J23 | S |
| 1112 | H | MeO | A2 | J24 | S |
| 1113 | H | MeO | A2 | J25 | S |
| 1114 | H | MeO | A2 | J26 | S |
| 1115 | H | MeO | A2 | J27 | S |
| 1116 | H | MeO | A2 | J28 | S |
| 1117 | H | MeO | A2 | J29 | S |
| 1118 | H | MeO | A2 | J30 | S |
| 1119 | H | MeO | A2 | J31 | S |
| 1120 | H | MeO | A2 | J32 | S |
| 1121 | H | MeO | A3 | J1 | S |
| 1122 | H | MeO | A3 | J2 | S |
| 1123 | H | MeO | A3 | J3 | S |
| 1124 | H | MeO | A3 | J4 | S |
| 1125 | H | MeO | A3 | J5 | S |
| 1126 | H | MeO | A3 | J6 | S |
| 1127 | H | MeO | A3 | J7 | S |
| 1128 | H | MeO | A3 | J8 | S |
| 1129 | H | MeO | A3 | J9 | S |
| 1130 | H | MeO | A3 | J10 | S |
| 1131 | H | MeO | A3 | J11 | S |
| 1132 | H | MeO | A3 | J12 | S |
| 1133 | H | MeO | A3 | J13 | S |
| 1134 | H | MeO | A3 | J14 | S |
| 1135 | H | MeO | A3 | J15 | S |
| 1136 | H | MeO | A3 | J16 | S |
| 1137 | H | MeO | A3 | J17 | S |
| 1138 | H | MeO | A3 | J18 | S |
| 1139 | H | MeO | A3 | J19 | S |
| 1140 | H | MeO | A3 | J20 | S |
| 1141 | H | MeO | A3 | J21 | S |
| 1142 | H | MeO | A3 | J22 | S |
| 1143 | H | MeO | A3 | J23 | S |
| 1144 | H | MeO | A3 | J24 | S |
| 1145 | H | MeO | A3 | J25 | S |
| 1146 | H | MeO | A3 | J26 | S |
| 1147 | H | MeO | A3 | J27 | S |
| 1148 | H | MeO | A3 | J28 | S |
| 1149 | H | MeO | A3 | J29 | S |
| 1150 | H | MeO | A3 | J30 | S |
| 1151 | H | MeO | A3 | J31 | S |
| 1152 | H | MeO | A3 | J32 | S |

The benzimidazole derivatives represented by formula (I) may be converted to medically acceptable non-toxic salts, if necessary. The salts include salts with alkaline metal ions such as Na⁺ and K⁺; salts with alkaline earth metal ions such as Mg²⁺ and Ca²⁺; salts with metal ions such as Al³⁺ and Zn²⁺; ammonia; salts with organic bases such as triethylamine, ethylenediamine, propanediamine, pyrrolidine, piperidine, piperazine, pyridine, lysine, choline, ethanolamine, N,N-dimethylethanolamine, 4-hydroxypiperidine, glucosamine, N-methylglucamine and the like. Above all, salts with Na⁺, K⁺, Ca²⁺, lysine, choline, N,N-dimethylethanolamine or N-methylglucamine are preferred. Furthermore, salts with acids can be prepared. Such an acid includes for example, mineral acids such as hydrochloric acid, hydrobromic acid, sulfuric acid, phosphoric acid and carbonic acid; and organic acids such as maleic acid, citric acid, malic acid, tartaric acid, fumaric acid, methanesulfonic acid, trifluoroacetic acid, formic acid and the like. Further, compounds of formula (II) include racemic form, both enantiomers and all the stereoisomers (diastereomers, epimers, enantiomers and the like).

Chymase inhibitors in the present invention also include compounds represented by the following formula (II) described in WO 00/05204: 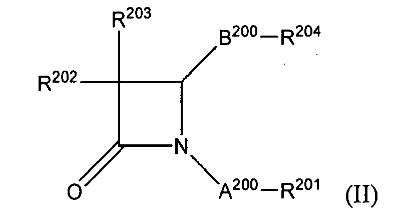 [wherein A²⁰⁰ represents a single bond, -CO-, -COO-, -COCO-, -CONH- or -SO₂-, R²⁰¹ represents lower alkyl optionally having substituents, lower alkenyl optionally having substituents, lower alkynyl optionally having substituents, cycloalkyl optionally having substituents, cycloalkenyl optionally having substituents or aryl optionally having substituents, and R²⁰¹ may be hydrogen when A²⁰⁰ is a single bond, -CO-, -COCO-, -CONH- or -SO₂-, R²⁰² and R²⁰³ represent independently hydrogen, halogen, lower alkyl optionally having substituents, lower alkoxycarbonyl optionally having substituents, acyl optionally having substituents, amino optionally having substituents, carbamoyl optionally having substituents or aryl optionally having substituents, B²⁰⁰ represents a single bond, -S-, -O-, -S-S-, -SO- or -SO₂-, and R²⁰⁴ represents hydrogen, lower alkyl optionally having substituents, aryl optionally having substituents or heterocyclyl optionally having substituents , wherein R²⁰⁴ may be acyl optionally having substituents when B²⁰⁰ is a single bond, -S-, -O-, -SO- or -SO₂-].

Another example of the chymase inhibitor is the compound or a prodrug, pharmaceutically acceptable salt or hydrate thereof represented by formula (II'): 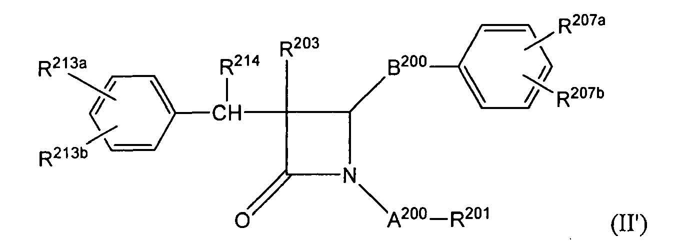 (wherein A²⁰⁰ and R²⁰¹ are as defined for formula (II), R²⁰³ represents hydrogen, halogen, lower alkoxycarbonyl optionally having substituents, acyl optionally having substituents, amino optionally having substituents, aryl optionally having substituents or benzyl optionally having substituents, R^{213a} and R^{213b} each independently represent hydrogen, halogen, hydroxyl, lower alkyl optionally having substituents, lower alkoxy optionally having substituents, amino optionally having substituents or lower alkylthio optionally having substituents, or R^{213a} and R^{213b} taken together form lower alkylenedioxy, R²¹⁴ represents hydrogen, hydroxyl, lower alkyl, lower alkoxy or acyloxy, R^{207a} represents hydrogen, 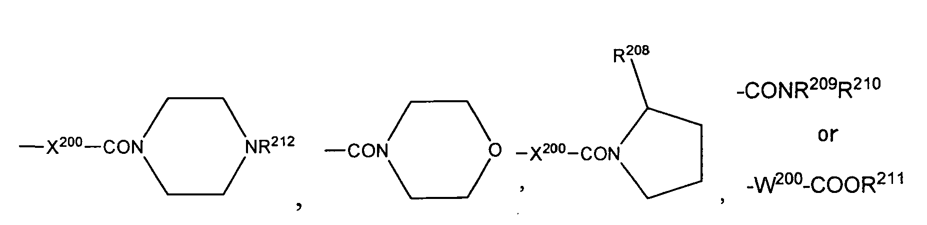 (wherein X²⁰⁰ and W²⁰⁰ represent a single bond, methylene or vinylene, R²⁰⁸ represents methyl or carbamoyl, R²⁰⁹ represents hydrogen or lower alkyl, R²¹⁰ represents lower alkyl optionally having substituents (lower alkylamino; phenyl optionally substituted with halogen; carboxyl; or lower alkoxycarbonyl optionally substituted with aryl), lower alkenyl, lower alkylamino, phenylamino, phenyl or benzenesulfonyl, R²¹¹ represents hydrogen or lower alkyl optionally having substituents (lower alkylamino; acyloxy; phenyl optionally substituted with halogen or methylenedioxy; or heterocyclyl), and
R²¹² represents C₁-C₃ alkyl or cyclohexyl);
R^{207b} is hydrogen, and B²⁰⁰ is O or S).

Still another example of the chymase inhibitor is 4-[1-[N-[bis(4-methylphenyl)methyl]-carbamoyl]-3-(2-ethoxybenzyl)-4-oxoazetidin-2-yloxy]benzoic acid, 4-[1-{[bis(4-methoxyphenyl)methyl]carbamoyl}-3-(2-ethoxybenzyl)-4-oxoazetidin-2-yloxy] benzoic acid or (6R,7R)-3-[1-(carboxymethyl)tetrazol-5-ylsulfanylmethyl]-7-methoxy-7-(2-methoxybenzamido)-1-oxa-3-cephem-4-carboxylic acid 3-methylbenzyl ester or prodrug, pharmaceutically acceptable salt or hydrate thereof.

In formula (II) each of the terms is defined as follows:

A "halogen" includes F, Cl, Br or I, preferably Cl or Br.

A "lower alkyl" means straight or branched alkyl having 1 to 10, preferably 1 to 6, more preferably 1 to 3 carbon atoms, specifically methyl, ethyl, n-propyl, isopropyl, n-butyl, isobutyl, sec-butyl, tert-butyl, n-pentyl, isopentyl, neopentyl, hexyl, isohexyl, heptyl, isoheptyl, octyl, isooctyl, nonyl, decyl or the like.

"A lower alkyl optionally having substituents" includes for example, lower alkyl optionally substituted at any position with one or more substituents selected from hydroxyl, halogen, lower alkoxy, carboxy, acyl, acyloxy, cycloalkyl, lower alkoxycarbonyl optionally having substituents (amino optionally substituted with lower alkyl, aryl or the like), amino optionally having substituents (lower alkyl, acyl or the like), carbamoyl, aryl optionally having substituents [halogen, lower alkyl optionally having substituents {carboxy, lower alkoxycarbonyl optionally having substituents (aryl, alkylamino or the like), lower alkenyloxycarbonyl optionally having substituents (aryl, alkylamino or the like), aryloxycarbonyl optionally having substituents (aryl, alkylamino or the like), or heterocyclylcarbonyl optionally having substituents (lower alkyl, carbamoyl or the like)}, lower alkenyl optionally having substituents {carboxy, lower alkoxycarbonyl optionally having substituents (aryl, alkylamino or the like), lower alkenyloxycarbonyl, aryloxycarbonyl, or heterocyclylcarbonyl optionally having substituents (lower alkyl, carbamoyl or the like) or the like}, lower alkoxy, carboxy, lower alkoxycarbonyl, aryl, acyl, amino optionally having substituents (lower alkyl or the like), carbamoyl optionally having substituents {lower alkyl optionally having substituents (lower alkylamino, aryl or the like); lower alkenyl optionally having substituents (lower alkylamino, aryl or the like), or aryl optionally having substituents (lower alkylamino, aryl or the like)}, aryloxy, heterocyclyl, heterocyclylcarbonyl optionally having substituents (lower alkyl, carbamoyl or the like), or lower alkylenedioxy], heterocyclyl, and heterocyclylcarbonyl optionally having substituents (lower alkyl or the like). Preferred examples of lower alkyl substituted with optionally substituted aryl are unsubstituted benzyl, lower alkyoxybenzyl and diphenylmethyl.

The alkyl moiety of "lower alkoxy", "lower alkoxycarbonyl", "lower alkylamino" or "lower alkylthio" is as defined by "lower alkyl", and the substituent thereon, if present, is the same as that on the alkyl described above.

A "lower alkenylene" includes straight or branched C₁-C₆ alkylene, for example, methylene, ethylene, trimethylene, tetramethylene, propylene, ethylethylene and the like, preferably methylene.

A "lower alkylenedioxy" includes methylenedioxy, ethylenedioxy and the like, preferably methylenedioxy.

A "lower alkenyl" includes straight or branched alkenyl having 2 to 10, preferably 2 to 6, more preferably 2 to 4 carbon atoms. Specifically it includes vinyl, 1-propenyl, allyl, isopropenyl, butenyl, isobutenyl, butadienyl, pentenyl, isopentenyl, pentadienyl, hexenyl, isohexenyl, hexadienyl, heptenyl, octenyl, nonenyl, decenyl and the like, and it has one or more double bonds at arbitrary positions. The substituent in the "lower alkenyl optionally having substituents" includes hydroxyl, halogen, lower alkoxy, carboxy, acyl, acyloxy, cycloalkyl, lower alkoxycarbonyl, aryl, heterocyclyl, heterocyclylcarbonyl optionally having substituents (lower alkyl, carbamoyl or the like) and the like. These substituents may be present at one or more arbitrary positions in the lower alkenyl.

The lower alkenyl moiety in "lower alkenyloxycarbonyl" and the substituents in "optionally substituted lower alkenyloxycarbonyl" are the same as those defined above.

A "lower alkenylene" includes, for example, groups having one or more double bonds at arbitrary positions in the "lower alkylene" described above having 2 to 6, preferably 2 to 4 carbon atoms. It includes specifically vinylene, propenylene, butenylene, pentenylene, methylpropenylene and the like.

A "lower alkynyl" refers to straight or branched alkynyl or the like having 2 to 10, preferably 2 to 6, more preferably 2 to 4 carbon atoms and specifically includes ethynyl, propynyl, butynyl, pentynyl, hexynyl, heptynyl, octynyl, nonynyl, decynyl and the like. These groups have one or more triple bonds at arbitrary positions and may also have double bonds. The substituent in the "lower alkynyl optionally having substituents" is the same as defined for the lower alkenyl.

An "acyl" includes aliphatic acyl having 1 to 10, preferably 1 to 6, more preferably 1 to 3 carbon atoms, aroyl and the like. Specifically it includes formyl, acetyl, propionyl, butyryl, isobutyryl, valeryl, pivaloyl, hexanoyl, acryloyl, propioloyl, methacryloyl, crotonoyl, cyclohexanecarbonyl, benzoyl and the like. The substituent in "acyl optionally having substituents" includes hydroxyl, halogen, lower alkoxy, carboxy, lower alkoxycarbonyl, aryl, heterocyclyl and the like. These substituents may be present at one or more arbitrary positions.

The acyl moiety in "acyloxy" or "acylamino" and the substituents in "acyloxy optionally having substituents" or "acylamino optionally having substituents" are the same as defined for the acyl described above. A preferred example of the acyloxy is acetyloxy.

A "cycloalkyl" refers to for example, three- to six-membered carbocyclyl or the like. Specifically it includes cyclopropyl, cyclobutyl, cyclopentyl, cyclohexyl and the like. The substituent in "cycloalkyl optionally having substituents" includes hydroxyl, halogen, lower alkoxycarbonyl, lower alkoxy, aryl, heterocyclyl and the like. These substituents may be present at one or more arbitrary positions.

A "cycloalkenyl" refers to a group having one or more double bonds at any positions in the ring of the cycloalkyl described above. Specifically it includes cyclopropenyl, cyclobutenyl, cyclopentenyl, cyclohexenyl, cyclohexadienyl and the like. The substituent in "cycloalkenyl optionally having substituents" is the same as that defined for the cycloalkyl described above. These substituents may be present at one or more arbitrary positions. An "amino optionally having substituents" includes substituted amino and unsubstituted amino. It may be substituted with one or more hydroxyl, halogen, lower alkyl, lower alkylamino, acyl, carbamoyl, aryl, heterocyclyl or the like.

A "carbamoyl optionally having substituents" includes substituted carbamoyl and unsubstituted carbamoyl. The substituent therein is selected from lower alkyl optionally having substituents (for example, unsubstituted lower alkyl), lower alkenyl optionally having substituents (for example, unsubstituted lower alkenyl), lower alkylsulfonyl, sulfamoyl, acyl optionally having substituents (such as halogen), amino, aryl optionally having substituents (for example, unsubstituted aryl) and the like.

An "aryl" includes phenyl, naphthyl, anthracenyl, indenyl, phenanthrenyl and the like. Particularly phenyl is preferred.

The substituent in "aryl optionally having substituents" includes: hydroxyl, halogen, lower alkyl optionally having substituents [halogen, carboxy, lower alkoxycarbonyl optionally having substituents (lower alkylamino, aryl or the like), lower alkenyloxy carbonyl optionally having substituents (lower alkylaminoaryl or the like), aryloxycarbonyl optionally having substituents (lower alkylamino, aryl or the like), or heterocyclylcarbonyl optionally having substituents (lower alkyl, carbamoyl or the like)], lower alkenyl optionally having substituents [halogen, carboxy, lower alkoxycarbonyl optionally having substituents (lower alkylamino, aryl or the like), lower alkenyloxycarbonyl optionally having substituents (lower alkylamino, aryl or the like), aryloxycarbonyl optionally having substituents (lower alkylamino, aryl or the like), or heterocyclylcarbonyl optionally having substituents (lower alkyl, carbamoyl or the like)], lower alkoxy optionally having substituents (hydroxyl, halogen, lower alkoxy, carboxy, lower alkoxycarbonyl, amino, lower alkylamino or the like), carboxy, lower alkoxycarbonyl optionally having substituents (acyloxy; lower alkylamino; aryl optionally substituted with alkylenedioxy or halogen; heterocyclyl or the like), lower alkenyloxycarbonyl, lower alkylenedioxy, acyl, acyloxy, amino optionally having substituents (lower alkyl, acyl or the like), nitro, carbamoyl optionally having substituents [lower alkyl optionally having substituents (carboxy; amino optionally substituted with lower alkyl or aroyl; lower alkoxycarbonyl optionally substituted with aryl; halogen; aryl optionally substituted with lower alkyl or lower alkoxy; or the like), cycloalkyl optionally having substituents (aryl or the like), lower alkenyl optionally having substituents (lower alkylamino, aryl or the like), amino optionally having substituents (lower alkyl, aryl or the like), aryl optionally having substituents (lower alkylamino, aryl or the like), arylsulfonyl or the like], aryl, aryloxy, heterocyclyl and heterocyclylcarbonyl optionally having substituents (lower alkyl, arylalkyl optionally substituted with lower alkylenedioxy; cycloalkyl, carbamoyl, heterocyclyl or the like). These substituents may be present at one or more arbitrary positions. The aryl moiety in "aryloxy", "arylsulfonyl" and "arylamino" is the same as "aryl" defined above, and the substituent in " aryloxy optionally having substituents " and "arylsulfonyl optionally having substituents " are the same as that on the aryl described above.

A "benzyl optionally having substituents" may have, at the methylene moiety, substituent defined as the substituent in the "lower alkyl optionally having substituents" or lower alkyl. It may have, at the phenyl moiety, substituent defined as the substituent in the "aryl optionally having substituents". The substituent on the methylene moiety includes specifically lower alkyl, aryl and the like.

A "heterocyclyl" refers to a heterocycle the ring of which contains one or more heteroatoms selected from O, S and N. It includes specifically five- or six-membered aromatic heterocyclyls such as pyrrolyl, imidazolyl, pyrazolyl, pyridyl, pyridazinyl, pyrimidinyl, pyrazinyl,-triazinyl, isoxazolyl, oxazolyl, oxadiazolyl, isothiazolyl, thiazolyl, thiadiazolyl, furyl, thienyl and the like, fused aromatic heterocyclyls such as indolyl, benzimidazolyl, indazolyl, indolizinyl, quinolyl, isoquinolyl, cinnolinyl, phthalazinyl, quinazolinyl, naphthyridinyl, quinoxalinyl, pteridinyl, benzisoxazolyl, benzoxazolyl, xadiazolyl, benzisothiazolyl, benzothiazolyl, benzothiadiazolyl, benzofuryl and benzothienyl and the like, and alicyclic heterocyclyls such as ethyleneoxizinyl, dioxanyl, thiiranyl, oxathiolanyl, azetidinyl, thianyl, pyrrolidinyl, imidazolidinyl, pyrazolidinyl, piperidinyl, piperazinyl and morpholinyl.

The substituent in "heterocyclyl optionally having substituents" includes hydroxyl, halogen, lower alkyl optionally having substituents (for example, unsubstituted lower alkyl), lower alkenyl, lower alkoxy, carboxy, lower alkoxycarbonyl, carbamoyl optionally having substituents (for example, unsubstituted carbamoyl), aryl, heterocyclyl and the like. These substituents may be present at one or more arbitrary positions. The heterocyclyl moiety and substituent in "heterocyclylcarbonyl" and "heterocyclylcarbonyl optionally having substituents" are the same as in the "heterocyclyl" and the substituent in the "heterocyclyl optionally having substituents", respectively. Preferred examples of the "heterocyclylcarbonyl" are morpholylcarbonyl, piperazinylcarbonyl, methylpiperazinylcarbonyl, pyrimidinylpiperazinylcarbonyl, cyclohexylpiperazinylcarbonyl, piperidylcarbonyl, bipiperidylcarbonyl and the like.

Pharmaceutically acceptable salts of compound (II) are for example, salt with mineral acid such as hydochloric acid, sulfuric acid, nitric acid, phosphoric acid, hydrofluoric acid, hydrobromic acid and the like; salt with organic acid such as formic acid, acetic acid, tartaric acid, lactic acid, citric acid, fumaric acid, succinic acid and the like; ammonium salt; salt with organic base such as trimethylammonium, triethylammonium and the like; salt with alkaline metal such as sodium and potassium; salt with alkaline earth metal such as magnesium and calcium. The compound of formula (II) includes hydrate thereof wherein any number of water molecules may be conjugated with one molecule of (II), (II') or (II"). Furthermore, compound of formula (II) includes racemic form, both enantiomers and all stereoisomers (diastereomers, epimers, enantiomers and the like).

Among the chymase inhibitors represented by formula (II) it has already been reported that 4-[1-{[bis(4-methylphenyl)methyl]carbamoyl}-3-(2-ethoxybenzyl)-4-oxoazetizin-2-yloyl]benzoic acid has an effect on a hamster model of myocardial infarction when administrated alone (Life Sci. 2002, vol. 71, p. 437). Therefore it can be expected that this compound can be remarkably effective on various diseases associated with glucose intolerance.

Another example of the chymase inhibitor in the present invention is the compound disclosed in WO 98/09949 represented by formula (III): 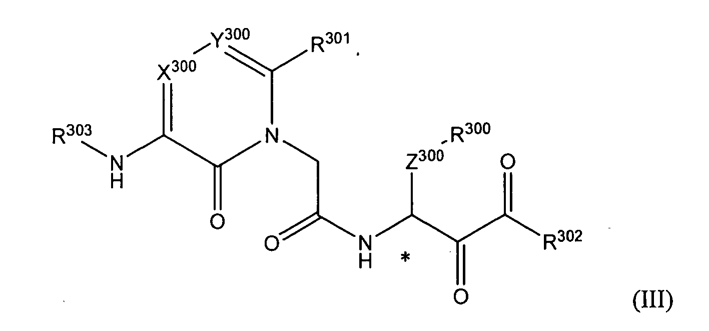 [wherein:
R³⁰⁰ is phenyl, which may have one or more substituents selected from group A³⁰⁰ defined below, (wherein A³⁰⁰ is halogen, nitro, hydroxyl, lower alkoxy, lower alkyl or halogenated lower alkyl);
R³⁰¹ is (III-i) aryl, (III-ii) heteroaryl or (III-iii) straight, branched or cyclic C₁-C₆ alkyl and each independently may have one or more substituents defined in group A³⁰⁰; or R³⁰¹ may have, on group (III-i) - (III-iii), one or more substituents selected from group B³⁰⁰, wherein group B³⁰⁰ is OR^{300a}, COOR^{300a}, CONR^{300b}R^{300c}, NR^{300b}R^{300c}, NR^{300b}CHO, NR^{300b}COR^{300a}, SO₂OR^{300a}, SO₂R^{300a}, CONR^{300b}SO₂R^{300a} or P(O)(OR^{300a})₂ (wherein, R^{300a}-R^{300c} are independently hydrogen, lower alkyl or substituted lower alkyl; or R^{300a}-R^{300c} are independently aryl(C₁-C₇)alkyl, heteroaryl(C₁-C₇)alkyl, aryl or heteroaryl wherein the ring of aryl or heteroaryl may have one or more, usually one to three substituents selected from group A defined above and the lower alkyl has one to three substituents selected from halogen, nitro and hydroxyl); or R³⁰¹ may have on group (III-i) - (III-iii) one or more substituents selected from cyclic group G³⁰⁰ defined below, (wherein G represents five- or six-membered heterocyclyl having one to three oxygen or nitrogen and optionally has substituents);
R³⁰² is C₁-C₈ alkyl, aryl(C₁-C₇)alkyl, heteroaryl(C₁-C₇)alkyl or aryl; or R³⁰² is group B³⁰⁰ defined above, C₁-C₈ alkyl substituted with group B³⁰⁰ or C₁-C₈ alkyl substituted with cyclic group G³⁰⁰ defined above;
R³⁰³ is hydrogen; or R³⁰³ is acyl represented by (i) D³⁰⁰(CH₂)₀₋₃CO, (ii) D³⁰⁰COE³⁰⁰CO or (iii) D³⁰⁰SO₂E³⁰⁰CO; or R³⁰³ is sulfonyl represented by D³⁰⁰(CH₂)₀₋₃SO₂ or D³⁰⁰COE³⁰⁰SO₂ (wherein group D³⁰⁰ is hydrogen, straight, branched or cyclic C₁-C₆ alkyl, aryl, halogenated lower alkyl, halogenated lower alkoxy, amino, lower alkoxyamino, halogenated lower alkylamino, R^{300b}R^{300c}N, R^{300b}R^{300c}NO, R^{300a}O, R^{300a}, R^{300a}OCO, R^{300b}R^{300c}NCO, R^{300a}SO₂NR^{300b}, R^{300a}S, or cyclic group G³⁰⁰ defined above and group E³⁰⁰ represents divalent bridging group having 1 to 6 carbon atoms); or R³⁰³ is urea represented by R^{300b}R^{300c}NCO; or R³⁰³ is thiourea represented by R^{300b}R^{300c}NCS; or R³⁰³ is R^{300a};
X³⁰⁰ and Y³⁰⁰ are each independently nitrogen or carbon and may be substituted with a group represented by R^{300a}-R^{300c}; and
Z³⁰⁰ is polymethylene wherein each hydrogen may be independently substituted with R^{300a} or R^{300b}].

Other examples of the chymase inhibitor includes:
acetamide derivative represented by formula (III) and pharmacologically acceptable salt thereof wherein R³⁰⁰ is unsubstituted phenyl, R³⁰¹ is unsubstituted phenyl, R³⁰² is unsubstituted C₁-C₈ alkyl or C₁-C₈ alkyl having substituents selected from pyrrolidin-1-yl, pyridyloxy, 2-oxo-1,2-dihydropyridin-1-yl, pyrimidyloxy, pyrazyloxy, pyridazyloxy, lower alkyl-substituted piperazin-1-yl and lower alkyl-substituted piperazin-1-ylcarbonyl, X³⁰⁰ is unsubstituted carbon, Y³⁰⁰ is nitrogen and Z³⁰⁰ is -CH₂-, 2-(5-substituted 6-oxo-2-phenyl-1,6-dihydropyrimidin-1-yl)-N-{2,3-dioxo-6-(2-pyridyloxy)-1-phenylmethyl}hexylacetamide, wherein the substituent is amino, t-butyloxycarbonylamino, benzylsulfonylamino, formylamino, benzylaminosulfonylamino, 4-pyridylmethyloxycarbonylamino or acetylamino, and N-[1-benzyl-2,3-dioxo-6-(2-pyridyloxy)hexyl]-2-[5-(formylamino)-6-oxo-2-phenyl-1,6,-dihydropyrimidin-1-yl]acetamide.

In the compound of formula (III), group A³⁰⁰ is selected from halogen, hydroxyl, lower alkoxy, lower alkyl or halogenated lower alkyl;
Group B³⁰⁰, is selected from OR^{300a}, COOR^{300a} CONR^{300b}R^{300c}, NR^{300b}R^{300c}, NR^{300b}CHO, NR^{300b}COR^{300a}, SO₂OR^{300a}, SO₂R^{300a}, CONR^{300b}, SO₂R^{300a} or P(O)(OR^{300a})₂;
R^{300a}-R^{300c} are independently hydrogen, lower alkyl, aryl(C₁₋C₇)alkyl, heteroaryl(C₁-C₇)alkyl, aryl or heteroaryl, wherein the aryl or heteroaryl ring may have one or more substituents selected from group A defined above;
Cyclic group G³⁰⁰ is five- or six-membered heterocyclyl having 1 to 3 oxygen or nitrogen atoms and may have substituents;
Group D³⁰⁰ is hydrogen, straight, branched or cyclic C₁-C₆ alkyl, halogenated lower alkyl such as trifluoromethyl, halogenated lower alkoxy such as 2,2,2-trifluoroethoxy, lower alkoxyamino such as methoxyamino, halogenated lower alkylamino such as 2,2,2-trifluoroethylamino, R^{300b}R^{300c}N, R^{300b}R^{300c}NO, R^{300a}O, R^{300a}, R^{300a}OCO, R^{300b}R^{300c}NCO, R^{300a}SO₂NR^{300b}, R^{300a}S or group G³⁰⁰ defined above;

Group E³⁰⁰ is divalent bridging group having 1 to 6 carbon atoms and may contain 1 to 3 heteroatoms selected from oxygen, nitrogen and sulfur. For example, group E³⁰⁰ includes phenylene, which is a divalent benzene nucleus, heteroarylene, which is a divalent heteroaryl nucleus, 1,4-piperazindiyl, straight or branched aliphatic divalent bridging group having 1 to 6 carbon atoms such as methylene, dimethylene, trimethylene and 2-methyltrimethylene, alicyclic bridging group such as cyclohexylene, 1,4-cyclohexadienylene and the like.

In formula (III) each of the terms is defined as follows:

The halogen represents F, Cl, Br or I.

The alkyl chain in the alkyl, alkoxy or the like represents straight, branched or cyclic alkyl, and the number of carbon atoms is preferably between 1 and 20;

The lower alkyl and the lower alkoxy are either straight or branched group having 1 to 6 carbon atoms. The lower acyloxy represents acyloxy with an alkyl chain having 1 to 6 carbon atoms. The aryl represents phenyl or ortho-fused carbocyclyl or hetero-carbocyclyl that has 9 to 10 atoms in the rings and at least one aromatic ring. The heteroaryl contains 2 to 4 heteroatoms selected from carbon, oxygen, nitrogen and sulfur, and it is either five- or six-membered monocyclic aromatic group or ortho-fused hetero-heterocyclic group having about 8 to 10 atoms composing the rings.

The compound of formula (III), due to the presence of a chiral carbon marked by "*" in formula (III), exists as either a single enantiomer or racemic form. When a compound of formula (III) contains another chiral atom, it exists as either a single diastereomer or a mixture of diastereomers. Either of them can be isolated. In the present invention compound of formula (III) includes each of the diastereomers and diastereomeric mixture and furthermore it includes each of the enantiomers and enantiomeric mixture.

As those engaged in this field understand, the adjacent-dicarbonyl moiety in formula (III) sometimes exists as solvate, particularly hydrate. Therefore, the compound represented by formula (III) includes solvates thereof.

Besides the solvates described above, some of the compounds represented by formula (III) exist as various polymorphic forms such as a tautomer of solvate. Therefore, the present invention comprises all the compounds that have inhibitory activity against chymotrypsin-like enzymes, including any polymorphic form, racemic and optically active forms and solvates.

The following demonstrates examples of the groups in formula (III), which are not limitation but merely examples.

Preferable groups for A³⁰⁰ are F, Cl, Br, nitro, hydroxyl, methyl, ethyl and methoxy.

R^{300a}, R^{300b} and R^{300c} are for example, hydrogen, lower alkyl such as methyl, ethyl, propyl, butyl, isopropyl and the like, aryl(C₁-C₇)alkyl such as benzyl, phenethyl, phenylpropyl and the like, heteroaryl(C₁-C₇)alkyl such as pyridylmethyl, pyridylethyl, pyridylpropyl, furylmethyl, furylethyl, furylpropyl and the like, aryl such as phenyl, halogenated phenyl and the like or heteroaryl such as pyridyl, pyrimidyl, furyl, thienyl and the like.

OR^{300a} in group B³⁰⁰, group D³⁰⁰ or the like is, for example, hydroxyl, methoxy, ethoxy, propyloxy, isopropyloxy, butoxy, benzyloxy, pyridylmethyloxy, phenoxy, pyridyloxy, pyrrolidinoxy and the like.

COOR^{300a} in group B³⁰⁰, group D³⁰⁰ or the like is, for example, methoxycarbonyl, ethoxycarbonyl, propyloxycarbonyl, isopropyloxycarbonyl, butoxycarbonyl, benzyloxycarbonyl, pyridylmethyloxycarbonyl, phenoxycarbonyl and the like.

CONR^{300b}R^{300c} in group B³⁰⁰, group D³⁰⁰ or the like is, for example, dimethylaminocarbonyl, methylethylaminocarbonyl, diethylaminocarbonyl, dipropylaminocarbonyl and the like.

NR^{300b}R^{300c} in group B³⁰⁰, group D³⁰⁰ or the like is, for example, monomethylamino, dimethylanimo, methylethylamino, diethylamino, dipropylamino and the like. NR^{300b}CHO in group B³⁰⁰ or the like is, for example, formylamino, formylmethylamino and the like. NR^{300b}COR^{300a} in group B³⁰⁰ or the like is, for example, methylcarbonylamino, ethylcarbonylamino, propylcarbonylamino, methylcarbonylmethylamino and the like. SO₂OR^{300a} in group B or the like is, for example, sulfonic acid group and the like. SO₂R^{300a} in group B³⁰⁰ or the like is, for example, methylsulfonyl, ethylsulfonyl, propylsulfonyl, butylsulfonyl, t-butylsulfonyl, benzylsulfonyl, toluenesulfonyl, benzenesulfonyl, formaminobenzenesulfonyl, nitrobenzenesulfonyl, methoxybenzenesulfonyl, pyridylsulfonyl, pyridylmethylsulfonyl, trifluoromethylsulfonyl and the like.

CONR^{300b}SO₂R^{300a} in group B³⁰⁰ or the like is, for example, methylsulfonylaminocarbonyl, phenylsulfonylaminocarbonyl, phenylmethylaminosulfonylcarbonyl and the like. P(O)(OR^{300a})₂ in group B³⁰⁰ or the like is, for example, diethylphosphono, diphenylphosphono, dibenzylphosphono and the like. Preferable groups for B³⁰⁰ are methoxy, ethoxy, propyloxy, isopropyloxy, phenylmethyloxy, phenethyloxy, phenylpropyloxy, pyridylmethyloxy, pyridylethyloxy, pyridylpropyloxy, furylmethyloxy, furylethyloxy, furylpropyloxy, pyridyloxyethyloxy, pyridyloxypropyloxy and the like.

Group G³⁰⁰ is, for example, five- or six-membered heteroaryl or five- or six-membered alicyclic group having heteroatoms, preferably 4-morpholin-4-yl, 4-methylpiperazin-1-yl, pyrrolidin-1-yl, piperidin-1-yl, 2-oxo-1,2-dihydropyridin-1-yl or 2-pyridyloxy. Preferable groups for D³⁰⁰ are hydrogen, methyl, cyclohexyl, phenyl, pyridyl, trifluoromethyl, 2,2,2-trifluoroethyloxy, methyloxyamino, 2,2,2-trifluoroethylamino, phenylmethylamino and the like.

D³⁰⁰(CH₂)₀₋₃CO in R³⁰³ is for example, formyl, acetyl, propionyl, cyclopropanecarbonyl, valeryl, butyryl, cyclopropylmethylcarbonyl, pivaloyl, trifluoroacetyl, phenylacetyl, 3-phenylpropionyl, pyridylcarbonyl, benzoyl, tetrahydro-2-furoyl, tetrahydro-3-furoyl, methoxycarbonyl, ethoxycarbonyl, propyloxycarbonyl, isopropyloxycarbonyl, butyloxycarbonyl, t-butyloxycarbonyl, benzyloxycarbonyl, 9-fluorenyloxycarbonyl, 2,2,2-trichloroethoxycarbonyl, allyloxycarbonyl, hydroxyoxalyl and the like.

The acyl group represented by D³⁰⁰COE³⁰⁰CO or D³⁰⁰SO₂E³⁰⁰CO in R³⁰³ is, for example, 4-[1-(4-morpholin-1-yl)carbonyl]benzenecarbonyl, [4-(1-pyrrolidin-1-yl)carbonyl]benzenecarbonyl, [4-(1-piperidin-1-yl)carbonyl]benzenecarbonyl, phenylsulfonylaminocarbonyl and the like.

D³⁰³(CH₂)₀₋₃SO₂ in R³⁰³ is, for example, toluenesulfonyl, benzenesulfonyl, formaminobenzenesulfonyl, nitrobenzenesulfonyl, methoxybenzenesulfonyl, pyridylsulfonyl, pyridylmethylsulfonyl, methylsulfonyl, ethylsulfonyl, propylsulfonyl, butylsulfonyl, t-butylsulfonyl, benzylsulfonyl, trifluoromethylsulfonyl, phenacylsulfonyl, aminosulfonyl, methylaminosulfonyl, ethylaminosulfonyl, propylaminosulfonyl, isopropylaminosulfonyl, butylaminosulfonyl, t-butylaminosulfonyl, phenylaminosulfonyl, benzylaminosulfonyl, pyridylaminosulfonyl, pyridylmethylaminosulfonyl and the like.

D³⁰⁰COE³⁰⁰SO₂ in R³⁰³ is, for example, benzoylaminosulfonyl and the like.

The thiourea represented by R^{300b}R^{300c}NCS in R³⁰³, is for example, methylaminothiocarbonyl, ethylaminothiocarbonyl, propylaminothiocarbonyl, butylaminothiocarbonyl, isopropylaminothiocarbonyl, valerylaminothiocarbonyl, benzylaminothiocarbonyl and the like.

A preferred group for R³⁰⁰ is phenyl, which may have 1 to 4 substituents selected from halogen, nitro, hydroxyl, lower alkoxy, lower alkyl and trifluoromethyl, as group A³⁰⁰ on the ring thereof.

Preferred groups for R³⁰¹ are phenyl, furyl, thienyl and pyridyl, which may have one or two substituents selected from group A³⁰⁰ on the ring thereof.

Preferred groups for R³⁰² are C₁-C₄ alkyl, aryl(C₁-C₃)alkyl and G³⁰⁰(C₁-C₃) alkyl substituted with a group selected from group G³⁰⁰ defined above. More preferred groups are methyl, ethyl, propyl, butyl, isopropyl, benzyl, phenethyl, phenylpropyl, pyridylmethyl, pyridylethyl, pyridylpropyl, furylmethyl, furylethyl, furylpropyl, pyridyloxymethyl, pyridyloxyethyl, pyridyloxypropyl, piperazin-1-yl(C₁-C₃)alkyl optionally substituted at the 4-position with a group selected from methyl, ethyl, propyl, butyl, isopropyl, benzyl and pyridylmethyl, piperidin-1-yl(C₁-C₃)alkyl, 4-morpholin-4-yl(C₁-C₃)alkyl, 2-pyridyloxy(C₁-C₃)alkyl, pyrrolidin-1-yl(C₁-C₃)alkyl, 2-oxo-1,2-dihydropyridin-1-yl(C₁-C₃)alkyl, methoxycarbonyl(C₀-C₃)alkyl, ethoxycarbonyl(C₀-C₃)alkyl, propyloxycarbonyl(C₀-C₃)alkyl, butyloxycarbonyl(C₀-C₃)alkyl, benzyloxycarbonyl(C₀-C₃)alkyl, t-butoxycarbonyl(C₀-C₃)alkyl, phenyloxycarbonyl(C₀-C₃)alkyl, nitrophenyloxycarbonyl(C₀-C₃)alkyl and bromophenyloxycarbonyl(C₀-C₃)alkyl. Furthermore preferred groups are methyl, ethyl, propyl, butyl, phenylpropyl, 4-morpholin-4-yl(C₁-C₃)alkyl, 2-oxo-1 ,2-dihydropyridin-1-yl(C₁-C₃)alkyl, 2-pyridyloxy(C₁-C₃)alkyl, ethoxycarbonyl(C₀-C₃)alkyl and 4-methylpiperazin-1-ylcarbonyl(C₁-C₃)alkyl.

R³⁰³ is preferably hydrogen, formyl, acetyl, propionyl, cyclopropanecarbonyl, valeryl, butyryl, cyclopropylmethylcarbonyl, pivaloyl, trifluoroacetyl, phenylacetyl, 3-phenylpropionyl, pyridylcarbonyl, benzoyl, tetrahydro-2-furoyl, tetrahydro-3-furoyl, methoxycarbonyl, ethoxycarbonyl, propyloxycarbonyl, isopropyloxycarbonyl, butyloxycarbonyl, t-butyloxycarbonyl, benzyloxycarbonyl, 9-fluorenyloxycarbonyl, 2,2,2-trichloroethoxycarbonyl, allyloxycarbonyl, hydroxyoxalyl, 4-[1-(4-morpholin-4-yl)carbonyl]benzenecarbonyl, [4-(1-pyrrolidin-1-yl)carbonyl]benzenecarbonyl, [4-(1-piperidin-1-yl)carbonyl]benzenecarbonyl, toluenesulfonyl, benzenesulfonyl, formaminobenzenesulfonyl, nitrobenzenesulfonyl, methoxybenzenesulfonyl, pyridylsulfonyl, pyridylmethylsulfonyl, methylsulfonyl, ethylsulfonyl, propylsulfonyl, butylsulfonyl, t-butylsulfonyl, benzylsulfonyl, trifluoromethylsulfonyl, phenacylsulfonyl, aminosulfonyl, methylaminosulfonyl, ethylaminosulfonyl, propylaminosulfonyl, isopopylaminosulfonyl, butylaminosulfonyl, t-butylaminosulfonyl, phenylaminosulfonyl, benzylaminosulfonyl, pyridylaminosulfonyl, pyridylmethylaminosulfonyl, methylaminothiocarbonyl, ethylaminothiocarbonyl, propylaminothiocarbonyl, butylaminothiocarbonyl, isopropylaminothiocarbonyl, valerylaminothiocarbonyl, benzylaminothiocarbonyl (wherein, the phenyl or heteroaryl ring, if present, may be substituted with one or two halogen or methyl), or methyl, ethyl, propyl, isopropyl, butyl, t-butyl, benzyl, phenethyl, thiazolyl, pyridylmethyl or 5-tetrazolylmethyl (wherein, the phenyl or heteroaryl ring, if present, may be substituted with one or two halogen or methyl).

X³⁰⁰ and Y³⁰⁰ are preferably carbon or nitrogen.

Z³⁰⁰ is preferably polymethylene having 1 to 3 carbon atoms, more preferably methylene.

Particularly valuable groups for the straight or branched C₁-C₈ alkyl are methyl, ethyl, propyl, isopropyl, butyl, isobutyl, amyl, isoamyl, hexyl, heptyl and octyl. Particularly valuable groups for the cyclic alkyl are cyclopropyl, cyclobutyl, cyclopentyl and cyclohexyl. Valuable groups for the alkylene moiety in the aryl(C₁-C₇)alkyl or heteroaryl(C₁-C₇)alkyl are methylene, ethylene, trimethylene, tetramethylene, pentamethylene, hexamethylene and heptamethylene. A particularly valuable group for the aryl is phenyl. Particularly valuable groups for the heteroaryl are pyridyl, pyrimidinyl, furyl and thienyl. Preferred groups for the aryl(C₁-C₇)alkyl are phenylmethyl, phenylethyl, phenylpropyl, phenylisopropyl, phenylbutyl, phenylisobutyl, phenylamyl, phenylisoamyl, phenylhexyl, phenylheptyl and the like. Preferred groups for the heteroaryl(C₁-C₇)alkyl are, wherein the heteroaryl is pyridyl, pyrimidinyl, furyl or thienyl, ones having the same alkyl moiety as the phenyl described above.

Particularly valuable groups for the lower alkyl are methyl, ethyl, propyl, isopropyl, butyl, isobutyl and t-butyl. Particularly valuable groups for the lower alkoxy are methoxy, ethoxy, propyloxy, isopropyloxy and butoxy. Particularly valuable groups for the halogen are F, Cl and Br.

A particular group of compound (III) consists of compounds wherein R³⁰⁰, R³⁰², R³⁰³, X³⁰⁰, Y³⁰⁰ and Z³⁰⁰ are selected from each of the groups described above and R³⁰¹ is phenyl.

A more specified group of compound (III) consists of compounds wherein each of the symbols is as follows:
R³⁰⁰ is phenyl, which may have one to three substituents selected from halogen, hydroxyl, lower alkoxy, lower alkyl and trifluoromethyl as group A³⁰⁰.
R³⁰¹ is phenyl, which may have one or more substituents independently selected from group A³⁰⁰ defined as described above; or R³⁰¹ may have one or more substituents selected from group B³⁰⁰, which contains OR^{300a}, COOR^{300a}, CONR^{300b}R^{300c}, NR^{300b}R^{300c}, NR^{300b}CHO, NR^{300b}COR^{300a}, SO₂OR^{300a}, SO₃R^{300a}, CONR^{300b}SO₂R^{300a} and P(O)(OR^{300a})₂.
R³⁰² is pyridyloxy(C₁-C₄)alkyl.
R³⁰³ is hydrogen; or R³⁰³ is acyl represented by (i) D³⁰⁰(CH₂)₀₋₃CO, (ii) D³⁰⁰COE³⁰⁰CO or (iii) D³⁰⁰SO₂E³⁰⁰CO; or R³⁰³ is sulfonyl represented by D³⁰⁰(CH₂)₀₋₃SO₂ or D³⁰⁰COE³⁰⁰SO₂ (wherein, group D³⁰⁰ represents hydrogen, straight, branched or cyclic C₁-C₆ alkyl, trifluoromethyl, 2,2,2-trifluoroethoxy, 2,2,2-trifluoroethylamino, COOR^{300a}, CONR^{300b}R^{300c},NR^{300b}R^{300c} or group G³⁰⁰ defined above; or R³⁰³ is thiourea represented by R^{300b}R^{300c}NCS and group E³⁰⁰ is independently phenyl, heteroaryl, 1,4-piperazindiyl, cyclohexyl or 1,4-cyclohexadienyl); or R³⁰³ is R³⁰⁰a.
X³⁰⁰ and Y³⁰⁰ are each independently nitrogen or unsubstituted carbon.
Z³⁰⁰ is -CH₂-, wherein the two hydrogen atoms may be independently substituted with R^{300a} or R^{300b}.

A particular group of more specified compound (I) consists of compounds wherein R³⁰⁰ is phenyl (which may contain one or two substituents independently selected from halogen, hydroxyl and methyl), R³⁰² is methyl, butyl, phenylpropyl, 4-morpholin-4-yl-propyl, 1-(ethoxycarbonyl)propyl, 4-methylpiperazin-1-ylpropyl, 2-oxo-1,2-dihydropyridin-1-yl-propyl, or 2-pyridyloxypropyl, R³⁰³ is hydrogen or formyl, X³⁰⁰ and Y³⁰⁰ are unsubstituted carbon or nitrogen, and Z³⁰⁰ is unsubstituted methylene. In a further specified case, R³⁰⁰ is phenyl, 3-fluorophenyl, 4-fluoropheyl, 3,4-difluorophenyl, 3,5-difluorophenyl or 3-fluoro-4-hydroxyphenyl.

Also, pharmacologically acceptable salts of compound (III) are not particularly limited. For example, when compound (III) is acidic, the pharmacologically acceptable salt thereof includes alkali metal salts, alkaline earth metal salts, aluminum salts, ammonium salts and salts with pharmaceutically acceptable cations derived from organic bases such as primary or tertiary lower alkylamines. (B) when compound (III) is basic, pharmacologically acceptable salts thereof include acid addition salts that generate pharmaceutically acceptable anions, wherein the acid addition salts are formed by using, for example, hydrochloric acid, sulfuric acid, sulfonic acid, phosphoric acid and the like.

In particular, among the compounds represented by formula (III), it has been reported that the compound shown by the following formula (III-I) is effective in a canine model of myocardial infarction through oral administration (The 75th annual meeting report of The Japanese Pharmacological Society), hence it is expected that this compound will be useful as a chymase inhibitor in the present invention.

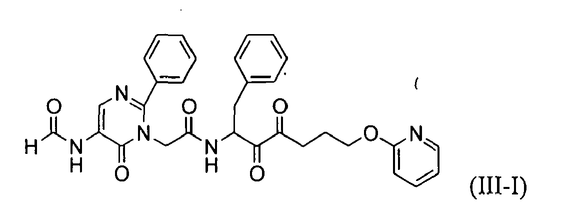

The chymase inhibitor in the present invention also includes the heterocyclic amide compounds represented by formula (IV) and pharmacologically acceptable salts thereof disclosed in WO 98/18794: 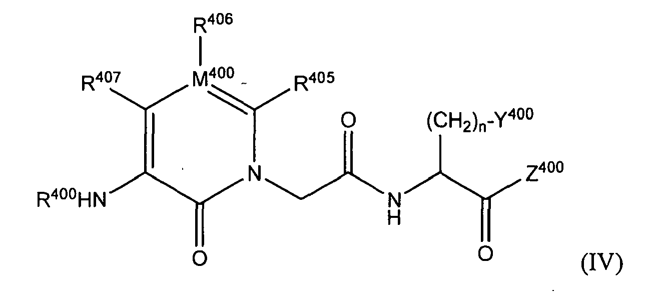 [wherein
R⁴⁰⁰ is hydrogen, alkyl, -CHO, -CONH₂, -COR⁴⁰¹, -COOR⁴⁰¹, -CONHOR⁴⁰¹, -CONHR⁴⁰¹, -CONR⁴⁰¹R^{401'}, -CONHSO₂R⁴⁰¹, -COSR⁴⁰¹, -COCOR⁴⁰², -COCOOR⁴⁰², -CONHCOOR⁴⁰², -COCONR⁴⁰³R⁴⁰⁴, -CSX⁴⁰⁰R⁴⁰¹, -SO₂WR⁴⁰¹, -SO₂NR⁴⁰¹R^{401'} or -SO₂E⁴⁰⁰ (wherein R⁴⁰¹ and R^{401'} may be the same or different and each independently represent alkyl, cycloalkyl, cycloalkylalkyl, aryl, arylalkyl, heteroaryl, heteroarylalkyl, heterocyclyl or heterocyclylalkyl; R⁴⁰², R⁴⁰³ and R⁴⁰⁴ may be the same or different and each independently represent hydrogen, alkyl or arylalkyl, or -NR⁴⁰³ R⁴⁰⁴ taken together may represent heterocyclyl, X⁴⁰⁰ represents a single bond, NH-, -O- or -S-, W⁴⁰⁰ represents a single bond, -NH-, -NHCO-, -NHCOO-or -NHCONH-, and E⁴⁰⁰ represents hydroxyl or amino), R⁴⁰⁵ , R⁴⁰⁶ and R⁴⁰⁷ may be the same or different, and either each independently represent hydrogen or alkyl or one of them is selected from aryl, arylalkyl, arylalkenyl, heteroaryl, heteroarylalkyl and heteroarylalkenyl with the other being hydrogen, M⁴⁰⁰ represents carbon or nitrogen, wherein R⁴⁰⁶ is absent if M⁴⁰⁰ is nitrogen, Y⁴⁰⁰ represents cycloalkyl, aryl or heteroaryl, Z⁴⁰⁰ represents the groups shown by formulas (IV-i), (IV-ii) and (IV-iii): 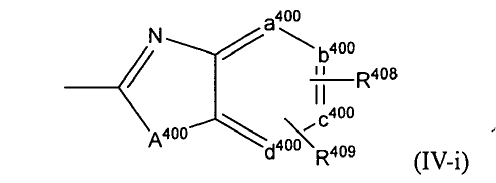 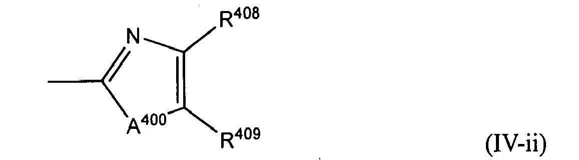 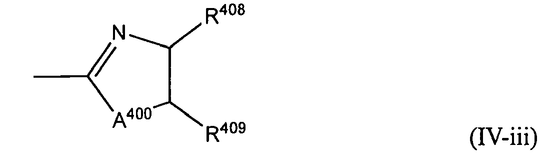 {wherein R⁴⁰⁸ and R⁴⁰⁹ may be the same or different and each independently represent hydrogen, alkyl, aryl, arylalkyl, heteroaryl, heteroarylalkyl, halogen, trifluoromethyl, cyano, nitro, -NR⁴¹⁰R^{410'}, -NHSO₂R⁴¹⁰, -OR⁴¹⁰, -COOR⁴¹⁰, -CONHSO₂R⁴¹⁰ or -CONR⁴¹⁰R^{410'} (wherein R⁴¹⁰ and R^{410'} may be the same or different and each independently represent hydrogen, alkyl, cycloalkyl, cycloalkylalkyl, aryl, arylalkyl, heteroaryl, heteroarylalkyl or trifluoromethyl, or -NR⁴¹⁰R^{410'} taken together may represent heterocyclyl), A⁴⁰⁰ represents -O-, -S- or -NR⁴¹²-, (wherein R⁴¹² represents hydrogen, alkyl, clycloalkyl or cycloalkylalkyl), and a⁴⁰⁰, b⁴⁰⁰, c⁴⁰⁰ and d⁴⁰⁰ are all carbon or one of them is nitrogen with the rest being carbon}, and n is 0 or 1.

In addition, each of the alkyl, cycloalkyl, cycloalkylalkyl, aryl, arylalkyl, arylalkenyl, heteroaryl, heteroarylalkyl, heteroarylalkenyl, heterocyclyl and heterocyclyl alkyl described above may have substituents].

As the chymase inhibitor may also be used the heterocyclic amide compound represented by formula (IV) or pharmacologically acceptable salts thereof wherein Y⁴⁰⁰ is aryl optionally having substituents, Z⁴⁰⁰ is the group represented by formula (IV-i), and one of R⁴⁰⁵ R⁴⁰⁶ and R⁴⁰⁷ is aryl optionally having substituents with the other being hydrogen (R⁴⁰⁶ is absent if M is nitrogen), 2-[2-[2-[5-amino-2-(3-methoxyphenyl)-6-oxo-1,6-dihydropyrimidin-1-yl]acetamido]-3-phenylpropionyl] benzoxazole-5-carboxylic acid methyl ester, and 2-[2-[5-amino-2-(4-fluorophenyl)-6-oxo-1,6-dihydropyrimidin-1-yl]acetamido]-3-phenylpropionyl)benzoxazole-5-carboxylic acid methyl ester.

Each of the terms in the formula (IV) is defined as follows:

The alkyl as R⁴⁰⁰, R⁴⁰¹, R⁴⁰¹, R⁴⁰²-R⁴¹⁰, R^{410'} or R⁴¹² is straight or branched alkyl having preferably 1 to 6 carbon atoms, for example, methyl, ethyl, n-propyl, isopropyl, n-butyl, isobutyl, sec-butyl, tert-butyl, n-pentyl, isopentyl, n-hexyl and the like.

The cycloalkyl as R⁴⁰¹, R^{401'}, R⁴¹⁰, R^{410'}, R⁴¹² or Y⁴⁰⁰ is preferably three- to seven-membered cycloalkyl, for example, cyclopropyl, cyclobutyl, cyclopentyl, cyclohexyl, cycloheptyl and the like.

The cycloalkylalkyl as R⁴⁰¹, R^{401'}, R⁴¹⁰, R^{410'}or R⁴¹² comprises the cycloalkyl described above and a straight or branched alkyl having preferably 1 to 3 carbon atoms. The examples are cyclopropylmethyl, 2-cyclobutylethyl, 3-cyclopentylpropyl, cyclohexylmethyl, 2-cyclohexylethyl, cycloheptylmethyl and the like.

The aryl as R⁴⁰¹, R^{401'}, R⁴⁰⁵-R⁴¹⁰, R^{410'} or Y⁴⁰⁰ is preferably phenyl, naphthyl, ortho-fused bicyclic groups having 8 to 10 atoms composing the rings and at least one aromatic ring (for example, indenyl) and the like.

The arylalkyl as R⁴⁰¹, R^{401'}, R⁴⁰²-R⁴¹⁰ or R^{410'} comprises the aryl described above and a straight or branched alkyl having preferably 1 to 3 carbon atoms. The examples are benzyl, phenethyl, 3 -phenylpropyl, 1-naphthylmethyl, 2-naphthylmethyl, 2-(1-naphthyl)ethyl, 2-(2-naphthyl)ethyl, 3-(1-naphthyl)propyl, 3-(2-naphthyl)propyl and the like. The arylalkenyl as R^{405_}R⁴⁰⁷ comprises the aryl described above and a straight or branched alkenyl having preferably 2 to 6 carbon atoms. The examples are styryl, 3-phenyl-2-propenyl, 4-phenyl-3-butenyl, 5-phenyl-4-pentenyl, 6-phenyl-5-hexenyl, 3-(1-naphthyl)-2-propenyl, 4-(2-naphthyl)-3-butenyl and the like.

The heteroaryl as R⁴⁰¹, R^{401'}, R⁴⁰⁵-R⁴¹⁰, R^{410'} or Y⁴⁰⁰ includes preferably five- or six-membered heteroaryl consisting of carbon atoms and 1 to 4 heteroatoms (oxygen, nitrogen or sulfur), ortho-fused bicyclic heteroaryl having 8 to 10 atoms composing the rings derived therefrom, particularly benzo-derivatives or derivatives fused with propenylene, trimethylene or tetramethylene and stable N-oxide thereof. The examples are pyrrolyl, furyl, thienyl, oxazolyl, isoxazolyl, imidazolyl, thiazolyl, isothiazoyl, pyrazolyl, triazolyl, tetrazolyl, 1,3,5-oxadiazolyl, 1,2,4-oxadiazolyl, 1,2,4-thiadiazolyl, pyridyl, pyranyl, pyrazinyl, pyrimidinyl, pyridazinyl, 1,2,4-triazinyl, 1,2,3-triazinyl, 1,3,5-triazinyl, benzoxazolyl, benzothiazolyl, benzimidazolyl, thianaphthenyl, isothianaphthenyl, benzofuranyl, isobenzofuranyl, chromenyl, isoindolyl, indolyl, indazolyl, isoquinolyl, quinolyl, phthalazinyl, quinoxalinyl, quinazolinyl, cinnolinyl, benzoxazinyl and the like.

The heteroarylalkyl as R⁴⁰¹, R^{401'}, R⁴⁰⁵-R⁴¹⁰ or R^{410'} comprises the heteroaryl described above and a straight or branched alkyl having preferably 1 to 3 carbon atoms. The examples are 2-pyrrolylmethyl, 2-pyridylmethyl, 3-pyridylmethyl, 4-pyridylmethyl, 2-thienylmethyl, 2-(2-pyridyl)ethyl, 2-(3-pyridyl)ethyl, 2-(4-pyridyl)ethyl, 3-(2-pyrrolyl)propyl and the like.

The heteroarylalkenyl as R⁴⁰⁵-R⁴⁰⁷ comprises the heteroaryl described above and a straight or branched alkenyl having preferably 2 to 6 carbon atoms. The examples are 2-(2-pyridyl)ethenyl, 3-(2-pyridyl)-2-propenyl, 4-(3-pyridyl)-3-butenyl, 5-(2-pyrrolyl)-4-pentenyl, 6-(2-thienyl)-5-hexenyl and the like. The heterocyclyl represented by R⁴⁰¹ or R^{401'} is four- to six-membered cyclic group consisting of carbon atoms and 1 to 4 heteroatoms (oxygen, nitrogen or sulfur), for example, azetidinyl, pyrrolidinyl, piperidinyl, piperidino, piperazinyl, morpholinyl, morpholino, thiomorpholinyl, oxothiomorpholinyl, dioxothiomorpholinyl, tetrahydropyranyl, dioxacyclohexyl and the like.

The heterocyclyl represented by -NR⁴⁰³R⁴⁰⁴ or -NR⁴¹⁰R^{410'} is four- to six-membered cyclic group consisting of carbon atoms and at least one nitrogen atom, which may further contain other heteroatoms (oxygen or sulfur). The examples are azetidinyl, pyrrolidinyl, piperidino, piperazinyl, morpholino, thiomorpholino, oxothiomorpholino, dioxothiomorpholino and the like.

The heterocyclylalkyl as R⁴⁰¹ or R^{401'} comprises the heterocyclyl defined above (R⁴⁰¹ or R^{401'}) and a straight or branched alkyl having preferably 1 to 3 carbon atoms. The examples are azetidinylethyl, pyrrolidinylpropyl, piperidinylmethyl, piperidinoethyl, piperazinylethyl, morpholinylpropyl, morpholinomethyl, thiomorpholinylethyl, oxothiomorpholinylethyl, dioxothiomorpholinylethyl, tetrahydropyranylpropyl, dioxacyclohexylmethyl and the like.

The halogen as R⁴⁰⁸ or R⁴⁰⁹ includes F, Cl, Br and I.

In addition, among the substituents described above, alkyl, cycloalkyl, cycloalkylalkyl, aryl, arylalkyl, arylalkenyl, heteroaryl, heteroarylalkyl, heteroarylalkenyl, heterocyclyl and heterocyclylalkyl each may be optionally substituted with one or more substituents described below.

The substituents on the substituents include halogen, hydroxyl, nitro, cyano, trifluoromethyl, alkyl, alkoxy, alkylthio, formyl, acyloxy, oxo, phenyl, arylalkyl, -COOR^{400a}, -CH₂COOR^{400a}, -OCH₂COOR^{400a}, -CONR^{400b}R^{400c}, -CH₂CONR^{400b}R^{400c}, -OCH₂CONR^{400b}R^{400c}, -COO(CH₂)₂NR^{400e}R^{400f}, -SO₂T⁴⁰¹, -CONR^{400d}SO₂T⁴⁰¹, -NR^{400e}R^{400f}, -NR^{400g}CHO, -NR^{400g}COT⁴⁰², -NR^{400g}COOT⁴⁰², -NR^{400h}CQ⁴⁰⁰NR⁴⁰⁰ⁱR^{400j}, -NR^{400k}SO₂T⁴⁰³, -SO₂NR⁴⁰¹R^{400m}, -SO₂NR⁴⁰⁰ⁿCOT⁴⁰⁴ and the like.

In the substituents on the substituents described above, the halogen, alkyl and arylalkyl are the same as defined earlier. The alkoxy comprises a straight or branched chain of preferably 1 to 6 carbon atoms. The examples are methoxy, ethoxy, propoxy, butoxy, pentyloxy, hexyloxy and the like. The alkylthio comprises a straight or branched chain of preferably 1 to 6 carbon atoms. The examples are methylthio, ethylthio, propylthio, butylthio, pentylthio, hexylthio and the like.

The acyloxy comprises a straight or branched chain of preferably 1 to 6 carbon atoms. The examples are formyloxy, acetyloxy, propionyloxy, butyryloxy, valeryloxy, pivaloyloxy, hexanoyloxy and the like.

In addition, R^{400a}-R⁴⁰⁰ⁿ represents hydrogen, alkyl (same as described above) or arylalkyl (same as described above). Further, -NR^{400b}R^{400c}, -NR^{400e}R^{400f}, -NR⁴⁰⁰ⁱR^{400j} or -NR⁴⁰¹R^{400m} taken together may represent heterocyclyl (which is the same as exemplified for -NR⁴⁰³R⁴⁰⁴ or -NR⁴¹⁰R^{410'} and may be substituted with the substituents described above). Further, -NR^{400e}R^{400f} may represent heterocyclyl containing =O (for example, 2-pyrrolidinon-1-yl, succinimide, oxazolidin-2-on-3-yl, 2-benzoxazolinon-3-yl, phthalimide, cis-hexahydrophthalimide and the like). T⁴⁰¹-T⁴⁰⁴ is the same as R⁴⁰¹, which may be optionally substituted with the substituents described above. Q⁴⁰⁰ represents =O or =S.

Compound (IV) exists as either optically active or racemic form due to an asymmetric carbon atom to which -(CH₂)ₙ-Y⁴⁰⁰ is bonded. The racemic form can be separated into each of the enantiomers by known techniques. Further, when compound (IV) contains additional asymmetric carbon atoms, it exists as either a single diastereomer or a diastereomeric mixture, which can be separated by known technique.

Compound (IV) may exhibit polymorphism, exist as more than one tautomeric form or exist as solvate (such as ketone solvate and hydrate).

Accordingly, the present invention includes any of stereoisomers, optical isomers, polymorphic forms, tautomeric forms, solvates and any mixtures thereof.

When compound (IV) is acidic, pharmacologically acceptable salts thereof include alkali metal salt (for example, salt with lithium, sodium, potassium or the like), alkaline earth metal salt (for example, salt with calcium, magnesium or the like), aluminum salt, ammonium salt, salt with an organic base (for example, salt with triethylamine, morpholine, piperidine, triethanolamine or the like) and the like.

When compound (IV) is basic, pharmacologically acceptable salts thereof include salt with an inorganic acid (for example, salt with hydrochloric acid, hydrobromic acid, hydroiodic acid, sulfuric acid, phosphoric acid or the like), salt with an organic acid (for example, salt with methanesulfonic acid, benzenesulfonic acid, p-toluenesulfonic acid, formic acid, acetic acid, trifluoroacetic acid, oxalic acid, citric acid, malonic acid, fumaric acid, glutaric acid, adipic acid, maleic acid, tartaric acid, succinic acid, mandelic acid, maleic acid or the like), salt with an amino acid (for example, salt with glutamic acid, aspartic acid or the like) and the like.

Suitable examples as compound (IV) include compounds of formula (IV) wherein Y⁴⁰⁰ is aryl optionally having substituents; compounds of formula (IV) wherein Z is the group of formula (IV-i); compounds of formula (IV) wherein one of R⁴⁰⁵, R⁴⁰⁶ and R⁴⁰⁷ is aryl optionally having substituents with the rest being hydrogen (R⁴⁰⁶ is absent when M⁴⁰⁰ is nitrogen); and the like.

Among the compounds of formula (IV), it was reported that the compound represented by the following formula (IV-I) exhibit activity as a chymase inhibitor in a mouse allergy model and the like through oral administration (WO 00/51640, J. Med. Chem., 2001, vol. 44, p.1286). Therefore it is expected that this compound will be effective as a chymase inhibitor in the present invention on various diseases associated with glucose intolerance.

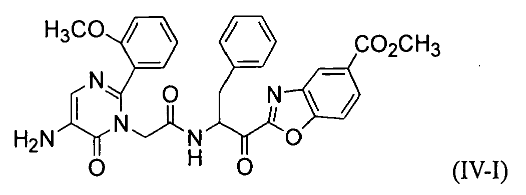

So far, there has been reported a number of chymase inhibitors other than described above. Either of these is potentially valuable for glucose intolerance and various diseases associated with it by using as a chymase inhibitor in the present invention.

An example of such chymase inhibitors is the N-substituted benzothiophenesulfonamide derivative represented by formula (V) or salt thereof described in WO 02/22595: 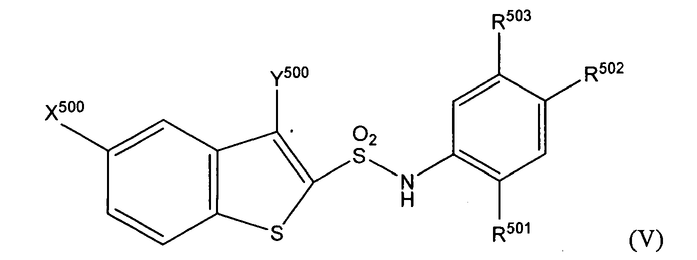 [wherein X⁵⁰⁰ represents hydrogen, halogen or lower alkyl, Y⁵⁰⁰ represents lower alkyl, R⁵⁰¹ and R⁵⁰² each may be different and represent independently hydrogen, lower alkoxycarbonyl, lower alkylsulfonyl, benzoyl, C₁-C₄ acyl, lower alkoxy, lower alkoxycarbonylmethylthioacetyl, nitro, -CONHR⁵⁰⁴ (wherein R⁵⁰⁴ represents hydrogen, lower alkoxycarbonylmethyl, carboxymethyl or -CH(CH₂OH)COOR⁵⁰⁵ (wherein R⁵⁰⁵ represents hydrogen or lower alkyl)); the group represented by 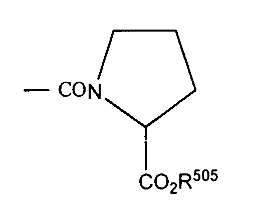 (wherein R⁵⁰⁵ is as defined above), the monocyclic heterocyclyl represented by 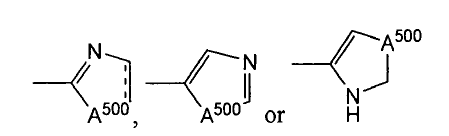 optionally substituted with -CO₂R⁵⁰⁵ (wherein A⁵⁰⁰ represents O, S or NH, the bond accompanying a dotted line represents a single or double bond, and R⁵⁰⁵ is as defined above), lower hydroxyalkyl or cyano (except for cases where both R⁵⁰¹ and R⁵⁰² are hydrogen), R⁵⁰³ represents hydrogen, lower alkoxy or lower alkyl] (except for compounds represented by the following formulas).

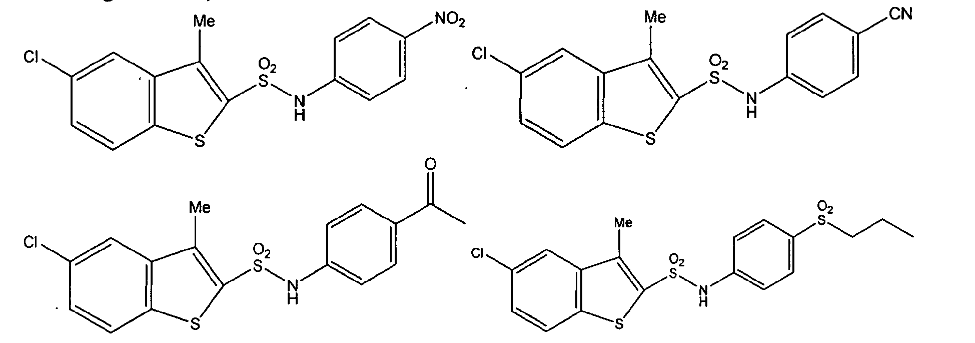

### Each of the terms in formula (V) is defined as follows:

The halogen as X⁵⁰⁰ is F, Cl, Br or I, preferably F or Cl. The lower alkyl as X⁵⁰⁰ is for example, methyl, ethyl, propyl, isopropyl, butyl, isobutyl, sec-butyl or tert-butyl, preferably methyl or ethyl.

The lower alkyl as Y⁵⁰⁰ is for example, methyl, ethyl, propyl, isopropyl, butyl, isobutyl, sec-butyl or tert-butyl, preferably methyl or ethyl.

The lower alkoxycarbonyl as R⁵⁰¹ or R⁵⁰² is for example, methoxycarbonyl, ethoxycarbonyl, propoxycarbonyl, isopropoxycarbonyl, butoxycarbonyl, isobutoxycarbonyl, sec-butoxycarbonyl or tert-butoxycarbonyl, preferably methoxycarbonyl, ethoxycarbonyl, isopropoxycarbonyl or tert-butoxycarbonyl.

The lower alkylsulfonyl as R⁵⁰¹ or R⁵⁰² is for example, methanesulfonyl, ethanesulfonyl, propanesulfonyl, isopropanesulfonyl, butanesulfonyl, isobutanesulfonyl, sec-butanesulfonyl or tert-butanesulfonyl, preferably methanesulfonyl or ethanesulfonyl.

The C₁-C₄ acyl as R⁵⁰¹ or R⁵⁰² is for example, formyl, acetyl, propionyl, butyryl or isobutyryl, preferably acetyl.

The lower alkoxy as R⁵⁰¹, R⁵⁰² or R⁵⁰³ is for example, methoxy, ethoxy, propoxy, isopropoxy, butoxy, isobutoxy, sec-butoxy or tert-butoxy, preferably methoxy or ethoxy.

The lower alkoxycarbonylmethylthioacetyl as R⁵⁰¹ or R⁵⁰² is for example, methoxycarbonylmethylthioacetyl, ethoxycarbonylmethylthioacetyl, propoxycarbonylmethylthioacetyl, isopropoxycarbonylmethylthioacetyl, butoxycarbonylmethylthioacetyl, isobutoxycarbonylmethylthioacetyl, sec-butoxycarbonylmethylthioacetyl or tert-butoxycarbonylmethylthioacetyl, preferably methoxycarbonylmethylthioacetyl or ethoxycarbonylmethylthioacetyl.

When R⁵⁰¹ or R⁵⁰² is -CONHR⁵⁰⁴, the lower alkoxycarbonylmethyl as R⁵⁰⁴ is for example, methoxycarbonylmethyl, ethoxycarbonylmethyl, propoxycarbonylmethyl, isopropoxycarbonylmethyl, butoxycarbonylmethyl, isobutoxycarbonylmethyl, sec-butoxycarbonylmethyl or tert-butoxycarbonylmethyl, preferably methoxycarbonylmethyl, ethoxycarbonylmethyl or isopropoxycarbonylmethyl.

When R⁵⁰¹ or R⁵⁰² is -CONHR⁵⁰⁴ and R⁵⁰⁴ is -CH(CH₂OH)COOR⁵⁰⁵, the lower alkyl as R⁵⁰⁵ is for example, methyl, ethyl, propyl, isopropyl, butyl, isobutyl, sec-butyl or tert-butyl, preferably methyl or ethyl.

When R⁵⁰¹ or R⁵⁰² is the group represented by 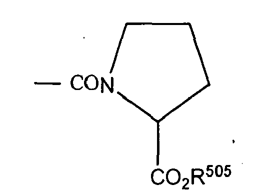 , the lower alkyl as R⁵⁰⁵ is as defined above.
When R⁵⁰¹ or R⁵⁰² is monocyclic heterocyclyl represented by 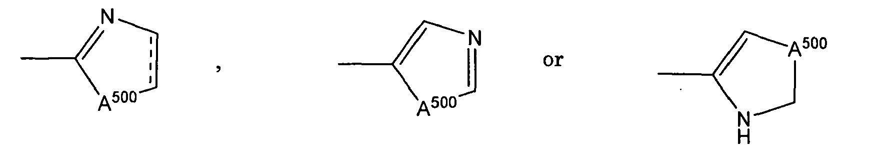 (wherein A⁵⁰⁰ represents O, S or NH and the bond accompanying a dotted line represents a single or double bond) which may be optionally substituted with CO₂R⁵⁰⁵, the lower alkyl as R⁵⁰⁵ is as defined above. Examples of such monocyclic heterocyclyl represented by 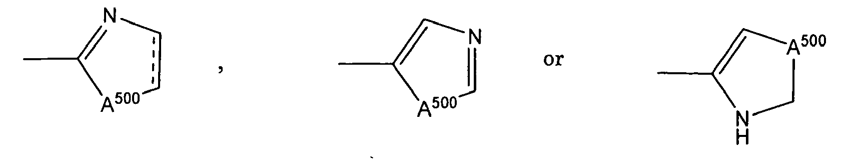 is 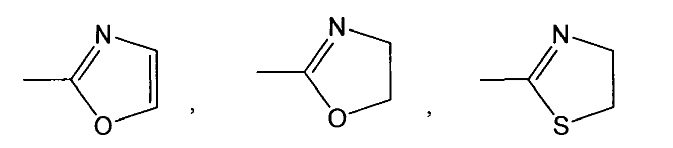 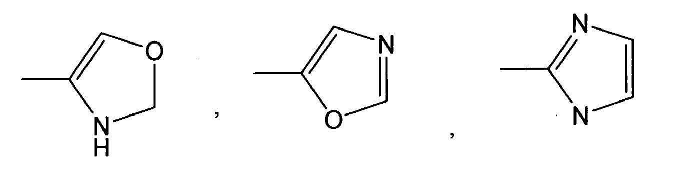 Specifically, preferred groups are 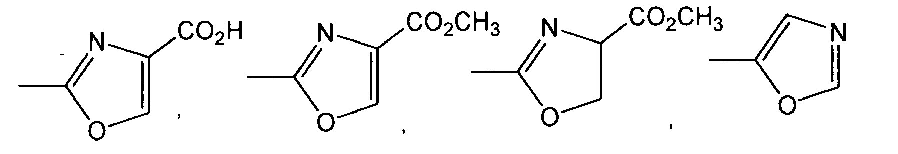 These substituents are preferably present as R⁵⁰². In this case, further preferably R⁵⁰¹ is methanesulfonyl and R⁵⁰³ is hydrogen.

The lower hydroxyalkyl as R⁵⁰¹ or R⁵⁰² is straight or branched lower hydroxy(C₁-C₄)alkyl, for example, hydroxymethyl, hydroxyethyl, hydroxypropyl, hydroxybutyl or the like, preferably hydroxymethyl, 1-hydroxyethyl or 2-hydroxyethyl.

The lower alkyl as R⁵⁰³ is for example, methyl, ethyl, propyl, isopropyl, butyl, isobutyl, sec-butyl or tert-butyl, preferably methyl or ethyl.

Examples of the compound (V) are specifically methyl
4-(5-chloro-3-methylbenzo[b]thiophene-2-sulfonylamino)-3-methanesulfonylbenzoate, sodium
4-(5-chloro-3-methylbenzo[b]thiophene-2-sulfonylamino)-3-methanesulfonylbenzoate, isopropyl
4-(5-chloro-3-methylbenzo[b]thiophene-2-sulfonylamino)-3-methanesulfonylbenzoate, N-(4-acetyl-2-methanesulfonylphenyl)-5-chloro-3-methylbenzo[b]thiophene-2-sulfonamide,
N-(4-benzoyl-2-methanesulfonylphenyl)-5-chloro-3-methylbenzo[b]thiophene-2-sulfonamide, ethyl 4-(5-chloro-3-methylbenzo[b]thiophene-2-sulfonylamino)-3-methanesulfonylbenzoate, tert-butyl
4-(5-chloro-3-methylbenzo[b]thiophene-2-sulfonylamino)-3-methanesulfonylbenzoate, methyl 4-(5-chloro-3-methylbenzo[b]thiophene-2-sulfonylamino)-3-ethanesulfonylbenzoate, methyl 4-(5-chloro-3-methylbenzo[b]thiophene-2-sulfonylamino)-5-methanesulfonyl-2 -methylbenzoate, dimethyl
4-(5-chloro-3-methylbenzo[b]thiophene-2-sulfonylamino)isophthalate, methyl
4-(5-chloro-3-methylbenzo[b]thiophene-2-sulfonylamino)-3-methoxybenzoate, methyl
4-(5-chloro-3-methylbenzo[b]thiophene-2-sulfonylamino)-3-nitrobenzoate, ethyl
4-(5-chloro-3-methylbenzo[b]thiophene-2-sulfonylamino)benzoate,
N-[2,4-bis(methanesulfonyl)phenyl]-5-chloro-3-methylbenzo[b]thiophene-2-sulfonamide,
N-(4-acetyl-2-nitrophenyl)-5-chloro-3-methylbenzo[b]thiophene-2-sulfonamide,
N-(4-hydroxymethyl-2-methanesulfonylphenyl)-5-chloro-3-methylbenzo[b]thiophene-2 -sulfonamide, N-(4-benzoylphenyl)-5-chloro-3-methylbenzo[b]thiophene-2-sulfoamide,
N-(2-methanesulfonylphenyl)-5-chloro-3-methylbenzo[b]thiophene-2-sulfonamide, methyl
4-(5-fluoro-3-methylbenzo[b]thiophene-2-sulfonylamino)-3-methanesulfonylbenzoate, methyl 4-(5-methyl-3-methylbenzo [b]thiophene-2-sulfonylamino) -3-methanesulfonylbenzoate,
N-(4-acetyl-2-methanesulfonylphenyl)-5-fluoro-3-methylbenzo[b]thiophene-2-sulfonamide, methyl 4-(3-methylbenzo[b]thiophene-2-sulfonylamino)-3-methanesulfonylbenzoate, methyl 2-[4-(5-chloro-3-methylbenzo[b]thiophene-2-sulfonylamino)-3-methanesulfonylphenyl] oxazole-4-carboxylate, methyl
2-[4-(5-fluoro-3-methylbenzo[b]thiophene-2-sulfonylamino)-3-methanesulfonylphenyl] oxazole-4-carboxylate,
2-[4-(5-chloro-3-methylbenzo[b]thiophene-2-sulfonylamino)-3-methanesulfonylphenyl] oxazole-4-carboxylic acid,
2-[4-(5-fluoro-3-methylbenzo[b]thiophene-2-sulfonylamino)-3-methanesulfonylphenyl] oxazole-4-carboxylic acid, sodium
2-[4-(5-chloro-3-methylbenzo[b]thiophene-2-sulfonylamino)-3-methanesulfonylphenyl] oxazole-4-carboxylate, sodium
2-[4-(5-fluoro-3-methylbenzo[b]thiophene-2-sulfonylamino)-3-methanesulfonylphenyl] oxazole-4-carboxylate and
2-[4-(5-fluoro-3-methylbenzo[b]thiophen-2-yl)sulfonamido-3-methanesulfonylphenyl] oxazole-4-carboxylic acid.

The chymase inhibitor also includes
4-[1-{[bis(4-methylphenyl)methyl]carbamoyl}-3-(2-ethoxybenzyl)-4-oxoazetidin-2-yloxy] benzoic acid and
2-(5-formylamino-6-oxo-2-phenyl-1,6-dihydropyrimidin-1-yl)-N-[{3,4-dioxo-1-phenyl-7-(2-pyridyloxy)}-2-heptyl]acetamide.

Other chymase inhibitors proposed so far include for example, compounds described in WO 01/32214, WO 02/18378, WO 01/12226, WO 01/32621, Japanese published unexamined application H10-87493, Japanese published unexamined application H11-1479, Japanese published unexamined application H10-251239, Japanese published unexamined application H8-208654, Japanese published unexamined application 2001-97957 and Japanese published unexamined application 2000-95770.

Any compound that inhibits human chymase activity may be used as a chymase inhibitor in the present invention. Specifically, it is the chymase inhibitor with an IC₅₀ value, determined by method (A) for IC₅₀ assay described below, of preferably 1000 nM or less, more preferably 500 nM or less, further preferably 100 nM or less, still further preferably 10 nM or less.

The method (A) for IC₅₀ assay is as follows. First, recombinant human mast cell prochymase is prepared according to the report of Urata et al. (J. Biol. Chem., vol. 266, p. 17173 (1991)). Namely, prochymase is purified from supernatant of a culture medium of insect cells (Th5) infected with recombinant baculoviruses containing cDNA coding human mast cell chymase, by heparin-sepharose. After activation of the prochymase according to the report of Murakami et al. (J. Biol. Chem., vol. 270, p. 2218 (1995)), purification on heparin-sepharose column gives active form of human mast cell chymase. Next, the inhibitory activity against recombinant human mast cell chymase is assayed. To 50 µL of buffer A (0.5-3.0 M NaCl, 50 mM Tris-HCl, pH 8.0) containing 1-5 ng of active form of human mast cell chymase prepared above, are added 2 µL of DMSO solution containing a chymase inhibitor and then 50 µL of buffer A containing 0.5 mM succinyl-alanyl-histidyl-prolyl-phenylalanyl-p-nitroanilide as a substrate. The resultant mixture is kept at room temperature for 5 min to allow the reaction to occur. The time course of absorbance at 405 nm is monitored to determine the inhibitory activity. This method is the same as that described in Examples 16 and 17 in WO 01/53291.

Other methods for IC₅₀ assay are described in Examples 19 and 20 in WO 01/53272, Examples 22 and 23 in WO 00/03997, Test example 1 in WO 00/005204, Test example 1 in WO 98/009949 and Experimental example 1 in WO 98/018794. In each of these reports it was confirmed that the compound described therein exhibits inhibitory activity against chymase by using the method described therein.

Furthermore, the drugs for improving glucose intolerance containing chymase inhibitors of the present invention as active ingredients can be used together with other drugs for improving glucose intolerance, improving insulin resistance or treating diabetes and/or diabetes complications, and in some cases synergistic effects may be expected by combination. Drugs that may be used together include PPARγ agonists such as rosiglitazone and pioglitazone, which improve glucose intolerance, and the like.

Also, it was reported that AT1 receptor antagonists which suppress major functions of angiotensin II irrespective of production pathways of angiotensin II, being ACE-dependent or ACE-independent, exhibit activity for improving insulin resistance (Effects of angiotensin receptor antagonist and angiotensin converting enzyme inhibitor on insulin sensitivity in fructose-fed hypertensive rats and essential hypertensives, American Journal of Hypertension, USA, 1995, vol. 8, part 4, No. 1, p. 353), that ACE inhibitors for treating hypertension such as captopril (CARPPP clinical study) and ramipril (HOPE study) or losartan which is an AT1 receptor antagonist (LIFE study) suppress new onset of diabetes in large scale clinical tests (Cardiovascular morbidity and mortality in the losartan intervention for endpoint reduction in hypertension study (LIFE): a randomised trial against atenolol, Lancet, USA, 2002, vol. 359, no. 9311, p. 995), and that imidapril which is an ACE inhibitor has been indicated for diabetic nephropathy associated with type I diabetes. Accordingly, it is preferred to use these drugs such as ACE inhibitors together with the drugs of the present invention.

The drug of the present invention may be in any dosage form as long as it contains a chymase inhibitor as an active ingredient.

Possible dosage forms include tablets, pills, granules, powder, liquid, suspension, syrup, capsules and the like. The dosage form is not particularly limited and may be a solid-solid, liquid-liquid or solid-liquid mixture. The drug may be administrated orally or parenterally.

Among the chymase inhibitors in the present invention, benzimidazole derivatives represented by formula (I) are preferably administrated orally or parenterally as medicinal compositions with pharmaceutically acceptable carriers in various dosage forms.

Possible dosage forms for the medicinal compositions in the present invention include, in the case of oral administration, tablets, pills, granules, powder, liquid, suspension, syrups, capsules and the like.

Here tablets can be shaped with pharmaceutically acceptable carriers such as excipients, binders and/or disintegrants by usual methods. Pills, granules and powder can similarly be formed by usual methods with excipients and the like. Liquid, suspension and syrups can be formed by usual methods with glycerol esters, alcohols, water and/or vegetable oils. Capsules can be formed by filling capsules such as gelatin with granules, powder or liquid.

### EXAMPLES

The present invention will be explained by an example bellow, which should not be construed to limit the scope of the present invention in any sense.

### [EXAMPLE 1]

### Improving activity for glucose intolerance

A group of 22 weeks-old wild type mice (C57Black) (Wild), a group of those in which human chymase gene was expressed (TGM) and a group of TGM that had been given feed containing 0.1 % sulfate of compound 58 (the IC₅₀ value of compound 58 is between 1 nM and 10 nM) as a chymase inhibitor (ChI) continuously for 12 weeks since 10 weeks old (TGM/ChI) were made fast overnight and orally administrated with 1.5 g/kg glucose. At 60 min after glucose load the concentrations of glucose and insulin in blood were assayed.

### Results:

At 60 min after glucose load, the blood glucose levels were 119±20 mg/dl for Wild, 181±22 mg/dl for TGM and 134±18 mg/dl^{*} for TGM/ChI (mean±SD, ^{*}p<0.01 vs. Wild, p=0.01 vs. TGM), indicating that the blood glucose level after glucose load significantly increased in TGM and that administration of ChI significantly repressed the increase (Fig.1).

On the other hand, the insulin concentrations in blood at this time were 386±97 ng/l for Wild, 809±288 ng/l for TGM and 425±158 ng/l for TGM/ChI (mean±SD), indicating that it significantly increased in TGM and that administration of ChI significantly repressed this increase, as in the case of the blood glucose level (Fig. 2).

It has been found that, compared with the wild type mice, mice in which human chymase gene is expressed exhibit significantly high values of the blood glucose level and the blood insulin concentration, showing that the glucose intolerance is caused by the expression of human chymase. It has also been shown that administration of a chymase inhibitor remarkably reduces the blood glucose level and the blood insulin concentration, improving glucose intolerance. Accordingly, it is clear that chymase inhibitors used in the present invention are inhibitors against human chymase that can be clinically applicable to inhibiting and/or treating various diseases associated with glucose intolerance induced by human chymase.

### INDUSTRIAL APPLICABILITY

The drugs comprising chymase inhibitors in the present invention can be used for improving glucose intolerance or for preventing and/or treating diabetes and/or diabetes complications such as diabetic nephropathy, diabetic retinopathy, diabetic peripheral neuropathy, hyperinsulinism, insulin resistance syndrome, arteriosclerosis, acute coronary syndrome, arteriosclerosis obliterans, angitis, stroke, hypertension, renal insufficiency, nephropathy, nephritis, renal artery aneurysm, renal infarction, obesity and the like.

## Claims

1. A drug for improving glucose intolerance comprising a chymase inhibitor as an active ingredient.

2. A preventive drug and/or therapeutic drug of diseases caused by glucose intolerance comprising a chymase inhibitor as an active ingredient.

3. A preventive and/or therapeutic drug according to claim 2 wherein the diseases caused by glucose intolerance are diabetes and/or diabetes complications.

4. A preventive and/or therapeutic drug according to claim 3 wherein the diabetes complications are diabetic nephropathy, diabetic retinopathy, diabetic peripheral neuropathy, hyperinsulinism, insulin resistance syndrome, arteriosclerosis, acute coronary syndrome, arteriosclerosis obliterans, angitis, stroke, hypertension, renal insufficiency, nephropathy, nephritis, renal artery aneurysm, renal infarction or obesity.

5. A preventive and/or therapeutic drug according to claim 3 wherein the diabetes complications are diabetic nephropathy, diabetic retinopathy or diabetic peripheral neuropathy.

6. A drug described according to any of claims 1-5 containing a chymase inhibitor at an amount sufficient for improving glucose intolerance.

7. A drug described in any of claims 1-6 comprising an ACE inhibitor.

8. A drug described according to any of claims 1-7 wherein the chymase inhibitor is the compound represented by formula (I): 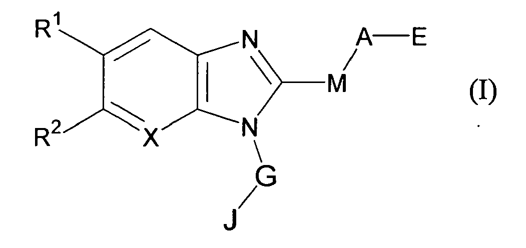 [wherein R¹ and R² simultaneously or each independently represent hydrogen, halogen, trihalomethyl, cyano, hydroxyl, C₁-C₄ alkyl or C₁-C₄ alkoxy, or R¹ and R² taken together represent -O-CH₂-O-, -O-CH₂CH₂-O- or -CH₂CH₂CH₂-, (wherein the carbon atoms may be optionally substituted by one or more C₁-C₄ alkyl);
A represents substituted or unsubstituted straight, cyclic or branched C₁-C₇ alkylene or alkenylene, which may be interrupted by one or more of atoms or groups selected from -O-, -S-, -SO₂- and -NR³- (wherein R³ represents hydrogen or straight or branched C₁-C₆ alkyl), the substituents on these groups being selected from halogen, hydroxyl, nitro, cyano, straight or branched C₁-C₆ alkyl, straight or branched C₁-C₆ alkoxy (including cases wherein the neighboring two form an acetal), straight or branched C₁-C₆ alkylthio, straight or branched C₁-C₆ alkylsulfonyl, straight or branched C₁-C₆ acyl, straight or branched C₁-C₆ acylamino, trihalomethyl, trihalomethoxy, phenyl, oxo or phenoxy optionally substituted with one or more halogen atoms, wherein one or more of these substituents may each independently be present at any position in the alkylene or alkenylene, except for the case wherein M represents a single bond and the carbon atom of A directly bonded to M is substituted with a hydroxyl and a phenyl at the same time;
E represents -COOR³, -SO₃R³, -CONHR³, -SO₂NHR³, tetrazol-5-yl, 5-oxo-1,2,4-oxadiazol-3-yl or 5-oxo-1,2,4-thaidiazol-3-yl, (wherein R³ is as defined above);
G represents substituted or unsubstituted straight or branched C₁-C₆ alkylene, which may be interrupted by one or more of atoms or groups selected from -O-, -S-, -SO₂- and -NR³- (wherein R³ is as defined above, provided that either of these atoms or groups is not directly attached to the benzimidazole ring), the substituents on the said alkylene being selected from halogen, hydroxyl, nitro, cyano, straight or branched C₁-C₆ alkyl, straight or branched C₁-C₆ alkoxy (including cases wherein neighboring two form an acetal), trihalomethyl, trihalomethoxy, phenyl or oxo;
M represents a single bond or -S(O)ₘ-, wherein m is an integer ranging from 0 to 2;
J represents substituted or unsubstituted C₄-C₁₀ heteroaryl (one or more heteroatoms selected from the group consisting of oxygen, nitrogen and sulfur in the ring), except for imidazole or unsubstituted pyridine ring, the substituents on the said heteroaryl are halogen, hydroxyl, nitro, cyano, straight or branched C₁-C₆ alkyl, straight or branched C₁-C₆ alkoxy (including cases wherein neighboring two form an acetal), straight or branched C₁-C₆ alkylthio, straight or branched C₁-C₆ alkylsulfonyl, straight or branched C₁-C₆ acyl, straight or branched C₁-C₆ acylamino, substituted or unsubstituted anilido, trihalomethyl, trihalomethoxy, phenyl, oxo, COOR³ or phenoxy optionally substituted with one or more halogen atoms, wherein one or more of these substituents may each independently be present at any position in the ring; and X represents -CH= or nitrogen].

9. A drug according to claim 8 wherein, in formula (I), R¹ and R² are simultaniously or each independently hydrogen, C₁-C₄ alkyl, C₁-C₄ alkoxy, halogen or cyano;
A is n-propylene;
E is -COOH;
G is methylene;
M is -S-;
J is substituted or unsubstituted benzothienyl or indolyl (wherein the substituent is halogen, hydroxyl, nitro, cyano, straight or branched C₁-C₆ alkyl, straight or branched C₁-C₆ alkoxy (including cases wherein neighboring two form an acetal), straight or branched C₁-C₆ alkylthio, straight or branched C₁-C₆ alkylsulfonyl, straight or branched C₁-C₆ acyl, straight or branched C₁-C₆ acylamino, substituted or unsubstituted anilido, trihalomethyl, trihalomethoxy, phenyl, oxo, COOR³ or phenoxy optionally substituted with one or more halogen atoms, wherein one or more of these substituents may each independently be present at any position in the ring); and
X is -CH=.

10. A drug according to claim 8 or 9 wherein R¹ and R² are simultaneously or each independently hydrogen, C₁-C₄ alkyl or C₁-C₄ alkoxy.

11. A drug according to claim 10 wherein R¹ and R² are simultaneously or each independently hydrogen, methyl or methoxy.

12. A drug according to any of claims 8-11 wherein J is benzothienyl.

13. A drug according to any of claims 8-12 wherein the substituent on J is halogen, cyano, straight or branched C₁-C₄ alkyl, straight or branched C₁-C₄ alkoxy (including cases wherein neighboring two form an acetal) or trihalomethyl.

14. A drug according to claim 13 wherein the substituent on J is F, Cl, cyano, methyl, methoxy or trifluoromethyl.

15. A drug according to claim 14 wherein the substituent on J is methyl.

16. A drug according to any of claims 1-7 wherein the chymase inhibitor is
4-(1-((3-indolyl)methyl)benzimidazol-2-ylthio)butanoic acid,
4-(1-((3-benzo[b]thienyl)methyl)-5-methoxybenzimidazol-2-ylthio)butanoic acid,
4-(1-((5-methylbenzo[b]thiophen-3-yl)methyl)-5-methoxybenzimidazol-2-ylthio)butanoic acid,
4-(1-((4-methylbenzo[b]thiophen-3-yl)methyl)-5-methoxybenzimidazol- 2-ylthio)butanoic-acid,
4-(1-((3-benzo[b]thienyl)methyl)-5-cyanobenzimidazol-2-ylthio)butanoic acid,
4-(1-((5-methylbenzo[b]thiophen-3-yl)methyl)-6-methoxybenzimidazol-2-ylthio)butanoic acid,
4-(1-((4-methylbenzo [b]thiophen-3-yl)methyl)-6-methoxybenzimidazol-2-ylthio)butanoic acid,
4-(1-((1,5-dimethylindol-3-yl)methyl)benzimidazol-2-ylthio)butanoic acid,
4-(1 -((1 -methyl-4-chloroindol-3 -yl)methyl)benzimidazol-2-ylthio)butanoic acid,
4-(1 -((1 -methyl-4-fluoroindol-3-yl)methyl)benzimidazol-2-ylthio)butanoic acid,
4-(1-((5-chlorobenzo[b]thiophen-3-yl)methyl)benzimidazol-2-ylthio)butanoic acid,
4-(1-((5-methylbenzo[b]thiophen-3-yl)methyl)benzimidazol-2-ylthio)butanoic acid,
4-(1-((4-methylbenzo[b]thiophen-3-yl)methyl)benzimidazol-2-ylthio)butanoic acid,
4-(1-((4-chlorobenzo[b]thiophen-3-yl)methyl)benzimidazol-2-ylthio)butanoic acid,
4-(1-((4,6-dimethylbenzo[b]thiophen-3-yl)methyl)benzimidazol-2-ylthio)butanoic acid,
4-(1-((1-methylindol-3-yl)methyl)-5,6-dimethylbenzimidazol-2-ylthio)butanoic acid,
4-(1-((1,4-dimethylindol-3-yl)methyl)-5,6-dimethylbenzimidazol-2-ylthio)butanoic acid,
4-(1-((1-methyl-4-chloroindol-3-yl)methyl)-5,6-dimethylbenzimidazol-2-ylthio)butanoic acid,
4-(1-((benzo[b]thiophen-3-yl)methyl)-5,6-dimethylbenzimidazol-2-ylthio)butanoic acid,
4-(1-((5-chlorobenzo[b]thiophen-3-yl)methyl)-5,6-dimethylbenzimidazol-2-ylthio)butanoic acid,
4-(1-((5-methylbenzo [b]thiophen-3-yl)methyl)-5,6-dimethylbenzimidazol-2-ylthio)butanoic acid,
4-(1-((4-methylbenzo[b]thiophen-3-yl)methyl)-5,6-dimethylbenzimidazol-2-ylthio)butanoic acid,
4-(1-((1,4-dimethylindol-3-yl)methyl)-5,6-dichlorobenzimidazol-2-ylthio)butanoic acid,
4-(1-((benzo[b]thiophen-3-yl)methyl)-5,6-dichlorobenzimidazol-2-ylthio)butanoic acid,
4-(1-((benzo[b]thiophen-3-yl)methyl)benzimidazol-2-ylthio)butanoic acid,
4-(1-((benzo[b]thiophen-3-yl)methyl)-5-methylbenzimidazol-2-ylthio)butanoic acid,
4-(1-((benzo[b]thiophen-3-yl)methyl)-6-methylbenzimidazol-2-ylthio)butanoic acid,
4-(1-((1,4-dimethylindol-3-yl)methyl)benzimidazol-2-ylthio)butanoic acid,
4-(1-((1,4-dimethylindol-3-yl)methyl)-5-methoxybenzimidazol-2-ylthio)butanoic acid or
4-(1-((1-methyl-4-chloroindol-3-yl)methyl)-5-methoxybenzimidazol-2-ylthio)butanoic acid.

17. A drug according to any of claims 1-7 wherein the chymase inhibitor is 4-(1-((4-methylbenzo[b]thiophen-3-yl)methyl)benzimidazol-2ylthio)butanoic acid.

18. A drug described in any of claims 1-7 wherein the chymase inhibitor is the compound represented by formula (II), a prodrug, a pharmaceutically acceptable salt thereof or a hydrate thereof: 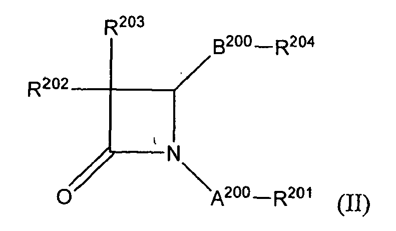 [wherein A²⁰⁰ represents a single bond, -CO-, -COO-, -COCO-, -CONH- or -SO₂-, R²⁰¹ represents lower alkyl optionally having substituents, lower alkenyl optionally having substituents, lower alkynyl optionally having substituents, cycloalkyl optionally having substituents, cycloalkenyl optionally having substituents or aryl optionally having substituents, R²⁰¹ may be hydrogen when A²⁰⁰ is a single bond, -CO-, -COCO-, -CONH- or -SO₂-, R²⁰² and R²⁰³ are each independently hydrogen, halogen, lower alkyl optionally having substituents, lower alkoxycarbonyl optionally having substituents, acyl optionally having substituents, amino optionally having substituents, carbamoyl optionally having substituents or aryl optionally having substituents, B²⁰⁰ represents a single bond, -S-, -O-, -S-S-, -SO- or -SO₂-, R²⁰⁴ represents hydrogen, lower alkyl optionally having substituents, aryl optionally having substituents or heterocyclyl optionally having substituents, and R²⁰⁴ may be acyl optionally having substituents when B²⁰⁰ is a single bond, -S-, -O-, -SO- or -SO₂-].

19. A drug of claim 18 wherein the chymase inhibitor is the compound represented by formula (II'), a prodrug, a pharmaceutically acceptable salt thereof or a hydrate thereof: 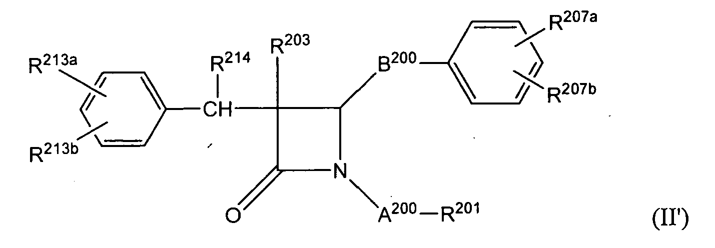 (wherein A²⁰⁰ and R²⁰¹ are as defined for formula (II), R²⁰³ represents hydrogen, halogen, lower alkoxycarbonyl optionally having substituents, acyl optionally having substituents, amino optionally having substituents, aryl optionally having substituents or benzyl optionally having substituents, R^{213a} and R^{213b} each independently represent hydrogen, halogen, hydroxyl, lower alkyl optionally having substituents, lower alkoxy optionally having substituents, amino optionally having substituents or lower alkylthio optionally having substituents, or R^{213a} and R^{213b} taken together form lower alkylenedioxy, R²¹⁴ represents hydrogen, hydroxyl, lower alkyl, lower alkoxy or acyloxy, R^{207a} represents hydrogen, 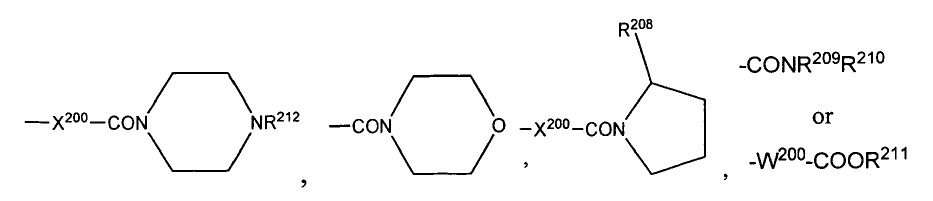 (wherein X²⁰⁰ and W²⁰⁰ represent a single bond, methylene or vinylene, R²⁰⁸ represents methyl or carbamoyl, R²⁰⁹ represents hydrogen or lower alkyl, R²¹⁰ represents lower alkyl optionally having substituents (lower alkylamino; phenyl optionally substituted with halogen; carboxyl; or lower alkoxycarbonyl optionally substituted with aryl), lower alkenyl, lower alkylamino, phenylamino, phenyl or benzenesulfonyl, R²¹¹ represents hydrogen or lower alkyl optionally having substituents (lower alkylamino; acyloxy; phenyl optionally substituted with halogen or methylenedioxy; or heterocyclyl) and R²¹² represents C₁-C₃ alkyl or cyclohexyl), R^{207b} is hydrogen, and B²⁰⁰ is O or S).

20. A drug according to claim 18 wherein the chymase inhibitor is
4-[1-[N-[bis(4-methylphenyl)methyl]carbamoyl]-3-(2-ethoxybenzyl)-4-oxoazetidin-2-yloxy] benzoic acid or
4-[1-[{(bis(4-methoxyphenyl)methyl]carbamoyl}-3-(2-ethoxybenzyl)-4-oxoazetidin-2-yloxy] benzoic acid, a prodrug, a pharmaceutically acceptable salt thereof or a hydrate thereof.

21. A drug according to any of claims 1-7 wherein the chymase inhibitor is the novel acetamide derivative represented by formula (III) below or a pharmaceutically acceptable salt thereof: 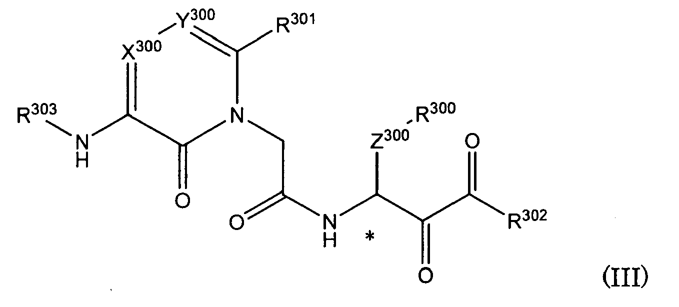 [wherein R³⁰⁰ is phenyl, which may have one or more substituents selected from group A³⁰⁰ defined below (wherein A³⁰⁰ is halogen, nitro, hydroxyl, lower alkoxy, lower alkyl or halogenated lower alkyl);
R³⁰¹ is (III-i) aryl, (III-ii) heteroaryl or (III-iii) straight, branched or cyclic C₁-C₆ alkyl and may have each independently one or more substituents selected from group A³⁰⁰; or R³⁰¹ may have, on group (III-i) - (III-iii), one or more substituents selected from group B³⁰⁰, consisting of OR^{300a}, COOR^{300a}, CONR^{300b}R^{300c}, NR^{300b}R^{300c}, NR^{300b}CHO, NR^{300b}COR^{300a}, SO₂OR^{300a}, SO₂R^{300a}, CONR^{300b}SO₂R^{300a} and P(O)(OR^{300a})₂ (wherein, R^{300a}-R^{300c} are independently hydrogen, lower alkyl or substituted lower alkyl; or R^{300a}-R^{300c} are independently aryl(C₁-C₇)alkyl, heteroaryl(C₁-C₇)alkyl, aryl or heteroaryl wherein the ring of aryl or heteroaryl may have one or more, usually one to three substituents selected from group A and the lower alkyl has one to three substituents selected from halogen, nitro and hydroxyl); or R³⁰¹ may have on group (III-i) - (III-iii) one or more substituents selected from cyclic group G³⁰⁰, (wherein G³⁰⁰ represents five- or six-membered heterocyclyl having one to three oxygen or nitrogen and optionally have substituents);
R³⁰² is C₁-C₈ alkyl, aryl(C₁-C₇)alkyl, heteroaryl(C₁-C₇)alkyl or aryl; or R³⁰² is group B³⁰⁰ defined above, C₁-C₈ alkyl substituted with group B³⁰⁰ or C₁-C₈ alkyl substituted with cyclic group G³⁰⁰ defined above;
R³⁰³ is hydrogen; or R³⁰³ is acyl represented by (i) D³⁰⁰(CH₂)₀₋₃CO, (ii) D³⁰⁰COE³⁰⁰CO or (iii) D³⁰⁰SO₂E³⁰⁰CO; or R³ is sulfonyl represented by D³⁰⁰(CH₂)₀₋₃SO₂ or D³⁰⁰COE³⁰⁰SO₂ (wherein group D³⁰⁰ is hydrogen, straight, branched or cyclic C₁-C₆ alkyl, aryl, halogenated lower alkyl, halogenated lower alkoxy, amino, lower alkoxyamino, halogenated lower alkylamino, R^{300b}R^{300c}N, R^{300b}R^{300c}NO, R^{300a}O, R^{300a}, R^{300a}OCO, R^{300b}R^{300c}NCO, R^{300a}SO₂NR^{300b}, R^{300a}S, or cyclic group G³⁰⁰ as defined above, and group E³⁰⁰ represents divalent bridging group having 1 to 6 carbon atoms); or R³⁰³ is urea represented by R^{300b}R^{300c}NCO; or R³⁰³ is thiourea represented by R^{300b}R^{300c}NCS; or R³⁰³ is R^{303a};
X³⁰⁰ and Y³⁰⁰ each represent independently nitrogen or carbon and may be substituted with a group represented by R^{300a}-R^{300c}; and
Z³⁰⁰ is polymethylene wherein each hydrogen may independently be substituted with R^{300a} or R^{300b}].

22. A drug according to claim 21 wherein R³⁰⁰ is unsubstituted phenyl, R³⁰¹ is unsubstituted phenyl, R³⁰² is unsubstituted C₁-C₈ alkyl or C₁-C₈ alkyl having substituents selected from pyrrolidin-1-yl, pyridyloxy, 2-oxo-1,2-dihydropyridin-1-yl, pyrimidyloxy, pirazyloxy, pyridazyloxy, lower alkyl-substituted piperazin-1-yl or lower alkyl-substituted piperazin-1-ylcarbonyl, X is unsubstituted carbon, Y is nitrogen and Z is -CH₂-.

23. A drug according to claim 21 wherein the chymase inhibitor is
2-(5-substituted-6-oxo-2-phenyl-1,6-dihydropyrimidin-1-yl)-N-{2,3-dioxo-6-(2-pyridyloxy)-1-phenylmethyl}hexylacetamide wherein the substituent is amino, t-butyloxycarbonylamino, benzylsulfonylamino, formylamino, benzylaminosulfonylamino,
4-pyridylmethyloxycarbonylamino or acetylamino, or
N-[1-benzyl-2,3-dioxo-6-(2-pyridyloxy)hexyl]-2-[5-(formylamino)-6-oxo-2-phenyl-1,6-dihydropyrimidin-1-yl]acetamide.

24. A drug described according to any of claims 1-7 wherein the chymase inhibitor is the heterocyclic amide represented by formula (IV) or a pharmacologically acceptable salt thereof: 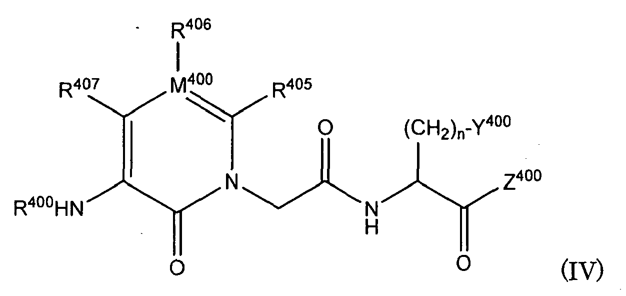 [wherein R⁴⁰⁰ is hydrogen, alkyl, -CHO, -CONH₂, -COR⁴⁰¹, -COOR⁴⁰¹, -CONHOR⁴⁰¹, -CONHR⁴⁰¹ ,-CONR⁴⁰¹R^{401'} , -CONHSO₂R⁴⁰¹, -COSR⁴⁰¹, -COCOR⁴⁰², -COCOOR⁴⁰² -CONHCOOR⁴⁰², -COCONR⁴⁰³R⁴⁰⁴_{'} -CSX⁴⁰⁰R⁴⁰¹, -SO₂WR⁴⁰¹, -SO₂NR⁴⁰¹R⁴⁰¹' or -SO₂E⁴⁰⁰ (wherein R⁴⁰¹ and R⁴⁰¹' may be the same or different and each independently represent alkyl, cycloalkyl, cycloalkylalkyl, aryl, arylalkyl, heteroaryl, heteroarylalkyl, heterocyclyl or heterocyclylalkyl, R⁴⁰², R⁴⁰³ and R⁴⁰⁴ may be the same or different and they each independently represent hydrogen, alkyl or arylalkyl, or -NR⁴⁰³R⁴⁰⁴ taken together may represent heterocyclyl, X⁴⁰⁰ represents a single bond, -NH-, -O- or -S-, W⁴⁰⁰ represents a single bond, -NH-, -NHCO-, NHCOO- or NHCONH-, and E⁴⁰⁰ represents hydroxyl or amino), R⁴⁰⁵, R⁴⁰⁶ and R⁴⁰⁷ may be the same or different, and either they each independently represent hydrogen or alkyl, or one of them represents aryl, arylalkyl, arylalkenyl, heteroaryl, heteroarylalkyl or heteroarylalkenyl with the rest being hydrogen, M⁴⁰⁰ represents carbon or nitrogen, wherein R⁴⁰⁶ is absent if M⁴⁰⁰ is nitrogen, Y⁴⁰⁰ represents cycloalkyl, aryl or heteroaryl, Z⁴⁰⁰ represents the groups shown by formula (IV-i), (IV-ii) and (IV-iii): 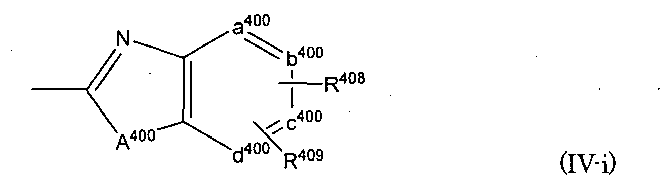 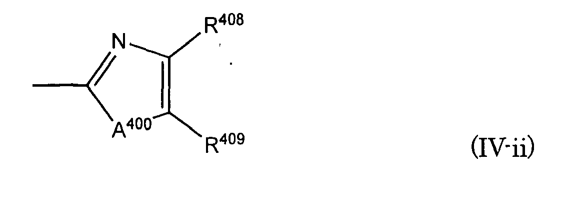 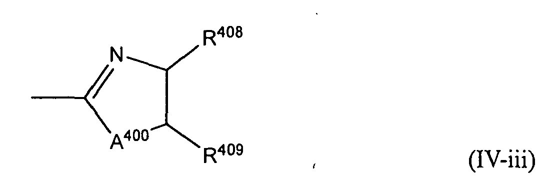 { wherein, R⁴⁰⁸ and R⁴⁰⁹ may be the same or different and they each independently represent hydrogen, alkyl, aryl, arylalkyl, heteroaryl, heteroarylalkyl, halogen, trifluoromethyl, cyano, nitro, -NR⁴¹⁰R^{410'}, -NHSO₂R⁴¹⁰, -OR⁴¹⁰, -COOR⁴¹⁰, -CONHSO₂R⁴¹⁰ or -CONR⁴¹⁰R^{410'} (wherein R⁴¹⁰ and R^{410'} may be the same or different and they each independently represent hydrogen, alkyl, cycloalkyl, cycloalkylalkyl, aryl, arylalkyl, heteroaryl, heteroarylalkyl or trifluoromethyl, or -NR⁴¹⁰R^{410'} taken together may represent heterocyclyl), A⁴⁰⁰ represents -O-, -S- or -NR⁴¹²- (wherein R⁴¹² represents hydrogen, alkyl, cycloalkyl or cycloalkylalkyl), a⁴⁰⁰, b⁴⁰⁰, c⁴⁰⁰ and d⁴⁰⁰ are all carbon or one of them is nitrogen with the rest being carbon}, n is 0 or 1; and
among the said groups alkyl, cycloalkyl, cycloalkylalkyl, aryl, arylalkyl, arylalkenyl, heteroaryl, heteroarylalkyl, heteroarylalkenyl, heterocyclyl and heterocyclylalkyl each may have substituents].

25. A drug according to claim 24 wherein Y⁴⁰⁰ is aryl optionally having substituents, Z⁴⁰⁰ is the group represented by formula (IV-i), one of R⁴⁰⁵, R⁴⁰⁶ and R⁴⁰⁷ is aryl optionally having substituents with the rest being hydrogen, wherein R⁴⁰⁶ is absent when M is nitrogen.

26. A drug according to claim 24 wherein the chymase inhibitor is methyl
2-[2-[2-[5-amino-2-(3-methoxyphenyl)-6-oxo-1,6-dihydropyrimidin-1-yl]acetamido]-3-phenylpropionyl]benzoxazole-5-carboxylate or methyl
2-[2-[5-amino-2-(4-fluorophenyl)-6-oxo-1,6-dihydropyrimidin-1-yl]acetamido]-3-phenylpropionyl]benzoxazole-5-carboxylate.

27. A drug according to any of claims 1-7 wherein the chymase inhibitor is the N-substituted benzothiophenesulfonamide derivative represented by formula (V) or a salt thereof: 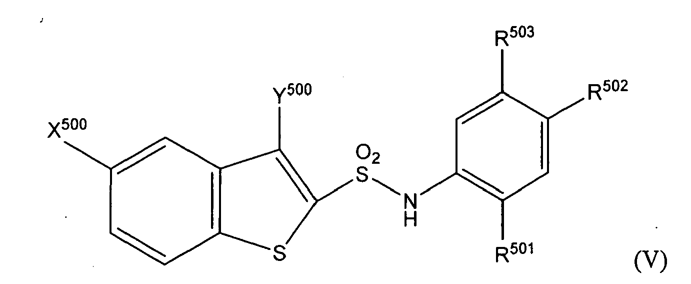 [wherein X⁵⁰⁰ represents hydrogen, halogen or lower alkyl, Y⁵⁰⁰ represents lower alkyl, R⁵⁰¹ and R⁵⁰² each may be the same or different and independently represent hydrogen, lower alkoxycarbonyl, lower alkylsulfonyl, benzoyl, C₁-C₄ acyl, lower alkoxy, lower alkoxycarbonylmethylthioacetyl, nitro, -CONHR⁵⁰⁴ (wherein R⁵⁰⁴ represents hydrogen, lower alkoxycarbonylmethyl, carboxymethyl or -CH(CH₂OH)COOR⁵⁰⁵ (wherein R⁵⁰⁵ represents hydrogen or lower alkyl)), the group represented by 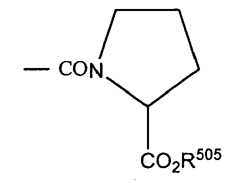 (wherein R⁵⁰⁵ is as defined above), the monocyclic heterocyclyl represented by 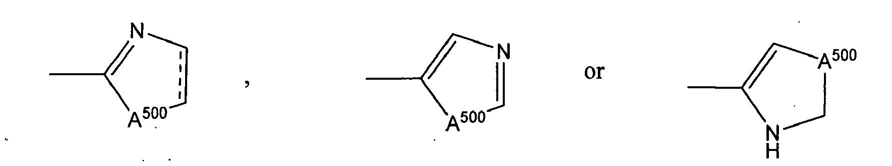 optionally substituted with -CO₂R⁵⁰⁵ (wherein A⁵⁰⁰ represents O, S or NH, the bond accompanying a dotted line represents a single or double bond and R⁵⁰⁵ is as defined above), lower hydroxyalkyl or cyano (except for cases wherein both R⁵⁰¹ and R⁵⁰² are hydrogen), and R⁵⁰³ represents hydrogen, lower alkoxy or lower alkyl], excluding compounds represented by the following formulas.

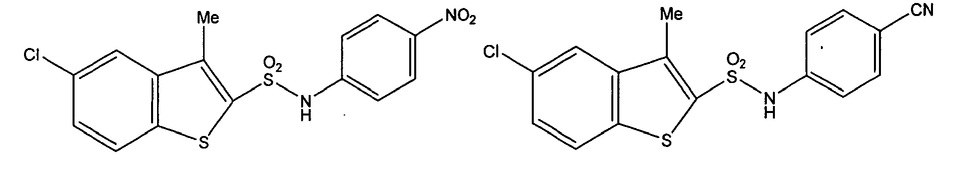

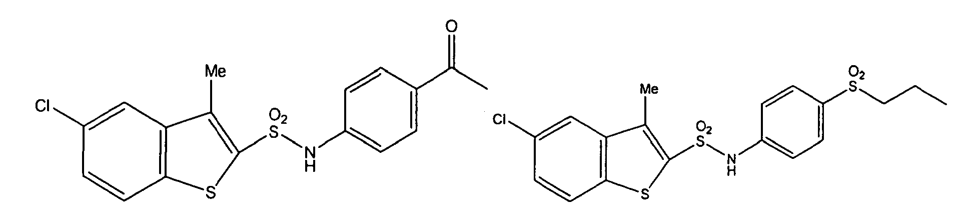

28. A drug according to claim 27 wherein the chymase inhibitor is
2-[4-(5-fluoro-3-methylbenzo[b]thiophen-2-yl)sulfonamido-3-methanesulfonylphenyl] oxazole-4-carboxylic acid.
